# FASCICULE DE BREVET EUROPEEN

(11) **EP 0 465 368 B1**
(45) Date de publication et mention de la délivrance du brevet: **20.05.1998**
(21) Numéro de dépôt: 91401857.7
(22) Date de dépôt: 04.07.1991
(51) Int. Cl.: C07D 233/84, C07D 233/90, A61K 31/415

(54) **Nouveaux dérivés soufrés de l'imidazole, leur procédé de préparation, les nouveaux intermédiaires obtenus, leur application à titre de médicaments et les compositions pharmaceutiques les renfermant**
Schwefelderivate von Imidazol, Verfahren zu ihrer Herstellung, Zwischenprodukte, ihre Verwendung als Arzneimittel und sie enthaltende pharmazeutische Zusammensetzungen
Sulfur derivatives of imidazole, process for their preparation, intermediates, their use as medicaments, and pharmaceutical compositions containing them

(30) Priorité: 05.07.1990 FR 9008538; 19.04.1991 FR 9104882
(43) Date de publication de la demande: 08.01.1992
(73) Titulaire: HOECHST MARION ROUSSEL, 92800 Puteaux (FR)
(72) Inventeur: Caille, Jean-Claude, F-75011 Paris (FR); Corbier, Alain, F-91370 Verrieres le Buisson (FR); Fortin, Michel, F-75018 Paris (FR); Hamon, Gilles, F-93340 Le Raincy (FR); Jouquey, Simone, F-75020 Paris (FR); Vevert, Jean-Paul, F-93500 Pantin (FR)
(74) Mandataire: Vieillefosse, Jean-Claude

(56) Documents cités:
- EP-A- 125 033
- EP-A- 253 310
- EP-A- 323 148
- EP-A- 324 377
- US-A- 4 179 512
- JOURNAL OF ORGANIC CHEMISTRY, vol. 49, no. 12, 1984, pages 2282 - 2284; K. AKUTAGAWA et al.: "Preparation of N-[Arylsulfonyl]sulfoximines by Oxidation of N-[Arylsulfonyl]sulfilimines with Sodium Hypochlorite in a Two-Phase..."
- Chemical Abstracts, Vol 95, 28 Heterocycles, 1981, Columbus Ohio, US; abstract no. 97673u

## Description

La présente invention concerne de nouveaux dérivés soufrés de l'imidazole, leur procédé de préparation, les nouveaux intermédiaires obtenus, leur application à titre de médicaments et les compositions pharmaceutiques les renfermant.

On connaissait déjà des dérivés de l'imidazole dans le domaine de l'angiotensine II par les demandes de brevets EP-A 324377 et EP-A 253310.

La présente invention a pour objet les produits de formule (I_{B}) : 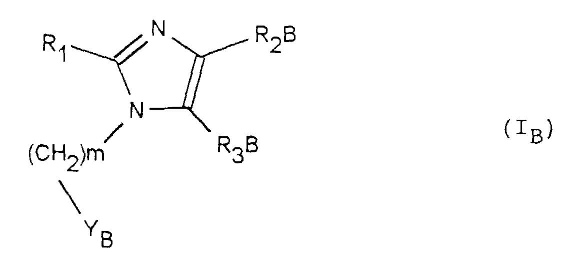 dans laquelle :
R₁ représente un radical alkyle renfermant au plus 4 atomes de carbone,
R_{2B} et R_{3B} identiques ou différents sont choisis parmi :
   - l'atome d'hydrogène,
   - un radical carboxy libre, salifié ou estérifié par un radical alkyle,
   - formyle, acyloxy choisi dans le groupe consistant en formyloxy, acétyloxy, propionyloxy, butyryloxy et benzoyloxy, sulfo,
   - alkyle et alcoxy éventuellement substitués,
   - phénylthio, phénylsulfonyle, phénylsulfinyle, alkylthio, alkylsulfonyle et alkylsulfinyle, éventuellement substitués, tel que dans tous ces radicaux que peuvent représenter R2B et R3B, les radicaux alkyle et alcoxy renferment au plus 6 atomes de carbone, et les radicaux alkyle, alcoxy et phényle sont éventuellement substitués par un ou plusieurs radicaux choisis parmi les atomes d'halogène et les radicaux hydroxyle, triflurorométhyle, acyloxy, carboxy libre salifié ou estérifié, phényle, pyridyle, tétrazolyle, alkyle et alcoxy renfermant au plus 4 atomes de carbone et eux-mêmes éventuellement substitués par un radical alcoxy renfermant au plus 4 atomes de carbone,
étant entendu que l'un au moins des groupes R_{2B} et R_{3B} représente un groupe choisi parmi phénylthio, phénylsulfonyle, phénylsulfinyle, alkylthio, alkylsulfonyle et alkylsulfinyle, éventuellement substitués,
et YB représente le radical -Y_{1B}-B-Y_{2B} dans lequel Y_{1B} représente un radical phényle, B représente une liaison simple carbone-carbone et Y_{2B} représente un radical carboxy libre ou estérifié par un radical alkyle linéaire ou ramifié renfermant au plus 4 atomes de carbone ou un radical phényle éventuellement substitué par un radical carboxy libre ou estérifié par un radical alkyle linéaire ou ramifié renfermant au plus 4 atomes de carbone, un radical cyano ou un radical tétrazolyle éventuellement salifié,
lesdits produits de formule (I_{B}) étant sous toutes les formes isomères possibles racémiques, énantiomères et diastéréoisomères, ainsi que les sels d'addition avec les acides minéraux et organiques ou aves les bases minérales et organiques desdits produits de formule (I_{B}).

L'invention a plus particulièrement pour objet les produits de formule (I_{B}) telle que définie ci-dessus et répondant à la formule (Iₐ) : 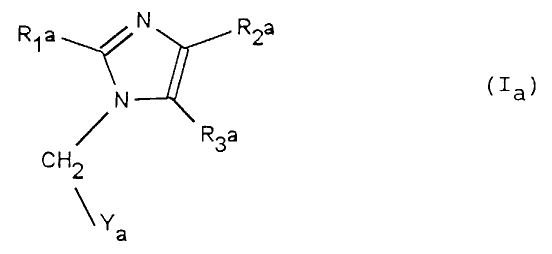 dans laquelle :
R₁ₐ représente un radical alkyle linéaire ou ramifié renfermant au plus 4 atomes de carbone,
R₂ₐ et R₃ₐ, identiques ou différents, sont choisis parmi :
   a) l'atome d'hydrogène, le radical mercapto, les radicaux formyle, carboxy libre, salifié ou estérifié,
   b) les radicaux R₄ₐ et -S(O)ₙR₄ₐ tel que n représente la valeur 0, 1 ou 2,
dans lesquels R₄ₐ représente un radical alkyle ou alkényle linéaire ou ramifié renfermant au plus 4 atomes de carbone, un radical cyclohexyle, un radical phényle, pyridyle, pyrimidinyle ou imidazolyle, tous ces radicaux étant éventuellement substitués par un ou plusieurs radicaux identiques ou différents choisis parmi :
-- les atomes d'halogène,
-- les radicaux hydroxyle, mercapto, acylthio, trifluorométhyle, trifluorométhylthio, trifluorométhoxy, cyano, azido, nitro,
-- le radical phényle,
-- les radicaux alkyle et alcoxy renfermant au plus 4 atomes de carbone, carboxy libre, salifié ou estérifié, tétrazolyle, acyle, acyloxy, et
-- les radicaux 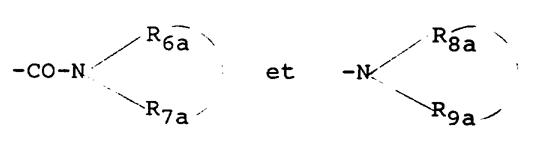
dans lesquels :
**ou bien** R₆ₐ, R₇ₐ, R₈ₐ et R₉ₐ, identiques ou différents, sont choisis parmi l'atome d'hydrogène, le radical hydroxyle, les radicaux alkyle, alcoxy, acyloxy ou acyle, ces radicaux renfermant au plus 6 atomes de carbone, carboxy libre, salifié ou estérifié, phényle, benzyle, phénéthyle, azépine, pipéridyle, morpholine, pyrrolidinyle, pipérazinyle,
**ou bien** d'une part R₆ₐ et R₇ₐ et d'autre part R₈ₐ et R₉ₐ forment respectivement avec l'atome d'azote auxquels ils sont liés un radical, ces radicaux identiques ou différents étant choisis parmi les radicaux imidazolyle, pyrrolyle, pyrrolinyle, pyrrolidinyle, pyridyle, pipéridinyle, pyrimidinyle, pyridazinyle, pyrazinyle, pipérazinyle, phénylpipérazinyle, pipéridyle, oxazolyle, morpholinyle et thiomorpholinyle, azépine, indolyle, ces radicaux étant éventuellement substitués par un ou plusieurs radicaux identiques ou différents choisis parmi les atomes d'halogène, les radicaux hydroxyle, trifluorométhyle, alkyle et alcoxy, ces radicaux renfermant au plus 6 atomes de carbone, étant entendu que l'un au moins de R₂ₐ ou R₃ₐ représente le radical -S(O)ₙR₄ₐ,
Yₐ représente le radical -Y₁ₐ-Bₐ-Y₂ₐ dans lequel :
- Y₁ₐ représente un radical phényle,
- Bₐ représente une simple liaison ou le radical -CO-NH-,
- Y₂ₐ est tel que :
   **soit,** si Bₐ représente une simple liaison ou un radical -CO-NH-, Y₂ₐ représente un radical phényle substitué en ortho par un radical carboxy libre, salifié ou estérifié ou un radical tétrazolyle,
   **soit,** si Bₐ représente une simple liaison, Y₂ₐ représente un radical cyano ou carboxy libre, salifié ou estérifié ou un radical tétrazolyle,
lesdits produits de formule (Iₐ) étant sous toutes les formes isomères possibles racémiques, énantiomères et diastéréoisomères, ainsi que les sels d'addition avec les acides minéraux et organiques ou avec les bases minérales et organiques desdits produits de formule (Iₐ).

Dans les produits de formules (I_{B}) et (I_{A}) et dans ce qui suit :
- le terme radical alkyle linéaire ou ramifié désigne de préférence les radicaux méthyle, éthyle, propyle, isopropyle, butyle, isobutyle, sec-butyle et tert-butyle mais peut également représenter un radical pentyle ou hexyle et particulièrement isopentyle et isohexyle,
- le terme radical alkényle linéaire ou ramifié désigne de préférence un radical vinyle, allyle, 1-propényle, butényle et particulièrement 1-butényle, ou pentényle,
- le terme atome d'halogène désigne de préférence l'atome de chlore, mais peut aussi représenter un atome de fluor, de brome ou d'iode,
- le terme radical alcoxy linéaire ou ramifié désigne de préférence les radicaux méthoxy, éthoxy, propoxy ou isopropoxy, mais peut aussi représenter un radical butoxy linéaire, secondaire ou tertiaire,
- le terme radical acyle désigne de préférence un radical ayant de 1 à 6 atomes de carbone tel que par exemple le radical formyle, acétyle, propionyle, butyryle ou benzoyle, mais également le radical pentanoyle, hexanoyle, acryloyle, crotonoyle ou carbamoyle,
- le terme radical acyloxy désigne par exemple un radical dans lequel le radical acyle a les valeurs indiquées ci-dessus et désigne de préférence un radical formyloxy, acétyloxy, propionyloxy, butyryloxy ou benzoyloxy,
- le terme radical alkylthio désigne de préférence les radicaux dans lesquels le radical alkyle est tel que défini ci-dessus comme par exemple dans méthylthio ou éthylthio,

Dans les produits de formules (I_{B}) et (I_{A}) et dans ce qui suit, les radicaux alkyle, alkényle, cyclohexyle et phényle :
- que peuvent représenter R₂B et R₃B,
- que peut renfermer le radical -S(O)ₙR_{4A} que peuvent représenter R_{2A} et R_{3A},
   ou
- que peuvent porter R_{2B} et R_{3B},
peuvent prendre les valeurs définies ci-dessus pour ces radicaux qui peuvent ou non être substitués par un ou plusieurs substituants identiques ou différents tels que définis ci-dessus pour ces radicaux.
R_{2B} et R_{3B} peuvent ainsi, par exemple, représenter un radical alkylthio, phénylthio, alkylsulfinyle, phénylsulfinyle, alkylsulfonyle ou phénylsulfonyle mais également un radical cyclohexylthio :
- les termes radical alkylthio, alkylsulfinyle et alkylsulfonyle désignent les radicaux dans lesquels le radical alkyle linéaire ou ramifié peut représenter, par exemple, les valeurs indiquées ci-dessus pour le radical alkyle ; ces radicaux représentent ainsi de préférence les radicaux méthylthio, hydroxyméthylthio, éthylthio, aminoéthylthio, méthylsulfinyle, éthylsulfinyle, méthylsulfonyle, éthylsulfonyle mais peut aussi représenter un radical propylthio, isopropylthio, butylthio, sec-butylthio, tert-butylthio, isopentylthio ou isohexylthio ou ces radicaux dans lesquels le radical thio est oxydé en radical sulfinyle ou sulfonyle,

Comme exemples de radicaux alkyle substitués par un radical aryle, on peut citer, par exemple, les radicaux benzyle, diphénylméthyle, triphénylméthyle, naphtylméthyle, indénylméthyle, thiénylméthyle tel que 2-thiényl méthyle, furylméthyle tel que furfuryle, pyridylméthyle, pyrimidylméthyle ou pyrrolylméthyle, étant entendu que dans la liste non exhaustive d'exemples de radicaux telle que citée ci-dessus, le radical alkyle peut être représenté tout aussi également par les radicaux éthyle, propyle ou butyle tel que, par exemple, dans le radical phénéthyle.

Comme exemples de radicaux alkényle substitués par un radical aryle, on peut citer, par exemple, les exemples donnés ci-dessus de radicaux arylalkyle dans lesquels le radical alkyle est remplacé par un radical alkényle tel que par exemple dans les radicaux phénylvinyle ou phénylallyle, étant entendu que dans ces radicaux le radical phényle peut être remplacé tout aussi également par un radical naphtyl, pyridyle ou encore par exemple l'un des radicaux aryles tels que définis ci-dessus.

Les radicaux carbamoyle et amino que peuvent représenter ou porter l'un ou plusieurs des éventuels substituants des radicaux définis dans les produits de formules (I_{B}) et (I) et dans ce qui suit et que peuvent représenter, en particulier : les radicaux 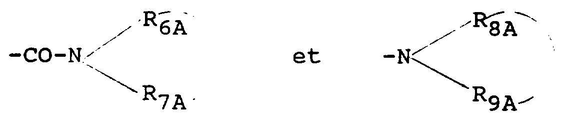 désigne des radicaux dans lesquels à l'atome d'azote sont liés deux radicaux, identiques ou différents, choisis parmi l'atome d'hydrogène pour donner le radical amino ; les radicaux alkyle tels que définis ci-dessus pour donner les radicaux monoalkyl- ou dialkylamino dans lesquels les radicaux alkyles linéaires ou ramifiés renferment de 1 à 6 atomes de carbone et en particulier des radicaux méthyle, éthyle, isopropyle, méthoxyméthyle, méthoxyéthyle, éthoxyéthyle ; les radicaux carbocycliques ou hétérocycliques que peuvent représenter R_{6A}, R_{7A}, R_{8A} et R_{9A} peuvent prendre les valeurs définies ci-dessus pour ces radicaux et en particulier phényle, benzyle, phénéthyle, naphtyle, indolyle, indolinyle, thiényle, furyle, pyrrolyle, pyridyle, pyrrolidinyle, pipéridino, morpholino, pipérazinyle, ces radicaux pouvant être substitués par un ou plusieurs radicaux tels que définis ci-dessus comme par exemple dans méthylpipérazinyle, fluorométhylpipérazinyle, éthylpipérazinyle, propylpipérazinyle, phénylpipérazinyle ou benzylpipérazinyle.

Lorsque R₆ et R₇ d'une part ou R₈ et R₉ d'autre part forment ensemble avec l'atome d'azote auquel ils sont liés un hétérocycle, il s'agit, par exemple, d'un cycle pyrrolyle, imidazolyle, pyrazinyle, indolyle, indolinyle, purinyle, pyrrolidinyle, pipéridyle, pipéridino, morpholino, pipérazinyle, imidazolidinyle , pyrazolidinyle, thiomorpholinyle, azépine ; ces radicaux peuvent être éventuellement substitués par les substituants déjà mentionnés précédemment et en particulier par un ou plusieurs radicaux choisis parmi les atomes de chlore et de fluor,les radicaux méthyle, éthyle, isopropyle, tert-butyle, méthoxy, éthoxy, propoxy, benzoyle, méthoxycarbonyle, éthoxycarbonyle, comme par exemple dans méthylpipérazinyle, éthylpipérazinyle, propylpipérazinyle, phénylpipérazinyle ou benzylpipérazinyle : dans ces deux derniers radicaux, les radicaux phényle et benzyle peuvent être substitués comme indiqué précédemment dans les radicaux aryle, arylalkyle et arylalkényle, comme par exemple dans chlorophényle ou trifluorophényle.

L'hétérocycle que peuvent former R_{6A} et R_{7A} d'une part ou R_{8A} et R_{9A} d'autre part respectivement avec l'atome d'azote auquel ils sont liés représente de préférence un hétérocycle saturé.

Les radicaux acyle que peuvent représenter R_{8A} et R_{9A} sont tels que définis précédemment et peuvent être choisis par exemple parmi les radicaux acétyle, propionyle, butyryle, pentanoyle ou carbamoyle.

Dans le cas où l'un ou les deux de R_{2A} et R_{3A}, identiques ou différents, renferment un radical carbamoyle ou amino soit : 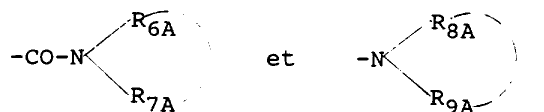 R_{6A}, R_{7A}, R_{8A} et R_{9A}, identiques ou différents, peuvent représenter tous les quatre des chaînes aliphatiques ou cyclisées ou l'un ou les deux de R_{6A} et R_{7A} d'une part et R_{8A} et R_{9A} d'autre part peuvent former avec l'atome d'azote auquel ils sont liés un hétérocycle tel que défini ci-dessus.

Les radicaux carbamoyle substitué 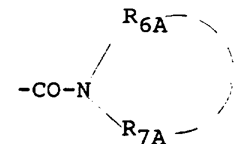 et amino substitué 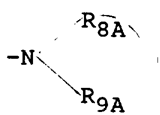 désignent respectivement les radicaux dans lesquels l'atome d'azote peut être substitué par un ou deux radicaux choisis parmi les radicaux tels que définis précédemment : à titre d'exemple et de façon non exhaustive, on peut citer comme radical carbamoyle substitué le groupe N-monoalkyl inférieur carbamoyle, par exemple, N-méthylcarbamoyle, N-éthylcarbamoyle ; le groupe N,N-dialkyl inférieur carbamoyle, par exemple, N,N-diméthylcarbamoyle, N,N-diéthylcarbamoyle ; le groupe N-(hydroxyalkyl inférieur) carbamoyle, par exemple, N-(hydroxyméthyl) carbamoyle, N-(hydroxyéthyl) carbamoyle ; le groupe carbamoylalkyle inférieur, par exemple carbamoylméthyle, carbamoyléthyle ; phénylcarbamoyle ; pyridylcarbamoyle ; benzylcarbamoyle ; N-méthyl N-phénylcarbamoyle ; pyridylméthylcarbamoyle.

Le radical amino substitué peut être par exemple un radical monoalkyl- ou dialkylamino dans lequel le radical alkyle est choisi parmi les radicaux méthyle, éthyle ou isopropyle.

Des exemples d'un tel radical amino sustitué sont donnés dans la partie expérimentale ci-après.

Lorsque R_{8A} ou R_{9A} représente un radical alcoxycarbonyle, ce radical est de préférence le radical tert-butyloxycarbonyle ou le radical benzyloxycarbonyle.

Le ou les radicaux carboxy des produits de formule (I_{B}) peuvent être salifiés ou estérifiés par les groupements divers connus de l'homme du métier parmi lesquels on peut citer, par exemple :
- parmi les composés de salification, des bases minérales telles que, par exemple, un équivalent de sodium, de potassium, de lithium, de calcium, de magnésium ou d'ammonium ou des bases organiques telles que, par exemple, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine,
- parmi les composés d'estérification, les radicaux alkyle pour former des groupes alcoxy carbonyle tel que, par exemple, méthoxycarbonyle, éthoxycarbonyle, tert-butoxycarbonyle ou benzyloxycarbonyle, ces radicaux alkyles pouvant être substitués par des radicaux choisis par exemple parmi les atomes d'halogène, les radicaux hydroxyle, alcoxy, acyle, acyloxy, alkylthio, amino ou aryle comme, par exemple, dans les groupements chlorométhyle, hydroxypropyle, méthoxyméthyle, propionyloxyméthyle, méthylthiométhyle, diméthylaminoéthyle, benzyle ou phénéthyle.

Les sels d'addition avec les acides minéraux ou organiques des produits de formule (I_{B}) peuvent être, par exemple, les sels formés avec les acides chlorhydrique, bromhydrique, iodhydrique, nitrique, sulfurique, phosphorique, propionique, acétique, formique, benzoïque, maléique, fumarique, succinique, tartrique, citrique, oxalique, glyoxylique, aspartique, ascorbique, les acides alcoylmonosulfoniques tels que par exemple l'acide méthanesulfonique, l'acide éthanesulfonique, l'acide propanesulfonique, les acides alcoyldisulfoniques tels que par exemple l'acide méthanedisulfonique,l'acide alpha, bêta-éthanedisulfonique, les acides arylmonosulfoniques tels que l'acide benzènesulfonique et les acides aryldisulfoniques.

Lorsque R_{2A} et R_{3A} représentent tous deux un groupement soufré de formule -S(O)ₙR_{4A}, R_{2A} et R_{3A} étant identiques ou différents, les produits préférés de l'invention sont en particulier les produits de formule (I_{A}) dans lesquels ces groupements soufrés ont le même degré d'oxydation.

Parmi les produits préférés de l'invention, se trouvent en particulier les produits de formule (I_{A}) dans lesquels l'un de R_{2A} et R_{3A} représente un groupement soufré éventuellement oxydé tel que défini ci-dessus par -S(O)ₙR_{4A} et l'autre de R_{2A} et R_{3A} représente un radical R_{4A} tel que défini ci-dessus et de préférence un radical alkyle, alkényle ou aryle éventuellement substitué par un ou plusieurs substituants ainsi qu'il est défini ci-dessus.

Parmi les produits préférés de l'invention, se trouvent tout particulièrement les produits de formule (I_{B}) dans lesquels R_{2B} représente un radical soufré.

Dans le cas où R_{2A} ou R_{3A} représente un radical -S(O)ₙR_{4A} et R_{4A} représente un radical amino, n est de préférence égal à 2.

R_{2B} et R_{3B} peuvent notamment représenter les radicaux alkylthio ou alkénylthio, éventuellement substitués par un ou plusieurs radicaux choisis parmi les radicaux formyle ; hydroxyle ; alcoxy ; acyloxy ; carboxy libre, salifié ou estérifié ; amino ; amino substitué ; carbamoyle ; carbamoyle substitué ; mercapto ; alkylthio ; acylthio tel que acétylthio ; arylthio tel que phénylthio ; sulfo, cycloalkyle tel que cyclohexyle ; pyridinyle ; pyrimidinyle ; phényle.

Parmi les substituants que peuvent porter les radicaux R_{2B} et R_{3B}, les radicaux amino et carbamoyle peuvent notamment être substitués par un ou deux radicaux choisis parmi les radicaux alkyle et les acides aminés choisis parmi les 20 acides aminés naturels tels que notamment la proline ou par exemple la glycine, l'alanine, la leucine, l'isoleucine, la valine ou la phénylalanine.

Les radicaux amino et carbamoyle que peuvent porter les radicaux R_{2B} et R_{3B} peuvent également constituer des dérivés cyclisés par la formation d'un radical cyclique entre l'atome d'azote et ses substituants ainsi qu'il est indiqué ci-dessus et ci-après.

R_{2B} et R_{3B} peuvent ainsi notamment représenter les radicaux alkylthio, substitués par un ou plusieurs atomes d'halogène tels que chlore et fluor, tels que par exemple les radicaux :
-S-CF₃ ; -S-CHF₂ ; -S-CH₂F ; -S-CF₂-CHF₂ ; -S-CF₂-CHFCl.

Les radicaux R_{2B} et R_{3B} peuvent ainsi représenter les radicaux suivants dans lesquels m, m1 et m2, identiques ou différents représentent les valeurs 0 à 2, -S-Me ; 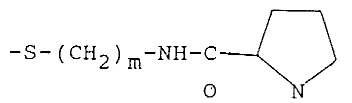 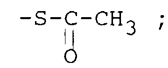 -SO₃H
-S-(CH₂)ₘ₁-S-(CH₂)ₘ₂-X₁ ;
-S-(CH₂)ₘ-X₁ ;
-S-(CH₂)ₘ₁-NH-(CH₂)ₘ₂-X₁ ;
-S-CH=CH-(CH₂)ₘ-X₁ ;
-S-(CH₂)ₘ₁-CH=CH-(CH₂)ₘ₂-X₁ ;
-S-C≡C-(CH₂)ₘ-X₁ ;
dans lesquels X₁ représente H, OH, cyclohexyle, pyridyle, pyrimidyle, phényle, naphtyle, CHO, COOH, NH₂ ou

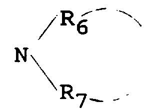

Les radicaux R_{2B} et R_{3B} peuvent également représenter, particulièrement, les radicaux suivants :
-COOH ; -NH₂ ; -C≡N ; -OMe ; -OEt ; -CH=CH-COOH ; tétrazolyle ; 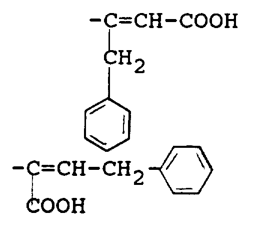 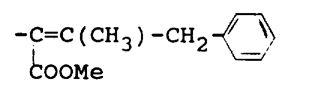 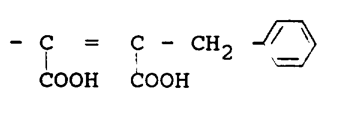 sous toutes leurs formes isomères, isomères cis-trans, 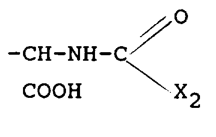 -NH-CH₂-COO-X₂
-NH-COO-X₂
X₂ représentant un radical alkyle ou aryle.

Les radicaux R_{2B} et R_{3B} peuvent tout particulièrement représenter le radical : 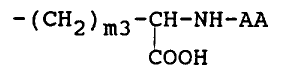 dans lequel m3 représente les valeurs 0 à 4 et AA représente un acide aminé naturel tel que notamment le proline ou le glycine et le radical :

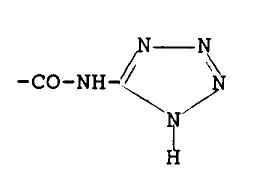

Les produits de formule (I_{B}) représentent donc particulièrement les produits dans lesquels R_{2B} et R_{3B} ont les significations indiquées ci-dessus et tout particulièrement les produits dans lesquels R_{2B} représente un radical alkylthio éventuellement substitué tel que défini ci-dessus ou alcoxy tel que par exemple méthoxy et R_{3B} représente un radical carboxy libre, salifié ou estérifié, ou amidifié tel que notamment -COOH, -COO méthyl, -CONH₂ ou

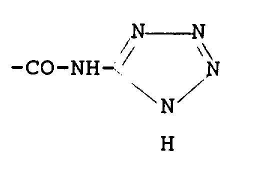

A ces significations indiquées ci-dessus de R_{2B} et R_{3B} dans les produits de formule (I_{B}) sont associées les valeurs indiquées ci-dessus pour Y_{B} et notamment le radical biphényle substitué en ortho par un radical carboxy libre, salifié ou estérifié, cyano ou tétrazolyle éventuellement substitué

Les radicaux alkyle, alkényle, aryle, aralkyle et arylalkényle peuvent eux-mêmes être substitués ainsi qu'il est indiqué ci-dessus pour ces radicaux.

On peut citer par exemple et de façon non exhaustive les radicaux : -SO₂-NH₂, -SO₂-NH-CH₃, -SO₂-NH-CF₃, -SO₂-NH-C₆H₅, -SO₂-NH-CH₂-C₆H₅, -CH₂-SO₂-NH₂, -CH₂-SO₂-NH-C₆H₅, -SO₂-NH-CO-NH-CH₃, -SO₂-NH-CO-NH-C₆H₅, -SO₂-NH-CO-NH-CF₃, -SO₂-NH-CO-NH-CH₂-C₆H₅, -SO₂-NH-CO-NH-D dans lequel D représente un radical phényle, pyridine ou pyrimidine éventuellement substitué par un atome de chlore, 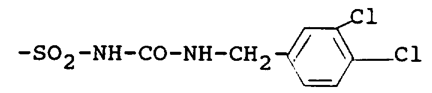 -SO₂-NH-CO-NH-CH=CH-CH₃, 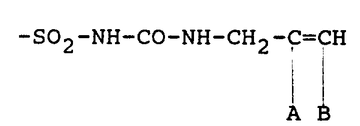 dans lequel A et B, identiques ou différents, sont choisis parmi l'atome d'hydrogène, les radicaux phényle, pyridyle et pyrimidyle, 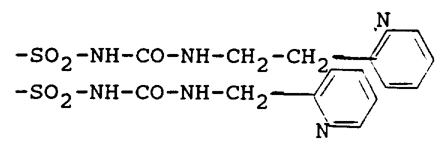 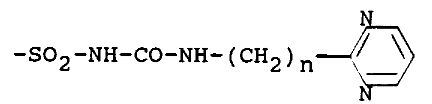 avec n = 1 ou 2.

On peut citer par exemple et de façon non exhaustive les radicaux :
-NH-SO₂-CH₃, -NH-SO₂-C₆H₅, -NH-SO₂-CF₃, -NH-CH₂-SO₂-NH-C₆H₅, -CO-NH-SO₂-C₂H₅, -CO-NH-SO₂-CH₃, -CO-NH-SO₂-CH₂-C₆H₅.

L'invention a particulièrement pour objet les produits de formule (I_{B}) telle que définie ci-dessus dans laquelle : R₁ représente un radical alkyle renfermant au plus 4 atomes de carbone,
R_{2B} et R_{3B} identiques ou différents sont choisis parmi les radicaux :
- carboxy libre, salifié ou estérifié par un radical alkyle linéaire ou ramifié renfermant au plus 4 atomes de carbone, formyle, acyloxy, choisi dans le groupe consistant en formyloxy, acétyloxy, propionyloxy, butyryloxy et benzoyloxy,
- alkyle renfermant au plus 4 atomes de carbone éventuellement substitués par un radical hydroxyle
- phénylthio, phénylsulfonyle, phénylsulfinyle,
- alkylthio, alkylsulfonyle et alkylsulfinyle,
dans lesquels le radical alkyle renferme au plus 4 atomes de carbone,
et Y_{B} représente le radical -Y_{1B}-B-Y_{2B} dans lequel Y₁ représente un radical phényle, B représente une liaison simple carbone-carbone et Y₂ représente un radical carboxy libre ou estérifié par un radical alkyle linéaire ou ramifié renfermant au plus 4 atomes de carbone ou un radical phényle éventuellement substitué par un radical carboxy libre ou estérifié par un radical alkyle linéaire ou ramifié renfermant au plus 4 atomes de carbone,
lesdits produits de formule (I_{B}) étant sous toutes les formes isomères possibles racémiques, énantiomères et diastéréoisomères, ainsi que les sels d'addition avec les acides minéraux et organiques ou avec les bases minérales et organiques desdits produits de formule (I_{B}).

L'invention a tout particulièrement pour objet les produits de formule (I_{B}) telle que définie ci-dessus, dans laquelle l'un de R_{2B} ou R_{3B} représente un radical alcoxy, alkylthio ou arylthio ces deux derniers étant éventuellement oxydés sous forme de sulfoxyde ou de sulfone et ces trois radicaux étant éventuellement substitués et l'autre représente un radical carboxy libre salifié ou estérifié, lesdits produits de formule (I_{B}) étant sous toutes les formes isomères possibles racémiques, énantiomères et diastéréoisomères, ainsi que les sels d'addition avec les acides minéraux et organiques ou avec les bases minérales et organiques uesdits produits de formule (I_{B}).

Parmi les substituants des radicaux alkylthio, alcoxy, arylthio et aryloxy, éventuellement oxydés, on peut citer par exemple les radicaux hydroxyle, alcoxy, carboxy libre, salifié ou estérifié, acyle, acyloxy, alkyle, phényle, les atomes d'halogène.

Parmi les produits objet de l'invention, peuvent être cités tout particulièrement les produits de formule (I) répondant aux formules suivantes :
- l'acide 2-butyl 1-[[2'-carboxy (1,1'-biphényl)-4-yl] méthyl] 4-(phénylthio) 1H-imidazole-5-carboxylique
- l'acide 2-butyl 1-[[2'-carboxy (1,1'-biphényl)-4-yl] méthyl] 4-(méthylthio) 1H-imidazole-5-carboxylique
- l'acide 4'-[[2-butyl 4-(éthylthio) 5-(hydroxyméthyl) 1H-imidazol-1-yl] méthyl] (1,1'-biphényl)-2-carboxylique
- l'acide 2-butyl 1-[[2'-carboxy (1,1'-biphényl)-4-yl] méthyl] 4-(éthylsulfonyl) 1H-imidazole-5-carboxylique
- l'acide 2-butyl 1-[[2'-carboxy (1,1'-biphényl)-4-yl] méthyl] 4-(éthylsulfinyl) 1H-imidazole-5-carboxylique
- l'acide 2-butyl 1-[[2'-carboxy (1,1'-biphényl)-4-yl] méthyl] 4-(éthylthio) 1H-imidazole-5-carboxylique
- l'acide 2-butyl 1-[[2'-carboxy (1,1'-biphényl)-4-yl] méthyl] 4-(phénylsulfonyl) 1H-imidazole-5-carboxylique
- l'acide 2-butyl 1-[[2'-carboxy (1,1'-biphényl)-4-yl] méthyl] 4-(phénylsulfinyl) 1H-imidazole-5-carboxylique
- l'acide 2-butyl 1-[[2'-tétrazolyl (1,1'-biphényl)-4-yl] méthyl] 4-(méthylthio) 1H-imidazole-5-carboxylique.

L'invention a également pour objet un procédé de préparation de produits de formule (I_{B}) telle que définie ci-dessus, caractérisé en ce que l'on fait réagir un composé de formule (II) : 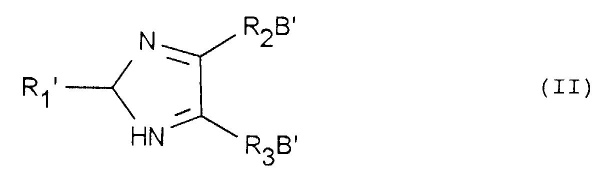 dans laquelle R₁', R_{2B}' et R_{3B}' ont les significations indiquées ci-dessus, respectivement pour R₁, R_{2B} et R_{3B} et dans lesquelles les éventuelles fonctions réactives sont éventuellement protégées par des groupements protecteurs, avec un composé de formule (III) :

Z-(CH₂)ₘ-Y_{B}' (III)

dans laquelle Z représente un atome d'halogène ou un sulfonate et Y_{B}' a la signification indiquée ci-dessus, pour Y_{B} dans laquelle les éventuelles fonctions réactives sont éventuellement protégées par des groupements protecteurs, pour obtenir un produit de formule (IV) : 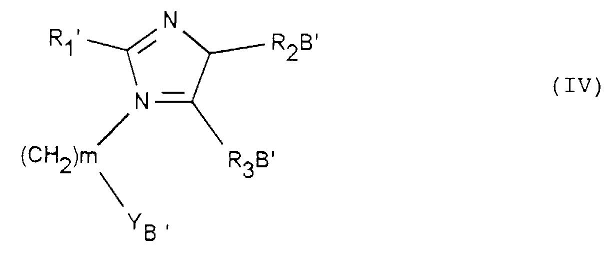 dans laquelle R₁', R_{2B}', R_{3B}' et Y_{B}' ont les significations indiquées ci-dessus, produit de formule (IV) que l'on soumet, si désiré et si nécessaire, à l'une ou plusieurs des réactions suivantes, dans un ordre quelconque :
a) une réaction d'élimination des groupements protecteurs que peuvent porter les fonctions réactives,
b) une réaction de salification par un acide minéral ou organique ou par une base pour obtenir le sel correspondant,
c) une réaction d'estérification de fonction acide,
d) une réaction de saponification de fonction ester en fonction acide,
e) une réaction de transformation de la fonction cyano en fonction acide,
f) une réaction de réduction de la fonction carboxy en fonction alcool,
g) une réaction de transformation de fonction alcoxy en fonction hydroxyle,
h) une réaction d'oxydation de groupement alkylthio ou arylthio en sulfoxyde ou sulfone correspondant,
i) une réaction de transformation de fonction sulfoxyde ou sulfone en fonction sulfoximine correspondante,
j) une réaction d'oxydation de fonction alcool en fonction aldéhyde ou acide,
k) une réaction de transformation de fonction nitrile en tétrazole,
l) une réaction de dédoublement des formes racémiques en produits dédoublés,
m) une réaction de transformation du radical formyle en radical carbamoyle,
n) une réaction de transformation du radical carbamoyle en radical nitrile,
lesdits produits de formule (I_{B}) ainsi obtenus étant sous toutes les formes isomères possibles racémiques, énantiomères et diastéréoisomères.

Dans des conditions préférentielles de mise en oeuvre de l'invention, le produit de formule (IV) peut être obtenu par addition du produit de formule (III) sur la fonction amine libre de l'imidazole de formule (II) :
dans le produit de formule (III), quand Z représente un atome d'halogène, Z représente de préférence un atome de brome mais peut également représenter un atome de chlore ou d'iode ; dans le produit de formule (III), Z peut également représenter un sulfonate de formule -O-SO₂-A dans laquelle A représente de préférence un radical alkyle renfermant au plus 4 atomes de carbone tel que par exemple méthyle ou un radical aryle tel que par exemple phényle éventuellement substitué par exemple par un radical alkyle renfermant au plus 4 atomes de carbone tel que par exemple méthyle : A peut ainsi représenter, par exemple, le radical méthyle ou encore le radical tolyl pour donner par exemple un tosylate.

La réaction du produit de formule (III) sur le produit de formule (II) peut être réalisée dans un solvant tel que par exemple le diméthylformamide ou encore le tétrahydrofuranne, le diméthoxyéthane ou le diméthylsulfoxyde au reflux du solvant ou à la température ambiante, de préférence sous agitation ; la réaction est réalisée de préférence en présence d'une base telle que par exemple l'hydrure de sodium ou de potassium ou encore du carbonate de sodium ou de potassium, du méthylate ou éthylate ou tert-butylate de sodium ou de potassium.

Selon les valeurs de R₁', R_{2B}', R_{3B}' et Y_{B}', les produits de formule (IV) constituent ou non des produits de formule (I_{B}).

Les diverses fonctions réactives que peuvent porter certains composés des réactions définies ci-dessus peuvent, si nécessaire, être protégées : il s'agit par exemple des radicaux hydroxyle, acyle, carboxy libres ou encore amino et mono-alkylamino qui peuvent être protégés par les groupements protecteurs appropriés.

La liste suivante, non exhaustive, d'exemples de protection de fonctions réactives peut être citée :
- les groupements hydroxyle peuvent être protégés par exemple par les radicaux alkyle tels que tert-butyle, triméthylsilyle, tert-butyldiméthylsilyle, méthoxyméthyle, tétrahydropyrannyle, benzyle ou acétyle,
- les groupements amino peuvent être protégés par exemple par les radicaux acétyle, trityle, benzyle, tert-butoxycarbonyle, phtalimido ou d'autres radicaux connus dans la chimie des peptides,
- les groupements acyles tel que le groupement formyle peuvent être protégés par exemple sous forme de cétals cycliques ou non cycliques tels que le diméthyl ou diéthylcétal ou l'éthylène dioxycétal,
- les fonctions acide des produits décrits ci-dessus peuvent être, si désiré, amidifiées par une amine primaire ou secondaire par exemple dans du chlorure de méthylène en présence, par exemple, de chlorhydrate de 1-éthyl-3-(diméthylaminopropyl) carbodiimide à la température ambiante :
- les fonctions acide peuvent être protégées par exemple sous forme d'esters formés avec les esters facilement clivables tels que les esters benzyliques ou ter butyliques ou des esters connus dans la chimie des peptides.

Les réactions auxquelles les produits de formule (IV) telle que définie ci-dessus peuvent être soumis, si désiré ou si nécessaire, peuvent être réalisées, par exemple, comme indiqué ci-après.
a) L'élimination de groupements protecteurs tels que par exemple ceux indiqués ci-dessus peut être effectuée dans les conditions usuelles connues de l'homme de métier notamment par une hydrolyse acide effectuée avec un acide tel que l'acide chlorhydrique, benzène sulfonique ou para-toluène sulfonique, formique ou trifluoroacétique ou encore par une hydrogénation catalytique.

Le groupement phtalimido peut être éliminé par l'hydrazine.

On trouvera une liste de différents groupements protecteurs utilisables par exemple dans le brevet BF 2 499 995.
b) Les produits décrits ci-dessus peuvent, si désiré, faire l'objet de réactions de salification par exemple par un acide minéral ou organique selon les méthodes usuelles connues de l'homme du métier.
c) Les produits décrits ci-dessus peuvent, si désiré, faire l'objet, sur les éventuelles fonctions carboxy, de réactions de salification par une base minérale ou organique ou d'estérification : ces réactions d'estérification et de salification peuvent être réalisées selon les méthodes usuelles connues de l'homme du métier.
d) Les éventuelles transformations de fonctions ester en fonction acide des produits décrits ci-dessus peuvent être, si désiré, réalisées dans les conditions usuelles connues de l'homme du métier notamment par hydrolyse acide ou alcaline par exemple par de la soude ou de la potasse en milieu alcoolique tel que, par exemple, dans du méthanol ou encore par de l'acide chlorhydrique ou sulfurique.
e) Les éventuelles fonctions cyano des produits décrits ci-dessus peuvent être, si désiré, transformées en fonction acide dans les conditions usuelles connues de l'homme du métier par exemple par une double hydrolyse réalisée en milieu acide tel que par exemple dans un mélange d'acide sulfurique, d'acide acétique glacial et d'eau, ces trois composés étant de préférence en proportions égales, ou encore dans un mélange de soude, d'éthanol et d'eau au reflux.
f) Les éventuelles fonctions carboxy libre ou estérifié des produits décrits ci-dessus peuvent être, si désiré, réduites en fonction alcool par les méthodes connues de l'homme de métier : les éventuelles fonctions carboxy estérifié peuvent être, si désiré, réduites en fonction alcool par les méthodes connues de l'homme du métier et notamment par de l'hydrure de lithium et d'aluminium dans un solvant tel que par exemple le tétrahydrofuranne ou encore le dioxane ou l'éther éthylique.

Les éventuelles fonctions carboxy libre des produits décrits ci-dessus peuvent être, si désiré, réduites en fonction alcool notamment par de l'hydrure de bore.
g) Les éventuelles fonctions alcoxy telles que notamment méthoxy des produits décrits ci-dessus peuvent être, si désiré, transformées en fonction hydroxyle dans les conditions usuelles connues de l'homme du métier par exemple par du tribromure de bore dans un solvant tel que par exemple le chlorure de méthylène, par du bromhydrate ou chlorhydrate de pyridine ou encore par de l'acide bromhydrique ou chlorhydrique dans de l'eau ou de l'acide acétique au reflux.
h) Les éventuels groupements alkylthio ou arylthio des produits décrits ci-dessus peuvent être, si désiré, transformés en les fonctions sulfoxyde ou sulfone correspondantes dans les conditions usuelles connues de l'homme du métier telles que par exemple par les peracides comme par exemple l'acide peracétique ou l'acide métachloroperbenzoïque ou encore par l'ozone, l'oxone, le périodate de sodium dans un solvant tel que par exemple le chlorure de méthylène ou le dioxanne à la température ambiante.

L'obtention de la fonction sulfoxyde peut être favorisée par un mélange équimolaire du produit renfermant un groupement alkylthio ou arylthio et du réactif tel que notamment un peracide.

L'obtention de la fonction sulfone peut être favorisée par un mélange du produit renfermant un groupement alkylthio ou arylthio avec un excés du réactif tel que notamment un peracide.
i) Les éventuelles fonctions sulfure, sulfoxyde ou sulfone des produits décrits ci-dessus peuvent être, si désiré, transformées en les fonctions sulfoximine correspondantes dans les conditions usuelles connues de l'homme du métier :
des exemples non exhaustifs de préparation de produits renfermant une fonction sulfoximine sont décrites ci-dessous.

Ainsi par exemple pour la préparation de composés tels que les N-(arylsulfonyl) sulfoximines et par exemple dans le cas où le groupement aryle que représente X' est un radical toluène, la sulfoximine peut être obtenue par action de l'azoture de paratoluènesulfonyle sur le sulfoxyde correspondant soit -S(O)CH₃ de préférence en présence de cuivre ainsi qu'il est indiqué, par exemple, dans la référence suivante : J. A. C. S., 95, pp. 4287 (1973) JOHNSON C.R. & coll.

Une autre méthode également utilisée consiste à traiter la N-tosylsulfilimine, elle-même préparée à partir du sulfure par action, par exemple, de la chloramine "T", par un agent oxydant comme par exemple, l'hypochlorite de sodium dans des conditions de transfert de phase ainsi qu'il est indiqué, par exemple, dans la référence suivante :
J. Org. Chem., 49, pp. 2282 (1984) AKUTAGAWA K.& coll.
j) Les éventuelles fonctions alcool des produits décrits ci-dessus peuvent être, si désiré, transformées en fonction aldéhyde ou acide par oxydation dans les conditions usuelles connues de l'homme du métier telles que par exemple par action de l'oxyde de manganèse pour obtenir les aldéhydes ou du réactif de Jones pour accéder aux acides.
k) Les éventuelles fonctions nitrile des produits décrits ci-dessus peuvent être, si désiré, transformées en tétrazole dans les conditions usuelles connues de l'homme du métier telles que par exemple par cycloaddition d'un azidure métallique tel que par exemple un azidure de trialkylétain sur la fonction nitrile ainsi qu'il est indiqué dans la méthode décrite dans l'article référencé comme suit :
   J. Organometallic Chemistry., 33, 337 (1971) KOZIMA S.& coll.
l) Les éventuelles formes optiquement actives des produits de formule (I_{B}) peuvent être préparées par dédoublement des racémiques selon les méthodes usuelles connues de l'homme du métier.

Les réactions de transformation de radical formyle en radical carbamoyle et de radical carbamoyle en radical nitrile sont réalisées selon les conditions usuelles connues de l'homme du métier. Ces réactions ainsi que la transformation du radical nitrile en tétrazole sont effectuées de préférence lorsque ces substituants sont portés en alpha du substituant biphényle que peut représenter -Y_{B}.

Les composés de formule (I_{B}) tels que définis ci-dessus ainsi que leurs sels d'addition avec les acides présentent d'intéressantes propriétés pharmacologiques.

Les produits sont doués de propriétés antagonistes pour le récepteur à l'angiotensine II et sont ainsi notamment inhibiteurs des effets de l'angiotensine II, en particulier de l'effet vasoconstricteur et également de l'effet trophique au niveau des myocytes.

Ces propriétés justifient leur application en thérapeutique et l'invention a également pour objet à titre de médicaments, les produits tels que définis par la formule (I_{B}) ci-dessus, lesdits produits de formule (I_{B}) étant sous toutes les formes isomères possibles racémiques ou optiquement actives, ainsi que les sels d'addition avec les acides minéraux ou organiques pharmaceutiquement acceptables desdits produits de formule (I_{B}).

L'invention a particulièrement pour objet à titre de médicaments, les produits de formule (I_{B}) telle que définie ci-dessus dans laquelle :
R₁ représente un radical alkyle renfermant au plus 4 atomes de carbone,
R₂ et R₃ identiques ou différents sont choisis parmi les radicaux :
   - carboxy libre, salifié ou estérifié par un radical alkyle linéaire ou ramifié renfermant au plus 4 atomes de carbone, formyle, acyloxy,
   - alkyle renfermant au plus 4 atomes de carbone éventuellement substitué par un radical hydroxyle
   - phénylthio, phénylsulfonyle, phénylsulfinyle,
   - alkylthio, alkylsulfonyle et alkylsulfinyle,
   dans lesquels le radical alkyle renferme au plus 4 atomes de carbone,
et Y représente le radical -Y₁-B-Y₂ dans lequel Y₁ représente un radical phényle, B représente une liaison simple carbone-carbone et Y₂ représente un radical carboxy libre ou estérifié par un radical alkyle linéaire ou ramifié renfermant au plus 4 atomes de carbone ou un radical phényle éventuellement substitué par un radical carboxy libre ou estérifié par un radical alkyle linéaire ou ramifié renfermant au plus 4 atomes de carbone,
lesdits produits de formule (I_{B}) étant sous toutes les formes isomères possibles racémiques, énantiomères et diastéréoisomères, ainsi que les sels d'addition avec les acides minéraux et organiques ou avec les bases minérales et organiques desdits produits de formule (I_{B}).

L'invention a tout particulièrement pour objet à titre de médicaments, les produits de formule (I_{B}) répondant à la formule (Iₐ) telles que définies ci-dessus, dans laquelle l'un de R₂ₐ ou R₃ₐ représente un radical alkylthio ou arylthio éventuellement oxydés sous forme de sulfoxyde ou de sulfone et éventuellement substitués comme indiqué ci-dessus, et l'autre représente un radical carboxy libre salifié ou estérifié, lesdits produits de formule (Iₐ) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (Iₐ).

L'invention a plus particulièrement pour objet, à titre de médicaments, les produits décrits ci-aprés dans les exemples et notamment les produits de formule (IB) suivants :
- l'acide 2-butyl 1-[[2'-carboxy (1,1'-biphényl)-4-yl] méthyl] 4-(phénylthio) 1H-imidazole-5-carboxylique
- l'acide 2-butyl 1-[[2'-carboxy (1,1'-biphényl)-4-yl] méthyl] 4-(méthylthio) 1H-imidazole-5-carboxylique
- l'acide 4'-[[2-butyl 4-(éthylthio) 5-(hydroxyméthyl) 1H-imidazol-1-yl] méthyl] (1,1'-biphényl)-2-carboxylique
- l'acide 2-butyl 1-[[2'-carboxy (1,1'-biphényl)-4-yl] méthyl] 4-(éthylsulfonyl) 1H-imidazole-5-carboxylique
- l'acide 2-butyl 1-[[2'-carboxy (1,1'-biphényl)-4-yl] méthyl] 4-(éthylsulfinyl) 1H-imidazole-5-carboxylique
- l'acide 2-butyl 1-[[2'-carboxy (1,1'-biphényl)-4-yl] méthyl] 4-(éthylthio) 1H-imidazole-5-carboxylique
- l'acide 2-butyl 1-[[2'-carboxy (1,1'-biphényl)-4-yl] méthyl] 4-(phénylsulfonyl) 1H-imidazole-5-carboxylique
- l'acide 2-butyl 1-[[2'-carboxy (1,1'-biphényl)-4-yl] méthyl] 4-(phénylsulfinyl) 1H-imidazole-5-carboxylique
- l'acide 2-butyl 1-[[2'-tétrazolyl (1,1'-biphényl)-4-yl] méthyl] 4-(méthylthio) lH-imidazole-5-carboxylique
ainsi que leurs sels d'addition avec les acides minéraux ou organiques pharmaceutiquement acceptables.

Les médicaments, objet de l'invention, peuvent être utilisés dans le traitement de l'hypertension artérielle, des insuffisances cardiaques, des insuffisances rénales et dans la prévention des resténoses post-angioplastie.

Ils peuvent également être utilisés dans le traitement de certains désordres gastro-intestinaux, gynécologiques et en particulier pour un effet relaxant au niveau de l'utérus.

L'invention s'étend aux compositions pharmaceutiques renfermant à titre de principe actif l'un au moins des médicaments tels que définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous toutes les formes pharmaceutiques couramment utilisées en médecine humaine comme, par exemple, les comprimés simples ou dragéifiés, les gélules, les granulés, les suppositoires, les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple, de 1 à 100 mg par jour chez l'adulte, par voie orale.

Certains produits de départ de formule (II) sont connus et peuvent être préparés par exemple comme indiqué dans le brevet européen EP 168 950.

Les produits de départ de formule (II) peuvent en particulier être préparés suivant un nouveau procédé et la présente invention concerne ainsi également ce nouveau procédé de préparation des produits de départ de formule (II) telle que définie ci-dessus, caractérisé en ce que l'on soumet un composé de formule (IIₐ) : 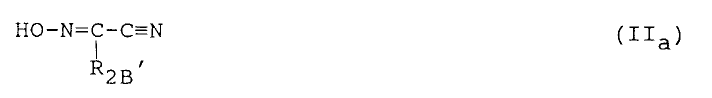 dans laquelle R_{2B}' a la signification indiquée ci-dessus, pour R_{2B} dans laquelle les éventuelles fonctions réactives sont éventuellement protégées par des groupements protecteurs, que l'on soumet à l'action d'un agent réducteur, pour obtenir l'amine correspondante de formule (II_{b}) : 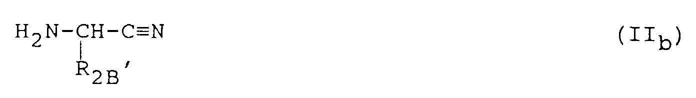 dans laquelle R_{2B}' a la signification indiquée précédemment, que l'on soumet à l'action d'un composé de formule (II_{c}) : 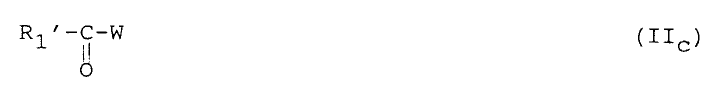 dans laquelle R₁' a la signification indiquée ci-dessus, pour R₁ dans laquelle les éventuelles fonctions réactives sont éventuellement protégées par des groupements protecteurs et W représente un radical hydroxyle ou un atome d'halogène, pour obtenir un produit de formule (II_{d}) : 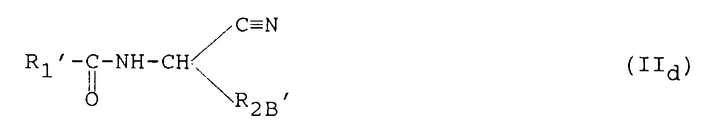 dans laquelle R₁' et R_{2B}' ont les significations indiquées précédemment, que l'on fait réagir avec un composé de formule (IIₑ) :

R_{3B}'-SH (IIₑ)

dans laquelle R_{3B}' a la signification indiquée ci-dessus pour R_{3B} dans laquelle les éventuelles fonctions réactives sont éventuellement protégées par des groupements protecteurs, pour obtenir un produit de formule (II_{f}) : 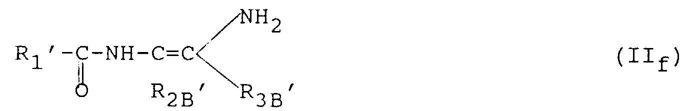 dans laquelle R₁', R_{2B}' et R_{3B}' ont les significations indiquées précédemment, que l'on soumet à une réaction de cyclisation pour obtenir un produit de formule (II), produit de formule (II) que l'on soumet, si désiré et si nécessaire, à l'une ou plusieurs des réactions suivantes, dans un ordre quelconque :
a) une réaction d'élimination des groupements protecteurs que peuvent porter les fonctions réactives protégées,
b) une réaction de salification par un acide minéral ou organique ou par une base pour obtenir le sel correspondant,
c) une réaction d'estérification de fonction acide,
d) une réaction de saponification de fonction ester en fonction acide,
e) une réaction de transformation de la fonction cyano en fonction acide,
f) une réaction de réduction de la fonction carboxy en fonction alcool,
g) une réaction de transformation de fonction alcoxy en fonction hydroxyle,
h) une réaction d'oxydation de groupement alkylthio ou arylthio en sulfoxyde ou sulfone correspondant,
i) une réaction de transformation de fonction sulfoxyde ou sulfone en fonction sulfoximine correspondante,
j) une réaction d'oxydation de fonction alcool en fonction aldéhyde ou acide,
k) une réaction de transformation de fonction nitrile en tétrazole,
l) une réaction de dédoublement des formes racémiques en produits dédoublés,
m) une réaction de transformation du radical formyle en radical carbamoyle,
n) une réaction de transformation du radical carbamoyle en radical nitrile,
lesdits produits de formule (II) ainsi obtenus étant sous toutes les formes isomères possibles racémiques, énantiomères et diastéréoisomères.

Dans des conditions préférentielles de mise en oeuvre de l'invention, le procédé ci-dessus est réalisé de la manière suivante :
- la réduction de l'oxime de formule (IIₐ) pour donner le composé de formule (II_{b}) peut être réalisée selon les méthodes usuelles connues de l'homme du métier telles que par exemple par un amalgame d'aluminium préparé dans les conditions usuelles telles que par exemple par action du chlorure de mercure sur de l'aluminium : la réaction est réalisée dans un solvant tel que par exemple le tétrahydrofuranne ou le toluène, de préférence à une température d'environ 50°C ;
- l'addition du produit de formule (II_{c}) dans laquelle W représente de préférence un atome de chlore mais peut aussi représenter un atome de brome sur l'amine de formule (II_{b}) ainsi obtenue peut être réalisée selon les méthodes connues de l'homme du métier telle que par exemple en présence d'une base telle que par exemple la pyridine ou encore la triéthylamine ; la réaction est réalisée de préférence à une température d'environ 0°C,
- l'addition du dérivé soufré de formule (IIₑ) sur l'amide de formule (II_{d}) ainsi obtenue pour obtenir le produit de formule (II_{f}) est réalisée, par exemple, par mise en solution de l'amide de formule (II_{d}) dans un solvant tel que par exemple un alcool tel que l'éthanol ou le méthanol, puis addition successive d'une base telle que par exemple la triéthylamine et du composé de formule (IIₑ) de préférence sous agitation et à la température ambiante,
- la réaction de cyclisation du composé de formule (II_{f}) ainsi obtenu en produit de formule (II) peut être réalisée dans un solvant tel que, par exemple, le dichlorométhane, le dichloroéthane ou encore le chloroforme : la réaction peut être réalisée par exemple en présence de pentachlorure de phosphore (PC15) dissous au préalable dans du dichorométhane à une température d'environ -78°C en présence d'une base telle que par exemple la pyridine ou la diméthylaminopyridine : la réaction peut être réalisée sous agitation à la température ambiante.

Le produit de formule (II) ainsi obtenu peut être soumis à l'une ou plusieurs des réactions indiquées ci-dessus, ces réactions pouvant être réalisées dans les mêmes conditions que celles définies ci-dessus pour les produits de formule (IV).

Le composé de formule (IIₐ) peut être, par exemple, l'éthylisonitrosocyanoacétate que l'on peut trouver, par exemple, sous forme de produit commmercialisé par LANCASTER sous la référence 8930.

Les composés de départ de formule (III) peuvent être disponibles dans le commerce ou peuvent être préparés selon les méthodes usuelles connues de l'homme du métier.

Un procédé de préparation de certains produits de formule (III) telle que définie ci-dessus, peut consister à soumettre le iodobenzoate de méthyle par exemple, sous forme de produit commmercialisé par JANSSEN, à l'action du iodotoluène par exemple, sous forme de produit commmercialisé par FLUKA, la réaction se réalisant par exemple en présence de cuivre en poudre à une température d'environ 100°C à 300°C, pour obtenir un produit : 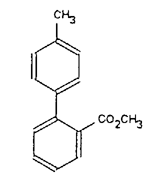 dont le radical carboxy estérifié peut, si désiré, être libéré du radical alkyle par les méthodes classiques connues de l'homme du métier ou indiquées ci-dessus, par exemple d'hydrolyse acide ou alcaline, que l'on peut soumettre à une réaction de bromation sur le radical méthyle par les méthodes classiques connues de l'homme du métier par exemple par action du n-bromosuccinimide dans le tétrachlorure de carbone.

Des exemples de préparation de composés de formule (III) sont décrits dans la littérature et des exemples en sont donnés notamment dans le brevet US 4,880,804 ou par exemple dans la référence Chemistry and Industry 7 september 1987 HOWARD and COLQUHOUN pp. 612-617.

La présente invention a enfin pour objet à titre de produits industriels nouveaux et notamment à titre de produits intermédiaires nécessaire à la préparation des produits de formule (I), les composés de formule (II) dans laquelle R₁' ne représente pas le radical méthyle.

Les exemples suivants illustrent l'invention sans toutefois la limiter.

### PREPARATION 1 : 2-butyl 4-(éthylthio) 1H-imidazole-5-carboxylate d'éthyle

### STADE A : Aminocyano acétate d'éthyle.

On agite pendant 2 minutes 4 g d'aluminium dans 40 cm³ d'une solution de chlorure mercurique à 5 % dans l'eau, on décante, lave à l'eau (2 fois 30 cm³) puis avec du tétrahydrofuranne. On ajoute 150 cm³ de tétrahydrofuranne puis en 3 minutes on introduit une solution de 10 g de cyano (hydroximino) acétate d'éthyle dans 60 cm³ de tétrahydrofuranne. On maintient la température inférieure à 60°C mais supérieure à 47°C. Après 15 minutes d'agitation le milieu réactionnel est filtré, l'insoluble est lavé avec du tétrahydrofuranne. On évapore le filtrat sous pression réduite et obtient 7 g du produit recherché utilisé tel quel pour le stade suivant.

### STADE B : cyano[(1-oxo pentyl) amino] acétate d'éthyle.

A une solution agitée à 0°C de 6,71 g du produit obtenu au stade A, ci-dessus, dans 100 cm³ de chlorure de méthylène, on ajoute à 0°C 4,24 cm³ de pyridine puis, en 30 minutes, 6,31 cm³ de chlorure de pentanoyle en maintenant la température inférieure à 6°C. On évapore ensuite, à sec sous pression réduite, entraine l'excès de pyridine avec du toluène et reprend le résidu avec 200 cm³ de chlorure de méthylène, on lave deux fois avec de l'eau puis évapore à nouveau sous vide. Après empâtage, du résidu obtenu, dans l'éther isopropylique, on recueille 8,4 g du produit recherché. F = 88°C.

### STADE C : 3-amino 3-(éthylthio) 2-[(1-oxo pentyl) amino] propénoate d'éthyle.

A une solution de 11,6 g de produit obtenu comme au stade B dans 250 cm³ d'éthanol, on ajoute 0,76 cm³ de triéthylamine et 8 cm³ d'éthanethiol. On agite à température ambiante 4 à 5 jours en procédant à des ajouts quotidiens de 8 cm³ d'éthanethiol jusqu'à absence de produit de départ. On évapore à sec sous pression réduite et empâte le résidu dans l'éther et obtient 10,8 g de produit recherché F = 110°C. Un second jet de 1,2 g de produit attendu est recueilli des liqueurs mères d'empâtage.

### STADE D : 2-butyl 4-(éthylthio) 1H-imidazole-5-carboxylate d'éthyle.

A une solution de 3,035 g de pentachlorure de phosphore dans 50 cm³ de chlorure de méthylène, refroidie à -78°C, on ajoute 1,96 g de diméthyl amino pyridine en solution dans 10 cm³ de chlorure de méthylène puis ensuite une solution de 2 g du produit obtenu au stade C dans 20 cm³ de chlorure de méthylène. On agite 16 heures à température ambiante. On verse le milieu réactionnel dans 200 cm³ d'une solution de bicarbonate de sodium, on agite 1 heure et extrait avec du chlorure de méthylène, on lave à l'eau saturée de chlorure de sodium, sèche, filtre et évapore à sec sous pression réduite. On obtient 2,6 g de résidu que l'on chromatograhie sur silice, (éluant : chlorure de méthylène-acétone (9-1)). On obtient 1,24 g du produit recherché.

| Analyse pour C₁₂H₂₀N₂O₂S = 256,355 | | | | |
|---|---|---|---|---|
| | C | H | N | S |
| % calculés | 56,30 | 7,86 | 10,94 | 12,52 |
| % trouvés | 56,20 | 7,90 | 10,70 | 12,40 |

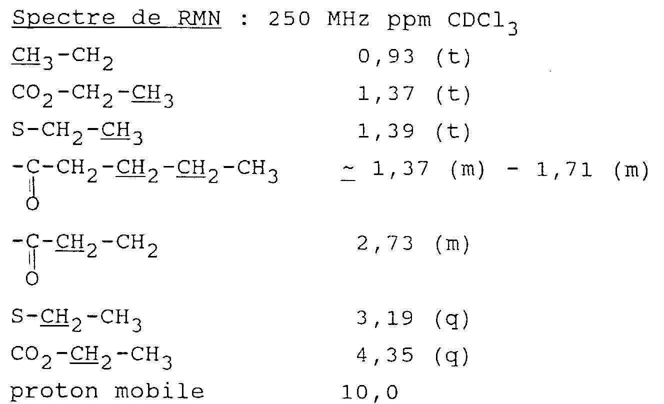

### PREPARATION 2 : 2-butyl 4-(phénylthio) 1H-imidazole-5-carboxylate d'éthyle

### STADE A : 3-amino 2-[(1-oxo pentyl) amino] 3-(phénylthio) propénoate d'éthyle.

On opère comme au stade C de la préparation 1 à partir de 3 g de produit obtenu au stade B de la préparation 1 en utilisant 2,9 cm³ de thiophénol, puis après 16 heures, à nouveau on ajoute 1,45 cm³ de thiophénol, on agite 48 heures supplémentaires et obtient 3,92 g du produit recherché, isolé après empâtage dans l'éther isopropylique. F = 115°C.

| Analyse pour C₁₆H₂₂N₂O₃S = 322,34 | | | | |
|---|---|---|---|---|
| | C | H | N | S |
| % calculés | 59,60 | 6,88 | 8,69 | 9,94 |
| % trouvés | 59,50 | 7,00 | 8,60 | 9,80 |

### STADE B : 2-butyl 4-(phénylthio) 1H-imidazole-5-carboxylate d'éthyle.

On opère comme au stade D de la préparation 1 à partir de 322 mg du produit obtenu au stade A ci-dessus. On obtient, après chromatographie sur silice (éluant : chlorure de méthylène-acétone (9-1)) 210 mg du produit recherché. F = 74°C.

| Analyse pour C₁₆H₂₀N₂O₂S = 304,41 | | | | |
|---|---|---|---|---|
| | C | H | N | S |
| % calculés | 63,13 | 6,62 | 9,20 | 10,53 |
| % trouvés | 62,70 | 6,60 | 9,10 | 10,30 |

### PREPARATION 3 : 4-(éthylthio) 2-méthyl 1H-imidazole-5-méthanol

A une solution de 1 g de 4-(éthylthio) 2-méthyl 1H-imidazole-5-carboxylate d'éthyle (obtenu comme à la préparation 1 en utilisant, au stade B, du chlorure d'acétyle à la place de chlorure de pentanoyle) dans 100 cm³ de chlorure de méthylène, on ajoute en 10 minutes à -70°C : 11,64 cm³ d'une solution 1,2 M d'hydrure de diisobutylaluminium dans le toluène. On agite 3 heures à température ambiante puis, on hydrolyse par ajout de 1 cm³ d'eau. On agite 15 minutes, filtre, lave l'insoluble avec 20 cm³ de chlorure de méthylène puis 5 fois 20 cm³ de mélange chlorure de méthylène-méthanol (9-1). Après séchage, le filtrat est amené à sec sous pression réduite. On empâte le résidu (760 mg) dans 7,6 cm³ de chlorure de méthylène et obtient 614 mg du produit attendu. F = 153°C.

### Spectre IR :

Absence 〉C = O
Absorption complexe région OH/NH
Hétéroaromatique 1582 cm⁻¹ - 1524 cm⁻¹

### PREPARATION 4 : 2-butyl 4-(éthylthio) 1H-imidazole-5-méthanol

On opère comme à la préparation 3 à partir de 500 mg du produit obtenu à la préparation 1, on obtient ainsi 320 mg du produit recherché. F = 128/130°C.

| Spectre de RMN : DMSO | |
|---|---|
| CH₃-(CH₂)₃ | 0,91 (t) |
| CH₃-(CH₂)₂-CH₂ | 1,37 (m) - 1,68 (m) |
| S-CH₂-CH₃ | 1,18 (t) |
| S-CH₂-CH₃ | 3,09 (m) |
| =C-CH₂-OH | 4,64 (s) |
| OH | 4,56 |

### EXEMPLE 1 : 4'-[[5-(éthylthio) 4-(hydroxyméthyl) 2-methyl 1H-imidazol-1-yl] méthyl] (1,1'-biphényl)-2-carboxylate de méthyle

A une suspension de 95 mg de méthylate de sodium avec 5 cm³ de diméthylformamide, on ajoute 300 mg de produit obtenu à la préparation 3 en solution dans 3 cm³ de diméthylformamide. On agite 20 minutes à température ambiante puis ajoute 1,19 g de 4'-(bromométhyl) (1,1'-biphényl)-2-carboxylate de méthyle en solution dans 5 cm³ de diméthylformamide, on agite 1 heure à température ambiante, verse dans 100 cm³ d'eau, extrait avec de l'acétate d'éthyle, lave à l'eau saturée de chlorure de sodium, sèche et évapore à sec sous pression réduite. On chromatographie le résidu (1,5 g) sur silice (éluant : chlorure de méthylène-acétate d'éthyle-méthanol (5-4-1)). On obtient 390 mg du produit recherché. F = 150°C et 165 mg de produit de l'exemple 3.

| Analyse pour C₁₆H₂₀N₂O₂S = 304,41 | | | | |
|---|---|---|---|---|
| | C | H | N | S |
| % calculés | 63,13 | 6,62 | 9,20 | 10,53 |
| % trouvés | 62,70 | 6,60 | 9,10 | 10,30 |

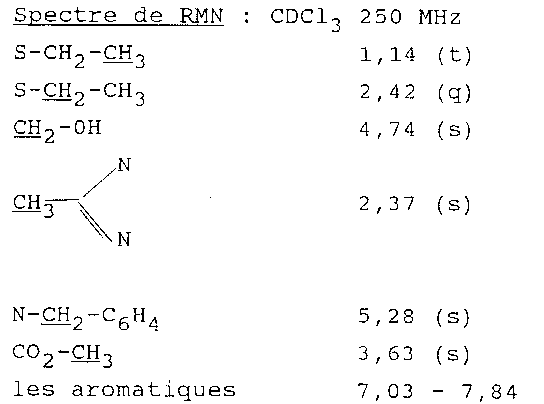

### EXEMPLE 2 : Acide 4'-[[5-(éthylthio) 4-(hydroxyméthyl) 2-méthyl 1H-imidazol-1-yl] méthyl] (1,1'-biphényl)-2-carboxylique

On agite pendant 7 heures au reflux une solution de 300 mg du produit obtenu à l'exemple 1 avec 15 cm³ d'éthanol et 0,75 cm³ de solution 2N d'hydroxyde de sodium. On refroidit puis neutralise par ajout de 0,75 cm³ d'acide chlorhydrique 2N. On évapore à sec sous pression réduite. On agite 15 minutes avec 2 cm³ d'eau, essore et obtient 255 mg de produit dont on recristallise 100 mg dans 10 cm³ d'isopropanol contenant 0,5 cm³ d'eau. On obtient 80 mg du produit recherché. F = 205°C.

| Analyse pour C₂₁H₂₂N₂O₃S = 382,49 | | | | |
|---|---|---|---|---|
| | C | H | N | S |
| % calculés | 65,94 | 5,79 | 7,32 | 8,38 |
| % trouvés | 66,10 | 5,80 | 7,20 | 8,20 |

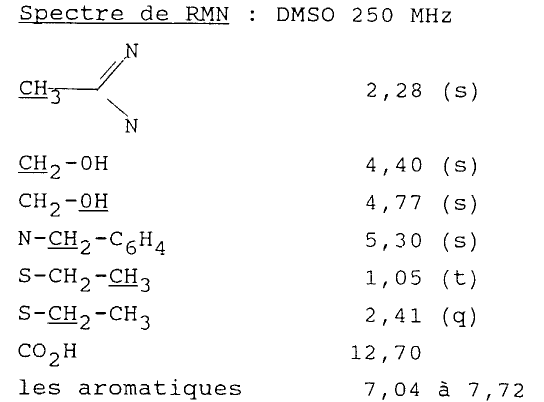

### EXEMPLE 3 : 4'-[[4-(éthylthio) 5-(hydroxyméthyl) 2-méthyl 1H-imidazol-1-yl] méthyl] (1,1'-biphényl)-2-carboxylate de méthyle

Le produit recherché est obtenu par chromatographie à l'exemple 1. On obtient 165 mg de produit.

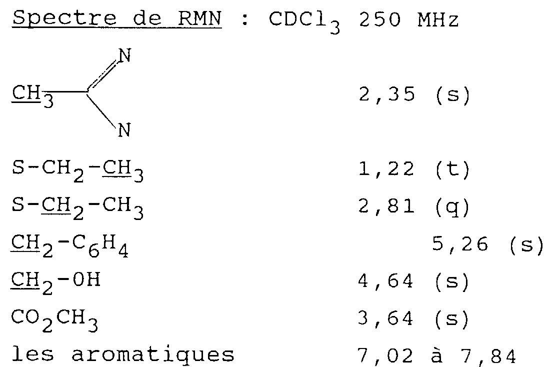

### EXEMPLE 4 : Acide 4'-[[4-(éthylthio) 5-(hydroxyméthyl) 2-méthyl 1H-imidazol-1-yl] méthyl] (1,1'-biphényl)-2-carboxylique

On opère comme à l'exemple 2 à partir de 170 mg du produit obtenu comme à l'exemple 3. On obtient 70 mg du produit recherché, isolé après empâtage dans l'acétate d'éthyle. F = 250°C.

| Analyse pour C₂₁H₂₂N₂O₃S = 382,49 | | | | |
|---|---|---|---|---|
| | C | H | N | S |
| % calculés | 65,94 | 5,79 | 7,32 | 8,38 |
| % trouvés | 62,40 | 5,40 | 6,70 | 7,80 |

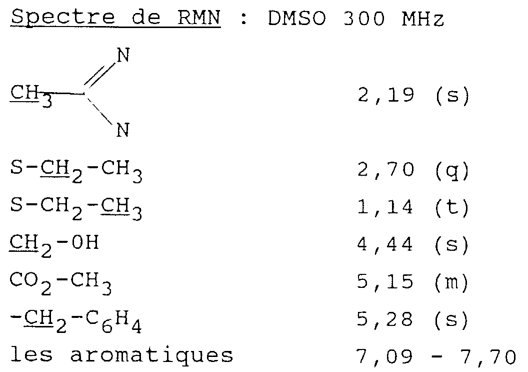

### EXEMPLE 5 : 2-butyl 4-(éthylthio) 1-[[2'-(méthoxycarbonyl) (1,1'-biphényl)-4-yl] méthyl] 1H-imidazole-5-carboxylate d'éthyle

On agite pendant 24 heures au reflux : 257 mg du produit obtenu à la préparation 1 avec 340 mg de 4'-(bromométhyl) (1,1'-biphényl)-2-carboxylate de méthyle, 180 mg de carbonate de potassium et 20 cm³ de diméthylformamide. On extrait avec 4 fois 100 cm³ d'acétate d'éthyle, lave avec une solution saturée de chlorure de sodium, sèche et évapore à sec sous pression réduite. On chromatographie le résidu (500 mg) sur silice, éluant (essence G-acétate d'éthyle (8-2)). On obtient 258 mg du produit recherché.

| Spectre de RMN : CDCl₃ ppm | |
|---|---|
| CH₃-CH₂-CH₂ | 0,89 (t) |
| CO₂-CH₂-CH₃ | 1,33 (t) |
| S-CH₂-CH₃ | 1,41 (t) |
| CH₃-CH₂ | 1,2 à 1,5 (m) |
| | 1,67 (m) |
| =C-CH₂-CH₂ | 2,65 (t) |
| S-CH₂-CH₃ | 3,21 (q) |
| CO₂-CH₂-CH₃ | 4,27 (q) |
| N-CH₂-C₆H₄ | 5,56 (s) |

### EXEMPLE 6 : Acide 2-butyl 1-[[2'-carboxy (1,1'-biphényl)-4-yl] méthyl] 4-(éthylthio) 1H-imidazole-5-carboxylique

On opère comme à l'exemple 2 à partir de 250 mg du produit obtenu à l'exemple 5. On obtient 180 mg de produit que l'on recristallise dans 10 cm³ d'acétone. On recueille 88 mg du produit recherché. F = 196°C.

| Analyse pour C₂₄H₂₆N₂O₄S = 438,55 | | | | |
|---|---|---|---|---|
| | C | H | N | S |
| % calculés | 65,73 | 5,97 | 6,39 | 7,31 |
| % trouvés | 65,50 | 6,0 | 6,20 | 7,30 |

| Spectre de RMN : DMSO 250 MHz | |
|---|---|
| CH₃-CH₂-CH₂ | 0,82 (t) |
| CH₃-CH₂-CH₂-CH₂ | 1,30 - 1,55 (m) |
| CH₂-C | 2,62 |
| S-CH₂-CH₃ | 1,30 (t) |
| S-CH₂-CH₃ | 3,07 (q) |
| N-CH₂-C₆H₄ | 5,59 (s) |
| H aromatiques | 7,04 - 7,71 |

### EXEMPLE 7 : 2-butyl 1-[[2'-[(1,1-diméthyl éthoxy) carbonyl] (1,1'-biphényl)-4-yl] méthyl] 4-(éthylthio) 1H-imidazole-5-carboxylate d'éthyle

On opère comme à l'exemple 5 à partir de 200 mg du produit obtenu à la préparation 1 en utilisant 405 mg de 4'-(bromométhyl) (1,1'-biphényl)-2-carboxylate de tert-butyle. On obtient après chromatographie sur silice (éluant : essence G-chlorure de méthylène-acétate d'éthyle (50-45-5)) 325 mg du produit recherché.

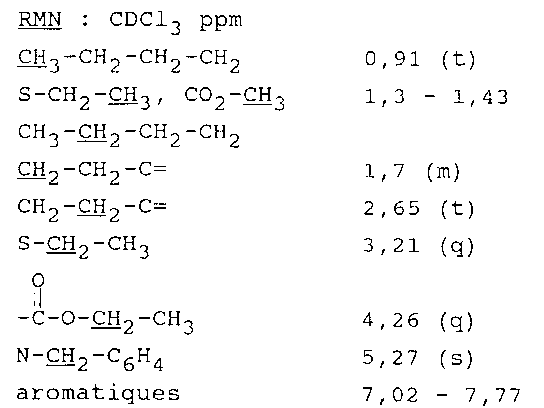

### EXEMPLE 8 : Acide 2-butyl 1-[[2'-carboxy (1,1'-biphényl)-4-yl] méthyl] 4-(éthylthio) 1H-imidazole-5-carboxylique

On agite pendant 2 heures, 325 mg du produit obtenu à l'exemple 7 avec 3 cm³ de chlorure de méthylène et 1,1 cm³ d'acide trifluoroacétique. On évapore à sec, entraine avec 3 fois 5 cm³ de toluène, on obtient 183 mg de produit brut. F = 156°C.

Après recristallisation obtenue par dissolution dans 2 cm³ de chlorure de méthylène chaud et ajout de 2 cm³ d'éther isopropylique, on obtient 120 mg du produit recherché. F = 156°C.

| Analyse pour C₂₆H₃₀N₂O₄S = 466,6 | | | | |
|---|---|---|---|---|
| | C | H | N | S |
| % calculés | 66,93 | 6,48 | 6,0 | 6,87 |
| % trouvés | 66,50 | 6,4 | 5,8 | 6,80 |

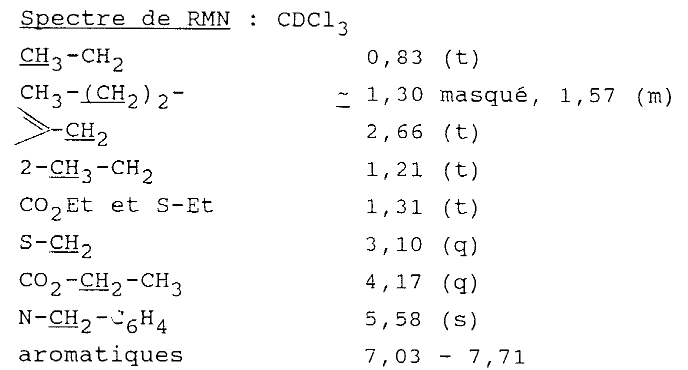

### EXEMPLE 9 : 2-butyl 4-(éthylthio) 1-[[(4-méthoxy carbonyl) phényl] méthyl] 1H-imidazole-5-carboxylate d'éthyle

On agite pendant 30 minutes, 20,6 mg d'hydrure de sodium à 50 % dans l'huile dispersé dans 4 cm³ de diméthylformamide et une solution de 100 mg du produit obtenu à la préparation 1 dans 1 cm³ de diméthylformamide. On ajoute ensuite 107,1 mg de 4-(bromométhyl) benzoate de méthyle. On agite 5 heures au reflux, on verse dans l'eau, extrait à l'acétate d'éthyle, lave, sèche et évapore à sec sous pression réduite. On obtient 177 mg de produit recherché.

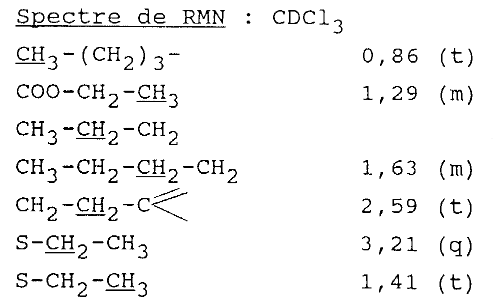

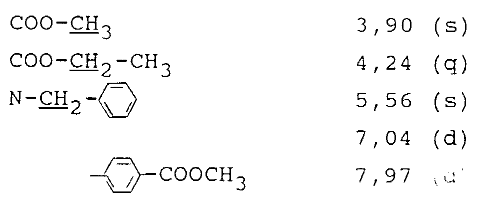

### EXEMPLE 10 : Acide 2-butyl 1-[(4-carboxy phényl) méthyl] 4-(éthylthio) 1H-imidazole-5-carboxylique

On opère comme à l'exemple 2 à partir de 117 mg du produit obtenu à l'exemple 9. On obtient 60 mg de produit brut que l'on recristallise dans l'acétone, on recueille ainsi 35 mg de produit recherché. F = 168°C.

| Analyse pour C₁₈H₂₂N₂O₄S = 362,45 | | | | |
|---|---|---|---|---|
| | C | H | N | S |
| % calculés | 59,65 | 6,12 | 7,73 | 8,84 |
| % trouvés | 59,70 | 6,3 | 7,5 | 8,80 |

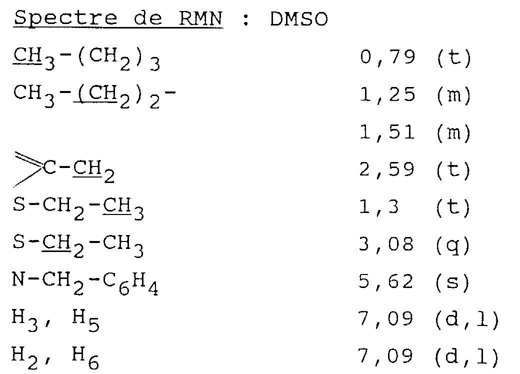

### EXEMPLE 11 : 4'-[[2-butyl 4-(éthylthio) 5-(hydroxymethyl) 1H-imidazol-1-yl] méthyl] (1,1'-biphényl)-2-carboxylate de méthyle

On opère comme à l'exemple 1, à partir de 2,35 g du produit obtenu à la préparation 4, en utilisant 3,99 g de 4'-(bromométhyl) (1,1'-biphényl)-2-carboxylate de méthyle. On obtient 6,3 g de produit que l'on chromatographie sur silice (éluant : chlorure de méthylène-acétone (5-5)). On obtient 609 mg du produit recherché et 2,48 g du produit correspondant à l'exemple 13.

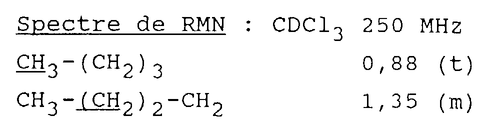

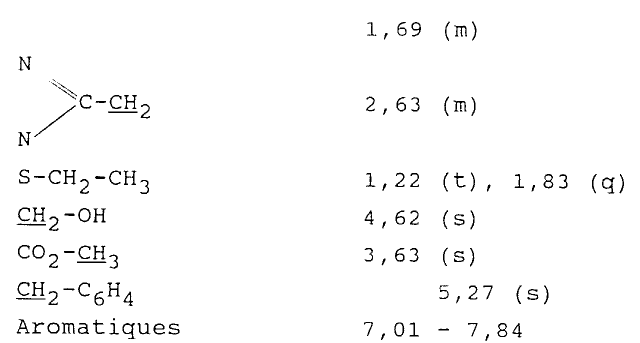

### EXEMPLE 12 : Acide 4'-[[2-butyl 5-(éthylthio) 4-(hydroxyméthyl) 1H-imidazol-1-yl] méthyl] (1,1'-biphényl)-2-carboxylique

On opère comme à l'exemple 2 à partir de 200 mg du produit obtenu à l'exemple 11. On obtient 176 mg que l'on recristallise dans 2,1 cm³ d'isopropanol contenant 0,5 cm³ d'eau. On recueille 154 mg de produit recherché. F = 208°C.

| Analyse pour C₂₄H₂₈N₂O₃S = 424,54 | | | | |
|---|---|---|---|---|
| | C | H | N | S |
| % calculés | 67,9 | 6,65 | 6,6 | 7,55 |
| % trouvés | 67,9 | 6,5 | 6,5 | 7,50 |

| Spectre de RMN : DMSO 250 MHz | |
|---|---|
| CH₃-(CH₂)₃ | 0,83 (t) |
| CH₃-(CH₂)₂-CH₂ | 1,29 (m) - 1,57 (m) |
| S-CH₂-CH₃ | 1,03 (t) |
| S-CH₂ | 2,42 (q) |
| CH₃-(CH₂)₂-CH₂ | 2,58 (m) |
| N-CH₂-C₆H₄ | 5,32 (s) |
| -CH₂-OH | 4,41 (s,l) |
| 1H mobile | 4,81 (m) |
| Aromatiques | 7,01 - 7,71 |

### EXEMPLE 13 : 4'-[[2-butyl 5-(éthylthio) 4-(hydroxyméthyl) 1H-imidazol-1-yl] méthyl] (1,1'-biphényl)-2-carboxylate de méthyle

Le produit est obtenu lors de la chromatographie à l'exemple 11.

On obtient 2,48 g de produit recherché. F = 110°C.

| Spectre de RMN : CDCl₃ 250 MHz | |
|---|---|
| CH₃-(CH₂)₃ | 0,88 (m) |
| CH₃-(CH₂)₂-CH₂ | 1,35 (m) |
| | 1,68 (m) |
| CH₃-(CH₂)₂-CH₂ | 2,63 (m) |
| 〉N-CH₂-C₆H₄ | 5,30 (s) |
| -CH₂-OH | 4,76 (s) |
| S-CH₂-CH₃ | 1,12 (t) |
| | 2,40 (q) |
| CO₂-CH₃ | 3,63 (s) |
| Aromatiques | 7,00 à 7,83 |

### EXEMPLE 14 : Acide 4'-[[2-butyl 4-(éthylthio) 5-(hydroxyméthyl) 1H-imidazol-1-yl] méthyl] (1,1'-biphényl)-2-carboxylique

On opère comme à l'exemple 2 à partir de 163 mg du produit obtenu à l'exemple 13. On obtient 130 mg de produit que l'on recristallise dans 1,5 cm³ d'isopropanol et 0,5 cm³ d'eau. On recueille 26 mg du produit recherché. F = 160°C.

| Analyse pour C₂₄H₂₈N₂O₃S = 424,57 | | | | |
|---|---|---|---|---|
| | C | H | N | S |
| % calculés | 67,89 | 6,64 | 6,59 | 7,55 |
| % trouvés | 66,8 | 6,7 | 6,5 | 7,80 |

| Spectre de RMN : DMSO 250 MHz | |
|---|---|
| CH₃-(CH₂)₃ | 0,82 (t) |
| CH₃-(CH₂)₂-CH₂ | 1,27 (m) - 1,52 (m) |
| CH₃-(CH₂)₂-CH₂ | 2,50 (masqué) |
| N-CH₂-C₆H₄ | 5,27 (s,l) |
| S-CH₂-CH₃ | 1,14 (t) - 2,70 (q) |
| -CH₂-OH | 4,44 (s,l) |
| 1H mobile | 5,20 (m, large) |
| Aromatiques | 7,02 - 7,60 |

### EXEMPLE 15 : 2-butyl 4-(éthylsulfinyl) 1-[[2'-(méthoxycarbonyl) (1,1'-biphényl)-4-yl] méthyl] 1H-imidazole-5-carboxylate d'éthyle

On agite pendant 2 heures à température ambiante 500 mg de produit obtenu à l'exemple 5 avec 5 cm³ de chlorure de méthylène et 215 mg d'acide méta-chloroperbenzoïque. On verse dans 50 cm³ d'eau, extrait avec 3 fois 50 cm³ de chlorure de méthylène, sèche, filtre et amène à sec, sous pression réduite. On chromatographie le résidu (675 mg) sur silice, (éluant : chlorure de méthylène-acétone (6-4)). On obtient 400 mg du produit recherché.

| Spectre IR : CHCl₃ | |
|---|---|
| S=O | 1038 cm⁻¹ |
| 〉C=0 | 1716 cm⁻¹ |

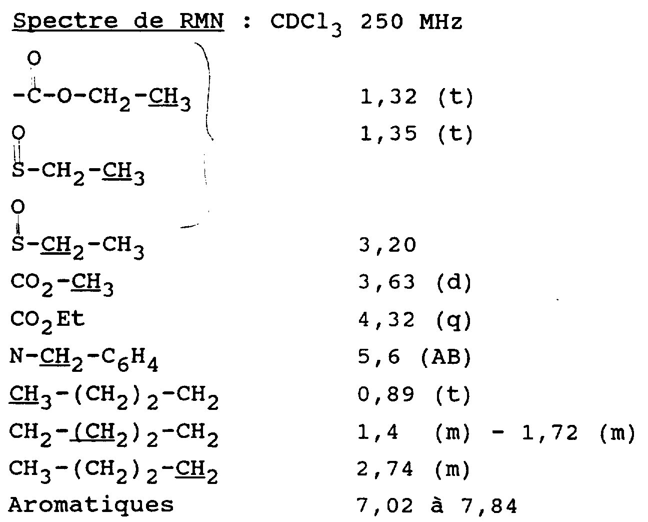

### EXEMPLE 16 : Acide 2-butyl 1-[[2'-carboxy (1,1'-biphényl)-4-yl] méthyl] 4-(éthylsulfinyl) 1H-imidazole-5-carboxylique

On opère comme à l'exemple 2, à partir de 0,2 g du produit obtenu à l'exemple 15. On obtient 154 mg de produit que l'on recristallise dans un mélange isopropanol-eau (3-7). On recueille 100 mg du produit recherché. F = 186°C.

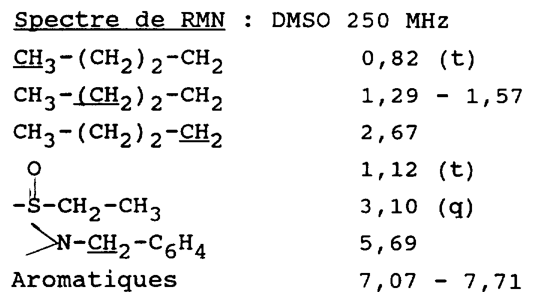

### EXEMPLE 17 : 2-butyl 4-(éthylsulfonyl) 1-[[(2'-méthoxycarbonyl) (1,1'-biphényl)-4-yl] méthyl] 1H-imidazole-5-carboxylate d'éthyle

On opère comme à l'exemple 15 à partir de 220 mg du produit obtenu à l'exemple 15 en utilisant 120 mg d'acide métachloroperbenzoïque. On obtient 400 mg de produit brut que l'on chromatographie sur silice (éluant : chlorure de méthylène-acétate d'éthyle (7-3)). On obtient 209 mg de produit recherché.

| Spectre IR : | |
|---|---|
| SO₂ | 1324 cm⁻¹ - 1136 cm⁻¹ |

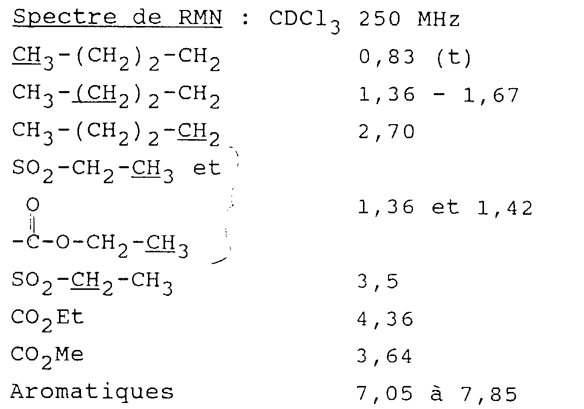

### EXEMPLE 18 : Acide 2-butyl 1-[[2'-carboxy (1,1'-biphenyl)-4-yl] méthyl] 4-(éthylsulfonyl) 1H-imidazole-5-carboxylique

On opère comme à l'exemple 2 à partir de 304 mg du produit obtenu à l'exemple 17. On obtient 210 mg de produit que l'on recristallise dans un mélange de 2 cm³ d'isopropanol et 6 cm³ d'eau. On recueille 111 mg du produit recherché. F = 192°C.

| Analyse pour C₂₄H₂₆N₂O₆S = 470,26 | | | | |
|---|---|---|---|---|
| | C | H | N | S |
| % calculés | 61,29 | 5,52 | 5,95 | 6,80 |
| % trouvés | 61,4 | 5,6 | 5,8 | 6,80 |

| Spectre de RMN : DMSO 250 MHz | |
|---|---|
| CH₃-(CH₂)₂-CH₂ | 0,82 (t) |
| CH₃-CH₂-(CH₂)₂ | 1,27 (m) |
| CH₃-CH₂-CH₂-CH₂ | 1,54 (m) |
| CH₃-(CH₂)₂-CH₂ | 2,66 (t) |
| S-CH₂-CH₃ | 1,20 (t) |
| N-CH₂-C₆H₄ | 5,55 (s,l) |
| Aromatiques | 7,10 - 7,72 |

### EXEMPLE 19 : 2-butyl 1-[[2'-(méthoxycarbonyl) (1,1'-biphenyl)-4-yl] méthyl] 4-(phénylthio) 1H-imidazole-5-carboxylate d'éthyle

On opère comme à l'exemple 5, à partir de 165 mg du produit obtenu à la préparation 2. On obtient 410 mg de produit que l'on chromatographie sur silice (éluant chlorure de méthylène-acétate d'éthyle (95-5), on recueille 163 mg de produit recherché et 102 mg de produit de l'exemple 23.

| | |
|---|---|
| CH₃-(CH₂)₂-CH₂ | 0,86 (t) |
| CH₃-CH₂-(CH₂)₂ | 1,33 (m) |
| CH₃-CH₂-CH₂-CH₂ | 1,62 (m) |
| CH₃-(CH₂)₂-CH₂ | 2,63 (t) |
| CO₂Et | 1,27 (t) 4,25 (q) |
| CO₂Me | 3,62 (s) |
| N-CH₂-C₆H₄ | 5,58 (s) |
| Aromatiques | 7,03 - 7,82 |

### EXEMPLE 20 : Acide 2-butyl 1-[[2'-carboxy (1,1'-biphényl)-4-yl] méthyl] 4-(phénylthio) 1H-imidazole-5-carboxylique

On opère comme à l'exemple 2 à partir de 122 mg du produit obtenu à l'exemple 19. On obtient 101 mg de produit que l'on recristallise dans 2 cm³ d'isopropanol et 0,4 cm³ d'eau. On recueille 86 mg de produit recherché. F = 155°C.

| Analyse pour C₂₈H₂₆N₂O₄S = 486,59 | | | | |
|---|---|---|---|---|
| | C | H | N | S |
| % calculés | 69,12 | 5,39 | 5,76 | 6,59 |
| % trouvés | 69,4 | 5,4 | 5,6 | 6,60 |

### EXEMPLE 21 : 2-butyl 1-[[2'-(méthoxycarbonyl) (1,1'-biphényl)-4-yl] méthyl] 4-(méthylthio) 1H-imidazole-5-carboxylate d'éthyle

On opère comme à l'exemple 5, à partir de 0,6 g de 2-butyl 4-(méthylthio) 1H-imidazole-5-carboxylate d'éthyle (préparé comme indiqué aux stades C et D de la préparation 1, en remplaçant l'éthanethiol par le méthanethiol), en utilisant 907 mg de 4'-(bromométhyl) (1,1'-biphényl)-2-carboxylate de méthyle. On obtient, après chromatographie sur silice (éluant : chlorure de méthylène-acétate d'éthyle (97-3)), 0,8 g du produit recherché.

| Spectre de RMN : CDCl₃ 250 MHz | |
|---|---|
| CH₃-(CH₂)₃- | 0,83 (t) |
| CH₃-CH₂-(CH₂)₂ | 1,38 (m) |
| CH₃-CH₂-CH₂-CH₂ | 1,67 (m) |
| CH₃-(CH₂)₂-CH₂ | 2,67 (t) |
| S-CH₃ | 2,61 (s) |
| CO₂Et | 1,3 (t) 4,28 (q) |
| CO₂Me | 3,62 (s) |
| N-CH₂-C₆H₄ | 5,57 (s) |
| Aromatiques | 7,03 à 7,82 |

### EXEMPLE 22 : Acide 2-butyl 1-[[2'-carboxy (1,1'-biphényl)-4-yl] méthyl] 4-(méthylthio) 1H-imidazole-5-carboxylique

On opère comme à l'exemple 2 à partir de 505 mg du produit obtenu à l'exemple 21. On obtient 395 mg de produit que l'on recristallise dans un mélange de 12 cm³ d'isopropanol et 8 cm³ d'eau. On recueille 305 mg du produit recherché. F = 220°C.

| Analyse pour C₂₃H₂₄N₂O₄S = 424,52 | | | | |
|---|---|---|---|---|
| | C | H | N | S |
| % calculés | 65,11 | 5,70 | 6,60 | 7,55 |
| % trouvés | 65,3 | 5,9 | 6,4 | 7,40 |

| Spectre de RMN : DMSO 250 MHz | |
|---|---|
| CH₃-(CH₂)₃- | 0,82 (t) |
| CH₃-(CH₂)₂-CH₂ | 1,23 - 1,54 (m) |
| CH₃-(CH₂)₂-CH₂ | 2,63 (t) |
| S-CH₃ | 2,46 (s) |
| N-CH₂-C₆H₄ | 5,6 (s) |
| Aromatiques | 7,04 à 7,70 |
| 1H mobile | 12,74 (s) |

### EXEMPLE 23 : 2-butyl 1-[[2'-(méthoxycarbonyl) (1,1'-biphényl)-4-yl] méthyl] 5-(phénylthio) 1H-imidazole-4-carboxylate d'éthyle

On opère comme à l'exemple 19 et recueille après chromatographie 102 mg du produit recherché.

| Spectre de RMN : CDCl₃ 250 MHz | |
|---|---|
| CH₃-(CH₂)₃- | 0,87 (t) |
| CH₃-CH₂-(CH₂)₂- | 1,63 (m) |
| CH₃-CH₂-CH₂-CH₂- | 1,65 (m) |
| CH₃-(CH₂)₂-CH₂ | 2,68 (m) |
| CO₂Et | 1,33 (t) - 4,39 (q) |
| CO₂CH₃ | 3,62 (s) |
| N-CH₂-C₆H₄ | 5,26 (s) |
| Aromatiques | 7,05 à 7,84 |

### EXEMPLE 24 : Acide 2-butyl 1-[[2'-carboxy (1,1'-biphényl)-4-yl] méthyl] 5-(phénylthio) 1H-imidazole-4-carboxylique

On opère comme à l'exemple 2, à partir de 160 mg du produit obtenu à l'exemple 23. On obtient 146 mg de produit que l'on recristallise dans un mélange de 3 cm³ d'isopropanol et 0,6 cm³ d'eau. On recueille 113 mg de produit recherché. F = 214°C.

| Analyse pour C₂₈H₂₆N₂O₄S = 486,59 | | | | |
|---|---|---|---|---|
| | C | H | N | S |
| % calculés | 69,12 | 5,39 | 5,76 | 6,59 |
| % trouvés | 69,3 | 5,5 | 5,6 | 6,50 |

| Spectre de RMN : DMSO 300 MHz | |
|---|---|
| CH₃-(CH₂)₃- | 0,82 (t) |
| CH₃-CH₂-(CH₂)₂- | 1,28 (m) |
| CH₃-(CH₂)-CH₂-CH₂- | 1,55 (m) |
| CH₃-(CH₂)₂-CH₂ | 2,64 (m) |
| N-CH₂-C₆H₄ | 5,31 (s,l) |
| Aromatiques | 6,96 à 7,71 |

### EXEMPLE 25 : 2-butyl 1-[[2'-(méthoxycarbonyl) (1,1'-biphényl)-4-yl] méthyl] 4-(phénylsulfonyl) 1H-imidazole-5-carboxylate d'éthyle

On opère comme à l'exemple 17 à partir de 264 mg du produit obtenu à l'exemple 19. On obtient, après chromatographie sur silice (éluant : chlorure de méthylène-acétate d'éthyle (95-5)), 176 mg du produit recherché.

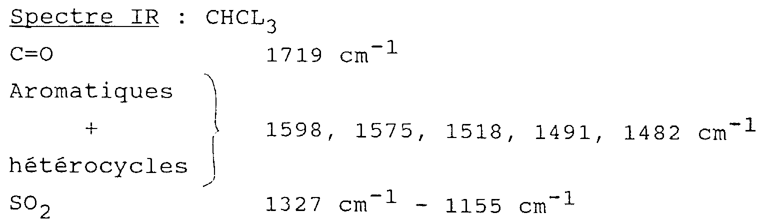

| Spectre de RMN : CDCl₃ 300 MHz | |
|---|---|
| CH₃-(CH₂)₃- | 0,86 (t) |
| CH₃-CH₂-(CH₂)₃- | 1,31 (m) |
| CH₃-CH₂-CH₂-CH₂- | 1,62 (m) |
| CH₃-(CH₂)₂-CH₂ | 2,66 (m) |
| CO₂Et | 1,31 - 4,33 (q) |
| CO₂Me | 3,61 (s) |
| N-CH₂-C₆H₄ | 5,42 (s) |
| Aromatiques | 7,04 à 8,08 |

### EXEMPLE 26 : Acide 2-butyl 1-[[2'-carboxy (1,1'-biphényl)-4-yl] méthyl] 4-(phénylsulfonyl) 1H-imidazole-5-carboxylique

On opère comme à l'exemple 2, à partir de 150 mg du produit obtenu à l'exemple 25. Après recristallisation dans l'isopropanol aqueux, on obtient 110 mg du produit recherché. F = 140°C.

| Analyse pour C₂₈H₂₆N₂O₆S = 518,59 | | | | |
|---|---|---|---|---|
| | C | H | N | S |
| % calculés | 64,85 | 5,05 | 5,40 | 6,18 |
| % trouvés | 64,3 | 5,1 | 5,3 | 6,00 |

| Spectre de RMN : DMSO 250 MHz | |
|---|---|
| CH₃-(CH₂)₃- | 0,78 (t) |
| CH₃-CH₂-(CH₂)₂- | 1,23 (m) |
| CH₃-CH₂-CH₂-CH₂- | 1,46 (m) |
| CH₃-(CH₂)₂-CH₂ | 2,58 (m) |
| N-CH₂-C₆H₄ | 5,44 (s) |
| Aromatiques | 7,10 - 7,95 |

### EXEMPLE 27 : 2-butyl 1-[[2'-(méthoxycarbonyl) (1,1'-biphényl)-4-yl] méthyl] 4-(phénylsulfinyl) 1H-imidazole-5-carboxylate d'éthyle

On opère comme à l'exemple 15, à partir de 264 mg du produit obtenu à l'exemple 19, en utilisant 101 mg d'acide métachloroperbenzoïque. Après chromatographie sur silice (éluant : chlorure de méthylène-acétate d'éthyle (9-1)). On obtient 160 mg de produit recherché.

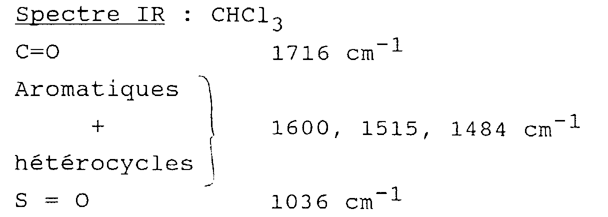

| Spectre de RMN : CDCl₃ 250 MHz | |
|---|---|
| CH₃-(CH₂)₃- | 0,84 (t) |
| CH₂-CH₂-(CH₂)₂- | 1,27 (m) |
| CH₃-CH₂-CH₂-CH₂- | 1,60 (m) |
| CH₃-(CH₂)₂-CH₂ | 2,67 (m) |
| CO₂Et | 1,37 (t) - 4,36 (q) |
| CO₂Me | 3,62 (s) |
| Aromatiques | 6,98 à 7,83 |

### EXEMPLE 28 : Acide 2-butyl 1-[[2'-carboxy (1,1'-biphényl)-4-yl] méthyl] 4-(phénylsulfinyl) 1H-imidazole-5-carboxylique

On opère comme à l'exemple 2, à partir de 138 mg du produit obtenu à l'exemple 27. On obtient après recristallisation dans l'isopropanol aqueux : 106 mg du produit recherché. F = 195°C.

| Analyse pour C₂₈H₂₆N₂O₅S = 502,59 | | | | |
|---|---|---|---|---|
| | C | H | N | S |
| % calculés | 66,91 | 5,21 | 5,57 | 6,37 |
| % trouvés | 66,4 | 5,1 | 5,5 | 6,20 |

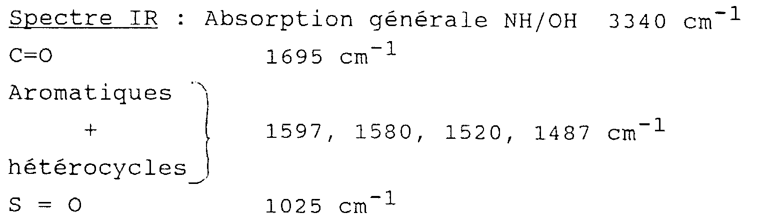

| Spectre de RMN : DMSO 250 MHz | |
|---|---|
| CH₃-(CH₂)₃- | 0,75 (t) |
| CH₂-CH₂-(CH₂)₂- | 1,19 (m) |
| CH₃-CH₂-CH₂-CH₂- | 1,43 (m) |
| CH₃-(CH₂)₂-CH₂ | 2,59 (m) |
| N-CH₂-C₆H₄ | 5,65 (système A, B) |
| Aromatiques | 7,05 à 7,70 |

### EXEMPLE 29 : 2-butyl 1-[[2'-(méthoxycarbonyl) (1,1'-biphényl)-4-yl] méthyl] 5-(phénylsulfonyl) 1H-imidazole-4-carboxylate d'éthyle

On opère comme à l'exemple 25, à partir de 264 mg du produit obtenu à l'exemple 23, en utilisant 242 mg d'acide métachloroperbenzoïque à 85 %. Après chromatographie sur silice, (éluant : chlorure de méthylène-acétate d'éthyle (9-1)). On obtient 216 mg de produit recherché.

| Spectre de RMN : CDCl₃ 250 MHz | |
|---|---|
| CH₃-(CH₂)₃- | 0,87 (t) |
| CH₃-CH₂-(CH₂)₂- | 1,34 (m) |
| CH₃-CH₂-CH₂-CH₂- | 1,66 (m) |
| CH₃-(CH₂)₂-CH₂ | 2,65 (m) |
| N-CH₂-C₆H₄ | 5,63 (s) |
| CO₂CH₃ | 3,66 (s) |
| CO₂Et | 1,42 (t) 4,46 (q) |
| H aromatiques | 6,88 à 7,87 |

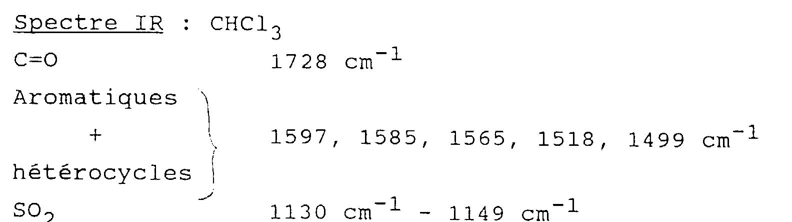

### EXEMPLE 30 : Acide 2-butyl 1-[[2'-carboxy (1,1'-biphényl)-4-yl] méthyl] 5-(phénylsulfonyl) 1H-imidazole-4-carboxylique

On opère comme à l'exemple 2, à partir de 190 mg du produit obtenu à l'exemple 29. On obtient 160 mg d'un produit que l'on recristallise dans le méthanol aqueux, on recueille 135 mg du produit recherché. F = 155°C.

| Analyse pour C₂₈H₂₆N₂O₆S = 518,59 | | | | |
|---|---|---|---|---|
| | C | H | N | S |
| % calculés | 64,85 | 5,05 | 5,40 | 6,18 |
| % trouvés | 65,0 | 5,1 | 5,4 | 6,10 |

| Spectre de RMN : DMSO 250 MHz | |
|---|---|
| CH₃-(CH₂)₃- | 0,78 (t) |
| CH₃-(CH₂)₂-CH₂ | 1,24 - 1,51 (m) |
| CH₃-(CH₂)₂-CH₂ | 2,56 (m) |
| N-CH₂-C₆H₄ | 5,58 (s) |
| Aromatiques | 6,82 à 7,89 |

En plus des produits décrits dans les exemples ci-dessus, qui illustrent l'invention sans toutefois la limiter, les produits répondant à la formule (I) telle que définie ci-dessus dans laquelle :
- R₁ représente un radical butyle,
- Y représente un radical biphényle substitué en ortho du radical phényle non lié au radical CH₂ par un radical carboxy libre ou estérifié ou un radical tétrazolyle,
- et R₂ et R₃, identiques ou différents, sont choisis parmi les radicaux suivants :
   mercapto, methyltio, propylthio, butylthio, allylthio, fluorométhylthio, trifluorométhylthio, 2,2,2-trifluoro éthylthio, 3,3,3-trifluoro propylthio, vinylthio, 2-fluoro éthylthio, phénylthio, benzylthio, 4-hydroxy benzylthio, 4-(trifluorométhyl) benzylthio, 2-pyridylthio, (2-pyridyl) méthylthio, (4-méthyl 1-pipérazinyl) méthyl thio, 2-(4-morpholinyl) éthylthio, 2-[4-(3-méthoxy phényl) 1-pipérazinyl] éthylthio, 2-(benzyloxy) éthylthio, 2-méthoxy éthylthio, amino méthylthio, 2-amino éthylthio (méthylamino) méthylthio, (diéthylamino) méthylthio, hydroxy méthylthio, 2-hydroxy éthylthio, carboxy méthylthio, (éthoxycarbonyl) méthylthio, (tert-butoxycarbonyl) méthylthio, 2-carboxy éthylthio, méthyl sulfinyle, éthylsulfinyle, propylsulfinyle, butylsulfinyle, phénylsulfinyle, méthylsulfonyle, éthylsulfonyle, chlorosulfonyle, phénylsulfonyle, aminosulfonyle, fluorométhylsulfonyle, 2-(triméthylsilyl) éthylsulfonyle, 2-fluoro éthylsulfonyle, carboxy, éthoxycarbonyle, carboxyméthyle, hydroxyméthyle, formule, acétoxyméthyle, N-tosyl méthylsulfonimidoyle ou N-tosyl phénylsulfonimidoyle, constituent des produits pouvant être obtenus dans le cadre de la présente invention.

### EXEMPLE 31 : 4'-[[2-butyl 5-(éthylsulfinyl) 4-(hydroxyméthyl) 1H-imidazol-1-yl] méthyl] (1,1'-biphényl)-2-carboxylate de méthyle

On opère comme à l'exemple 15 en utilisant au départ 500 mg de produit obtenu comme à l'exemple 13 dans 7,5 cm³ de chlorure de méthylène et 239 mg d'acide métachloroperbenzoïque. On obtient 287 mg de produit attendu.

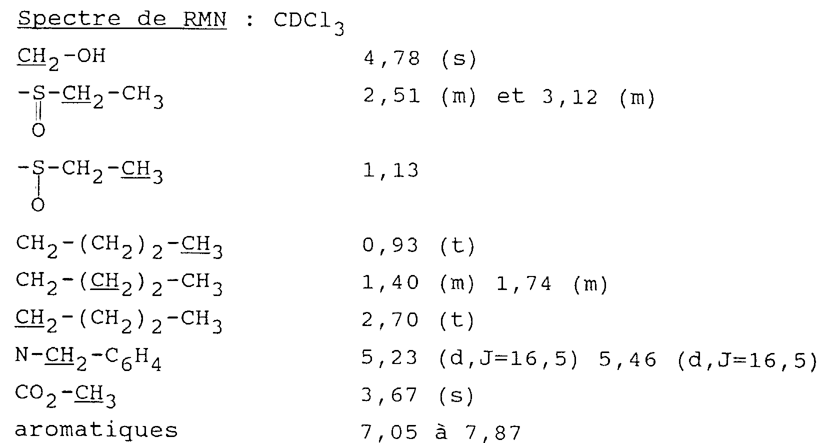

### EXEMPLE 32 : Acide 4'-[[2-butyl 5-(éthylsulfinyl) 4-(hydroxyméthyl) 1H-imidazol-1-yl] méthyl] (1,1'-biphényl)-2-carboxylique

On opère comme à l'exemple 2 à partir de 287 mg du produit obtenu à l'exemple 31. On obtient 170 mg de produit attendu. F = 240°C.

| Analyse pour C₂₄H₂₈N₂O₄S = 440,27 | | | | |
|---|---|---|---|---|
| | C | H | N | S |
| % calculés | 65,40 | 6,40 | 6,36 | 7,26 |
| % trouvés | 65,5 | 6,4 | 6,5 | 7,40 |

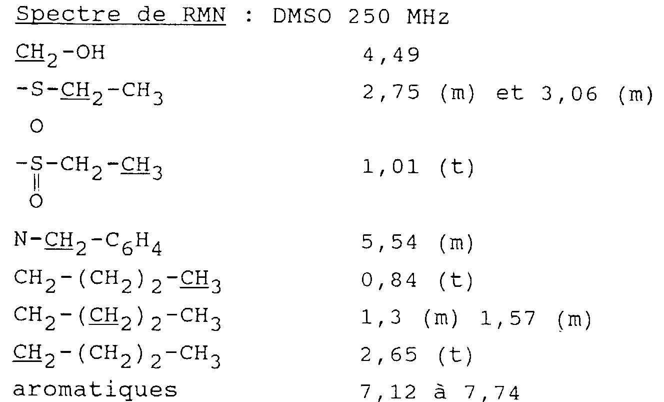

### EXEMPLE 33 : 4'-[[2-butyl 5-(éthylsulfonyl) 4-(hydroxyméthyl) 1H-imidazol-1-yl] méthyl] (1,1'-biphényl)-2-carboxylate de méthyle

On opère comme à l'exemple 15 en utilisant au départ 500 mg de produit obtenu comme à l'exemple 13 dans 5 cm³ de chlorure de méthylène et 474 mg d'acide métachloroperbenzoïque. On obtient 328 mg de produit attendu.

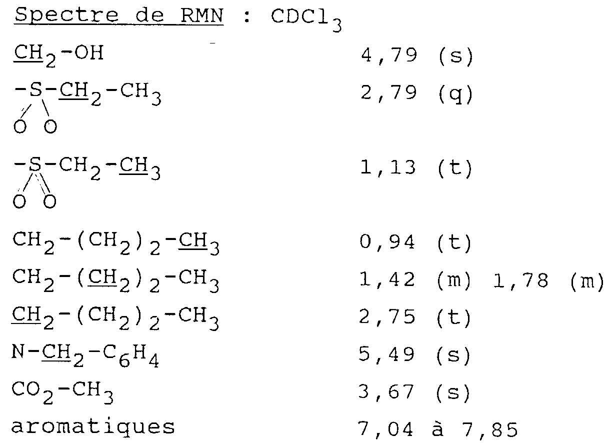

### EXEMPLE 34 : Acide 4'-[[2-butyl 5-(éthylsulfonyl) 4-(hydroxyméthyl) 1H-imidazol-1-yl] méthyl] (1,1'-biphényl)-2-carboxylique

On opère comme à l'exemple 2 à partir de 300 mg du produit obtenu à l'exemple 33. On obtient 160 mg de produit attendu. F = 210°C.

| Analyse pour C₂₄H₂₈N₂O₅S = 456,27 | | | | |
|---|---|---|---|---|
| | C | H | N | S |
| % calculés | 63,17 | 6,18 | 6,13 | 7,02 |
| % trouvés | 63,5 | 6,2 | 6,2 | 7.0 |

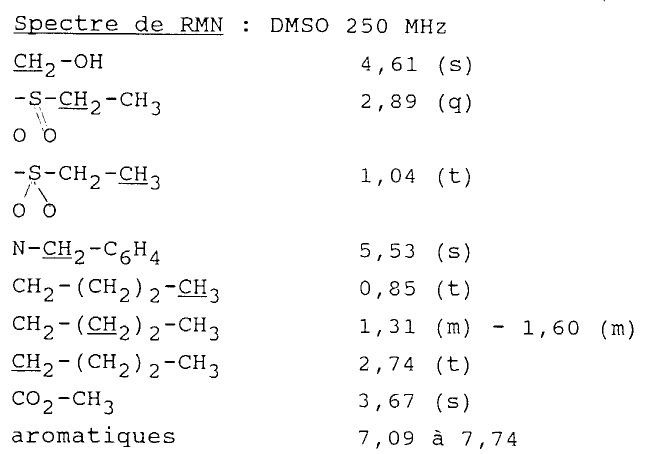

### EXEMPLE 35 : 2-butyl 4-(méthylsulfonyl) 1-[[2'-(méthoxycarbonyl) (1,1'-biphényl)-4-yl] méthyl] 1H-imidazole-5-carboxylate d'éthyle

On opère comme à l'exemple 15 à partir de 244 mg du produit obtenu à l'exemple 21 en utilisant 222 mg d'acide méta-chloroperbenzoïque. On obtient 480 mg de produit brut que l'on chromatographie sur silice (éluant : chlorure de méthylène-acétate d'éthyle (7-3)). On obtient 300 mg de produit recherché.

| Spectre IR : | |
|---|---|
| SO₂ | 1318 cm⁻¹ - 1157 cm⁻¹ |

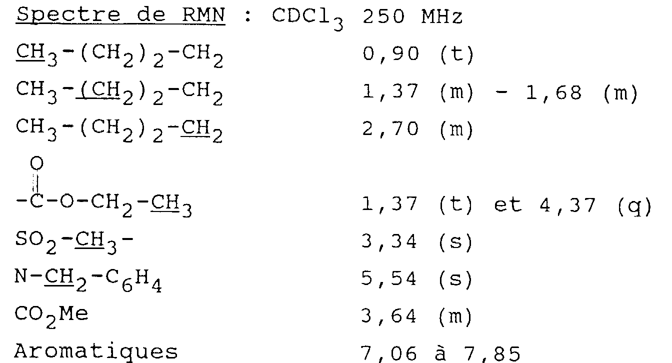

### EXEMPLE 36 : Acide 2-butyl 1-[[2'-carboxy (1,1'-biphényl)-4-yl] méthyl] 4-(méthylsulfonyl) 1H-imidazole-5-carboxylique

On opère comme à l'exemple 2 à partir de 217 mg du produit obtenu à l'exemple 35. On obtient 210 mg de produit que l'on recristallise dans un mélange de (80-20) d'isopropanol et d'eau. On recueille 150 mg du produit recherché. F = 196°C.

| Analyse pour C₂₃H₂₄N₂O₆S = 456,26 | | | | |
|---|---|---|---|---|
| | C | H | N | S |
| % calculés | 60,5 | 5,25 | 6,13 | 7,00 |
| % trouvés | 60,8 | 5,3 | 6,1 | 6,90 |

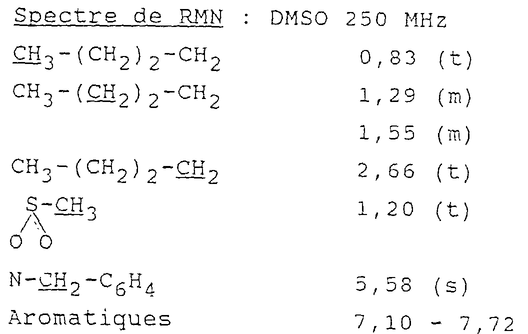

### EXEMPLE 37 : 4-[2-(acétyloxy) éthylthio] 2-butyl 1-[[2'-(méthoxycarbonyl) (1,1'-biphényl)-4-yl] méthyl] 1H-imidazole-5-carboxylate d'éthyle

On opère comme à l'exemple 5 à partir de 6,49 g de 4-[2-(acétyloxy) éthylthio] 2-butyl 1H-imidazole-5-carboxylate d'éthyle obtenu comme indiqué à la préparation ci-dessous et 7,56 g de dérivé bromé. On obtient 10,31 g de produit attendu après chromatographie sur silice (éluant : chlorure de méthylène-acétone (98-2)).

| Spectre de RMN : | |
|---|---|
| CH₃-(CH₂)₂-CH₂ | 0,89 (t) |
| CH₃-(CH₂)₂-CH₂ | 1,33 (m) 1,67 (m) |
| CH₃-(CH₂)₂-CH₂ | 2,63 (m) |
| CO-CH₃ (imidazole) | 2,08 (s) |
| CO₂-CH₂-CH₃ | 1,33 (t) |
| CO₂-CH₂-CH₃ | 4,27 (q) |
| S-CH₂-CH₂-O-CO- | 3,44 (t) |
| S-CH₂-CH₂-O-CO | 4,39 (t) |
| CO₂-CH₃ (phényle) | 3,62 (s) |
| N-CH₂-C₆H₄ | 5,55 (s) |
| Aromatiques | 7, 02 à 7,81 |

### Préparation du 4-[2-(acétyloxy) éthylthio] 2-butyl 1H-imidazole-5-carboxylate d'éthyle utilisé au départ de l'exemple 37

### STADE A : 2-(acétyloxy) éthylthio

On ajoute lentement à une température inférieure à 40°C, de l'anhydride acétique dans un mélange comprenant 55 g de mercaptoéthanol et 2,2 cm³ d'une solution à 10 % d'acide sulfurique dans l'acide acétique. On chauffe le mélange à 60°C pendant 1 heure puis abandonne 20 heures à température ambiante. On ajoute 500 cm³ d'éther, lave à l'eau, sèche, évapore les solvants. Après distillation du résidu à 62-64°C (16 mmHg) on obtient 75,78 g de produit attendu.

### STADE B : 3-amino 3-(2-acétyloxy éthylthio) 2-[(1-oxo pentyl) amino] propènoate d'éthyle.

On dissout 14 g de produit obtenu au stade B de la préparation 1 dans 300 cm³ d'éthanol, ajoute goutte à goutte 668 mg de triéthylamine puis 9,52 g de produit obtenu au stade A ci-dessus. On abandonne 5 jours à température ambiante, élimine le solvant sous pression réduite à température ambiante, reprend le résidu huileux dans l'éther isopropylique, laisse cristalliser à 4°C, essore et sèche le produit obtenu. On recueille 13,26 g de produit attendu. F = 84°C.

### STADE C : 4-[2-(acétyloxy) éthylthio] 2-butyl 1H-imidazole-5-carboxylate d'éthyle

On opère comme indiqué au stade D de la préparation 1 en utilisant 13,22 g de produit obtenu au stade B. On obtient 6,55 g de produit attendu. F = 68°C.

| Spectre de RMN : | |
|---|---|
| CH₃-(CH₂)₂-CH₂ | 0,93 (t) |
| CH₃-(CH₂)₂-CH₂ | 1,39 (m) 1,71 (m) |
| CH₃-(CH₂)₂-CH₂ | 2,71 (t) |
| CO₂-CH₂-CH₃ | 1,39 (t) 4,35 (q) |
| S-CH₂-CH₂-O-CO- | 3,41 (m) |
| S-CH₂-CH₂-O-CO | 4,35 (m) |
| O-CO-CH₃ | 2,08 (s) |
| NH- | 9,62 |

### EXEMPLE 38 : Acide 2-butyl-1-[[2'-carboxy (1,1'-biphényl)-4-yl] méthyl] 4-(2-hydroxy éthylthio) 1H-imidazole-5-carboxylique

On opère comme à l'exemple 2 en utilisant au départ 254,4 mg de produit obtenu à l'exemple 37. On obtient 147,4 mg de produit brut que l'on recristallise dans 2 cm³ d'isopropanol et 1 cm³ d'eau. On recueille ainsi 120,7 mg de produit attendu. F = 194°C.

| Spectre de RMN : | |
|---|---|
| CH₃-(CH₂)₂-CH₂ | 0,89 (t) |
| CH₃-(CH₂)₂-CH₂ | 1,36 (m) |
| CH₃-CH₂-CH₂-CH₂ | 1,68 (m) |
| CH₃-(CH₂)₂-CH₂ | 2,71 (t) |
| C-S-(CH₂)₂-O | 3,30 (m) 3,98 (m) |
| CO₂Me | 5,59 (s) |
| N-CH₂-C₆H₄ | 5,59 (s) |
| Aromatiques | 7,03 - 7,84 |

### EXEMPLE 39 : 4'-[[2-butyl 4-(hydroxyméthyl) 5-(phénylthio) 1H-imidazol-1-yl] méthyl] (1,1'-biphényl)-2-carboxylate de méthyle

On opère comme à l'exemple 1 à partir de 474 mg de produit obtenu à la préparation indiquée ci-dessous et 610 mg de dérivé bromé, on obtient après chromatographie sur silice (éluant : chlorure de méthylène-acétate d'éthyle (6-4)) 530 mg de produit attendu isomère 4-hydroxyméthyl 5-phénylthio et 225 mg d'isomèe 5-hydroxyméthyl 4-phénylthio corespondant à l'exemple 41 isomère 4-hydroxyméthyl 5-phénylthio.

| Spectre de RMN : DMSO 250 MHz | |
|---|---|
| CH₃-(CH₂)₂-CH₂ | 0,88 (t) |
| CH₃-(CH₂)₂-CH₂ | 1,37 (m) |
| | 1,67 (m) |
| CH₃-(CH₂)₂-CH₂ | 2,65 (t) |
| N-CH₂-C₆H₄ | 5,15 (s) |
| CH₂OH | 4,77 (s) |
| CO₂-CH₃ | 3,62 (s) |
| Aromatiques | 6,92 à 7,55 (m) 7,82 (dd) |

### Préparation du 2-butyl 4-phénylthio 1H-imidazole-5-méthanol

On opère comme à la préparation 3 en utilisant 609 mg de l'imidazole préparé au stade B de la préparation 2 et 5 cm³ d'une solution 1,2 M d'hydrure de diisobutylaluminium dans le toluène. On obtient 474 mg de produit attendu. F = 145°C.

### EXEMPLE 40 : Acide 4'-[[2-butyl 4-(hydroxyméthyl) 5-(phénylthio) 1H-imidazol-1-yl] méthyl] (1,1'-biphényl)-2-carboxylique

On opère comme à l'exemple 2 à partir de 508 mg de produit obtenu comme à l'exemple 39 (isomère 5-phénylthio) on obtient 386 mg de produit attendu. F = 220°C.

| Spectre de RMN : DMSO 250 MHz | |
|---|---|
| CH₃-(CH₂)₃ | 0,82 (t) |
| CH₃-(CH₂)₂-CH₂ | 1,28 (m) - 1,56 (m) |
| CH₃-(CH₂)₂-CH₂ | 2,60 (m) |
| 〉N-CH₂-C₆H₄ | 5,17 (s) |
| CH₂-OH | 4,44 (d, s après échange) |
| CO₂-H | 12,75 (m) |
| Aromatiques | 6,90 à 7,72 |

### EXEMPLE 41 : 4'-[[2-butyl 5-(hydroxyméthyl) 4-(phénylthio) 1H-imidazol-1-yl] méthyl] (1,1'-biphényl)-2-carboxylate de méthyle

Le produit est obtenu lors de la chromatographie à l'exemple 39.

On obtient 225 mg de produit recherché.

| Spectre de RMN : CDCL₃ 250 MHz | |
|---|---|
| CH₃-(CH₂)₃ | 0,88 (t) |
| CH₃-(CH₂)₂-CH₂ | 1,35 (m) - 1,70 (m) |
| CH₃-(CH₂)₂-CH₂ | 2,65 (m) |
| 〉N-CH₂-C₆H₄ | 5,32 (s) |
| CH₂-OH | 4,60 (s) |
| CO₂-CH₃ | 3,62 (s) |
| Aromatiques | 7,00 à 7,55 ; 7,84 (dd) |

### EXEMPLE 42 : Acide 4'-[[2-butyl 5-(hydroxyméthyl) 4-(phénylthio) 1H-imidazol-1-yl] méthyl] (1,1'-biphényl)-2-carboxylique

On opère comme à l'exemple 2 à partir de 183 mg du produit obtenu à l'exemple 41. On obtient 177 mg de produit que l'on recristallise dans 1,77 cm³ d'isopropanol et 0,17 cm³ d'eau. On recueille 103 mg du produit recherché. F = 215°C.

| Analyse pour C₂₄H₂₈N₂O₃S = 424,57 | | | | |
|---|---|---|---|---|
| | C | H | N | S |
| % calculés | 71,16 | 5,97 | 5,92 | 6,78 |
| % trouvés | 72,2 | 6,1 | 5,6 | 6,5 |

| Spectre de RMN : DMSO 250 MHz | |
|---|---|
| CH₃-(CH₂)₃ | 0,80 (t) |
| CH₃-(CH₂)₂-CH₂ | 1,26 (m) - 1,52 (m) |
| CH₃-(CH₂)₂-CH₂ | 2,54 (masqué) |
| N-CH₂-C₆H₄ | 5,38 (s) |
| -CH₂-OH | 4,49 (d,s après échange) |
| CO₂-H | 12,75 (s,l) |
| Aromatiques | 7,10 à 7,75 (m) |

### EXEMPLE 43 : 2-butyl 1-[[2'-carboxy (1,1'-biphényl)-4-yl] méthyl] 4-(2-fluoro éthylthio) 1H-imidazole-5-carboxylate d'éthyle

### STADE A : 2-butyl 1-[[2'-carboxy (1,1'-biphényl)-4-yl] méthyl] 4-[2-hydroxy éthylthio] 1H-imidazole-5-carboxylate d'éthyle

On dissout 10,17 g de produit obtenu à l'exemple 37 dans 50 cm³ d'éthanol, ajoute lentement 47 cm³ de carbonate de potassium (1M) et agite 3 heures à température ambiante. On ajoute 1 1 d'eau, extrait à l'acétate d'éthyle, lave les extraits organiques à l'eau salée, sèche et élimine le solvant sous pression réduite. Après chromatographie sur silice (éluant acétate d'éthyle-chlorure de méthylène (15-75)). On obtient 8,3 g de produit attendu.

| Spectre de RMN : CDCl₃ 250 MHz | |
|---|---|
| CH₃-(CH₂)₃ | 0,90 (t) |
| CH₃-(CH₂)₂-CH₂ | 1,34 (m) 1,70 (m) |
| CH₃-(CH₂)₂-CH₂ | 2,64 (m) |
| CO₂-CH₂-CH₃ | 1,34 (t) 4,30 (q) |
| S-(CH₂)₂-O | 3,29 (m) 4,02 (m) |
| CO₂-CH₃ | 3,62 (s) |
| N-CH₂-C₆H₄ | 5,57 (s) |
| Aromatiques | 7,04 à 7,82 |

### STADE B : 2-butyl 1-[[2'-carboxy (1,1'-biphényl)-4-yl] méthyl] 4-(2-fluoro éthylthio) 1H-imidazole-5-carboxylate d'éthyle

On refroidit à -78°C, 779,5 mg de trifluorure de diéthyl amino sulfure dans 50 cm³ de chlorure de méthylène. On ajoute 2 g de produit obtenu au stade A dans 50 cm³ de chlorure de méthylène, laisse revenir à température ambiante, ajoute 1 1 d'eau glacée et extrait à l'acétate d'éthyle. On élimine le solvant sous pression réduite, chromatographie le résidu sur silice (éluant acétone-chlorure de méthylène (2-98)). On obtient 1,06 g de produit attendu. F = 79°C.

| Spectre de RMN : CDCl₃ | |
|---|---|
| CH₃-(CH₂)₃ | 0,90 (t) |
| CH₃-(CH₂)₂-CH₂ | 1,33 (m) 1,67 (m) |
| CH₃-(CH₂)₂-CH₂ | 2,63 (m) |
| CO₂-CH₂-CH₃ | 1,33 (t) 4,27 (q) |
| CO₂-CH₃ | 3,62 (s) |
| N-CH₂-C₆H₄ | 5,55 (s) |
| S-CH₂- | 3,51 (dt, J=18 et 7) |
| CH₂-F | 4,70 (dt, J=47 et 7) |
| Aromatiques | 7,03 à 7,82 |

### EXEMPLE 44 : Acide 2-butyl 1-[[2'-carboxy (1,1'-biphényl)-4-yl] méthyl] 4-(2-fluoroéthylthio) 1H-imidazole-5-carboxylique

On ajoute à température ambiante 2,25 cm³ d'une solution 2N d'hydroxyde de sodium à 450 mg du produit obtenu à l'exemple 43 dissous dans 10 cm³ d'éthanol, puis abandonne 3 jours. On évapore les solvants sous pression réduite, reprend le résidu dans l'eau, ajoute 2,25 cm³ d'acide chlorhyddrique 2M/l. On filtre le précipité formé, le lave à l'eau, le sèche sous pression réduite et obtient 380 mg de produit attendu que l'on recristallise dans l'isopropanol. F = 185-186°C.

| Analyse pour C₂₄H₂₅FN₂O₄S = 456,54 | | | | |
|---|---|---|---|---|
| | C | H | F | N |
| % calculés | 63,14 | 5,52 | 4,16 | 6,13 |
| % trouvés | 63,40 | 5,60 | 4,10 | 6,10 |

### EXEMPLE 45 : 2-butyl 1-[[2'-carboxy (1,1'-biphényl)-4-yl] méthyl] 4-(méthylsulfinyl) 1H-imidazole-5-carboxylate d'éthyle

On opère comme à l'exemple 15 à partir de 1,55 g du produit obtenu à l'exemple 21 en utilisant 213 mg d'acide métachloroperbenzoïque. On obtient 1,8 g de produit brut que l'on chromatographie sur silice (éluant : chlorure de méthylène-acétone (7-3)). On obtient 1,5 g de produit recherché.

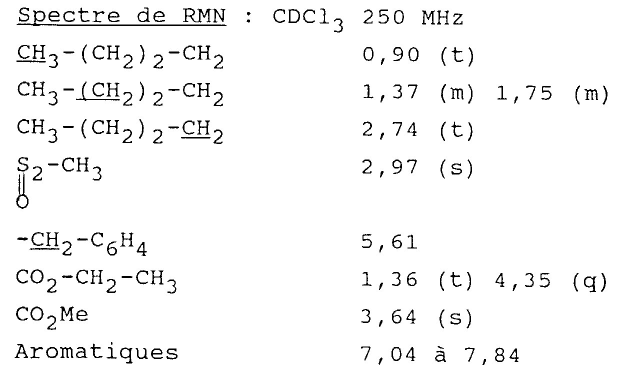

### EXEMPLE 46 : Acide 2-butyl 1-[[2'-carboxy (1,1'-biphényl)-4-yl] méthyl] 4-(méthylsulfinyl) 1H-imidazole-5-carboxylique

On opère comme à l'exemple 2 à partir de 380 mg du produit obtenu à l'exemple 45. On obtient 360 mg de produit que l'on recristallise dans un mélange de 3 cm³ d'isopropanol et 1 cm³ d'eau. On recueille 300 mg du produit recherché. F = 208°C.

| Analyse pour C₂₃H₂₄N₂O₅S = 440,52 | | | | |
|---|---|---|---|---|
| | C | H | N | S |
| % calculés | 61,7 | 5,45 | 6,36 | 7,26 |
| % trouvés | 63,0 | 5,5 | 6,3 | 7,20 |

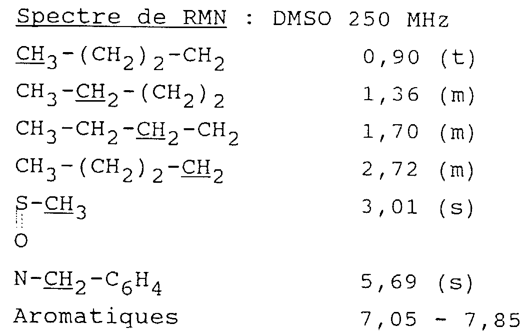

### EXEMPLE 47 : 2-butyl 1-[[2'-(méthoxycarbonyl) (1,1'-biphényl)-4-yl] méthyl] 4-(phénylméthylthio) 1H-imidazole-5-carboxylate d'éthyle et l'isomère 5-(phénylméthylthio) correspondant

On opère comme à l'exemple 5, à partir de 172 mg du produit obtenu à la préparation ci-dessous. On obtient 34 mg de produit que l'on chromatographie sur silice (éluant chlorure de méthylène-acétate d'éthyle (95-5), on recueille 250 mg de produit recherché et 30 mg de l'isomère 5-phénylthio correspondant.

| Spectre de RMN : CDCl₃ 300 MHz | |
|---|---|
| 1 - Isomère 4-(phénylméthylthio) | |
| CH₃-(CH₂)₂-CH₂ | 0,91 (t) |
| CH₃-CH₂-(CH₂)₂ | 1,36 (m) |
| CH₃-CH₂-CH₂-CH₂ | 1,71 (m) |
| CH₃-(CH₂)₂-CH₂ | 2,67 (m) |
| CO₂Et | 1,29 (t) 4,24 (q) |
| CO₂Me | 3,61 (s) |
| S-CH₂-C₆H₄ | 4,45 (d) |
| N-CH₂-C₆H₄ | 5,55 (s) |
| Aromatiques | 7,01 - 7,82 |

| 2 - Isomère 5-(phénylméthylthio) | |
|---|---|
| CH₃-(CH₂)₂-CH₂ | 0,87 (t) |
| CH₃-CH₂-(CH₂)₂ | 1,29 (m) |
| CH₃-CH₂-CH₂-CH₂ | 1,54 (m) |
| CH₃-(CH₂)₂-CH₂ | 2,55 (m) |
| CO₂Et | 1,47 (t) 4,48 (q) |
| CO₂Me | 3,60 (s) |
| S-CH₂-C₆H₄ | 4,02 (s) |
| N-CH₂-C₆H₄ | 4,74 (s) |
| Aromatiques | 6,83 - 7,82 |

### EXEMPLE 48 : Acide 2-butyl 1-[[2'-carboxy (1,1'-biphényl)-4-yl] méthyl] 4-[(phénylméthyl) thio] 1H-imidazole-5-carboxylique

On introduit 214 mg du produit obtenu à l'exemple 47, dans 4,28 ml d'éthanol et 0,78 ml de soude (2N), amène au reflux et agite durant 3 heures.

On concentre sous pression réduite, dissout dans 5 ml d'eau, neutralise par addition de 0,78 ml d'acide chlorhydrique 2N, agite environ 15 minutes, essore et rince par de l'eau.

On recristallise dans 1 ml d'isopropanol à chaud additionné de 0,5 ml d'eau, laisse plusieurs heures au repos et essore. On obtient 170 mq du produit attendu. F = 190°C.

| Microanalyse : | | | | |
|---|---|---|---|---|
| | C | H | N | S |
| % calculés | 69,58 | 5,64 | 5,6 | 6,4 |
| % trouvés | 69,5 | 5,7 | 5,7 | 6,4 |

| Spectre IR dans NUJOL | |
|---|---|
| C=O | 1662 cm⁻¹ |
| Systèmes | 1610 cm⁻¹ |
| conjugués | 1574 cm⁻¹ |
| + aromatiques | 1500 cm⁻¹ |

### EXEMPLE 49 : 2-butyl 4-(méthylthio) 1-[[2'-cyano (1,1'-biphényl)-4-yl] méthyl] 1H-imidazole-5-carboxylate d'éthyle

On introduit 0,5 g de 2-butyl 4-(méthylthio) 1H-imidazole 5-carboxylate d'éthyle dans 7,5 ml de diméthylformamide, 0,313 g de bicarbonate de potassium et 0,616 g de 4'-(bromométhyl) (1,1'-biphényl) 2-carbonitrile et laisse à température ambiante pendant environ 24 heures.

On verse dans 50 ml d'eau et 50 ml d'acétate d'éthyle, décante, réextrait avec 2 x 50 ml d'acétate d'éthyle, lave avec 2 x 100 ml d'eau, sèche, filtre et concentre sous pression réduite.

Après chromatographie (éluant : chlorure de méthylène-acétate d'éthyle (95-5)), on sèche et obtient 0,737 g de produit attendu.

| Spectre IR dans chloroforme | |
|---|---|
| -C≡O | 2226 cm⁻¹ |
| C=O | 1690 cm⁻¹ |
| aromatiques | 1600 - 1597 - 1560 - 1509 - 1500 cm⁻¹ |

### EXEMPLE 50 : 2-butyl 4-(méthylthio) 1-[[2'-(1H-tétrazol-5-yl) (1,1'-biphényl)-4-yl] méthyl] 1H-imidazole-5-carboxylate d'éthyle

On introduit 1 g du produit obtenu à l'exemple 49 dans 20 ml de xylène et environ 1,3 à 1,7 g d'azoture de triméthylétain.

Après environ 40 à 48 heures à une température d'environ 115°C, on sèche, reprend par 50 ml d'eau, extrait avec 3 x 100 ml de chlorure de méthylène, sèche, filtre et sèche à nouveau.

Après chromatographie (éluant : chlorure de méthylène-méthanol (85-15)), on sèche sous pression réduite et obtient 858 mg de produit attendu.

| Spectre IR dans chloroforme | |
|---|---|
| =C-NH | 3410 cm⁻¹ |
| C=O | 1688 cm⁻¹ |
| Système conjugué | 1615 - 1600 - 1575 cm⁻¹ |
| + aromatique | 1538 - 1509 cm⁻¹ |

### EXEMPLE 51 : Acide 2-butyl 4-(méthylthio) 1-[[2'-(1H-tétrazol-5-yl) (1,1'-biphényl)-4-yl] méthyl] lH-imidazole-5-carboxylique

On introduit 0,9 g du produit obtenu à l'exemple 50 dans 9 ml d'éthanol et ajoute 2,85 ml de soude (2N).

On laisse environ 1 heure au reflux, sèche, reprend par 7 ml d'eau et ajoute 2,85 ml d'acide chlorhydrique 2N.

On filtre, lave à l'eau et sèche. On reprend par 23 ml d'isopropanol à chaud, ajoute à chaud 25 ml d'eau et laisse environ 48 heures à environ 0°C. On filtre, lave à l'eau et sèche.

On obtient 716 mg de produit attendu. F = 180°C.

| Microanalyse : | | | | |
|---|---|---|---|---|
| | C | H | N | S |
| % calculés | 61,58 | 5,39 | 18,73 | 7,14 |
| % trouvés | 61,7 | 5,3 | 18,4 | 7,1 |

| Spectre IR dans NUJOL | |
|---|---|
| Aromatiques | 1608 - 1516 cm⁻¹ |
| Hétéroatome | 1482 cm⁻¹ |

Les produits décrits dans le tableau ci-dessous qui constituent les exemples 52 à 113 répondent à la formule : 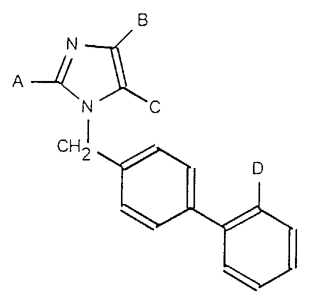 dans laquelle A, B et C ont les significations indiquées dans le tableau ci-dessous et D représente les valeurs, 1, 2, 3, 4, 5 et 6 telles que 1 représente -CO₂Me, 2 représente -COOH, 3 représente -C≡N, 4 représente 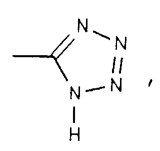 5 représente le radical tétrazole salifié et 6 représente -CO₂tBu.

Ces produits ont été obtenus selon les mêmes procédés que ceux indiqués ci-dessus. F°C désigne le point de fusion des produits.

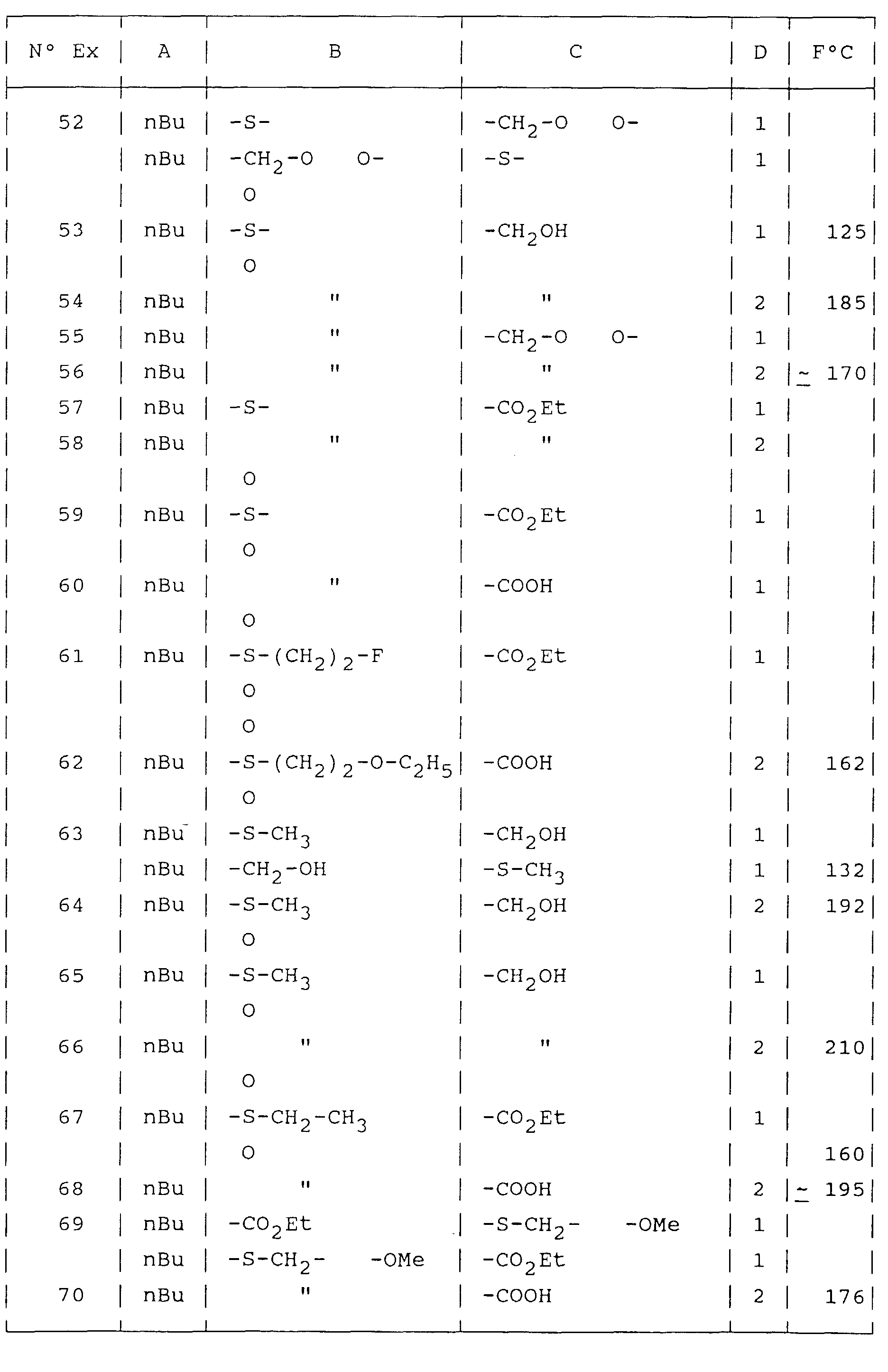

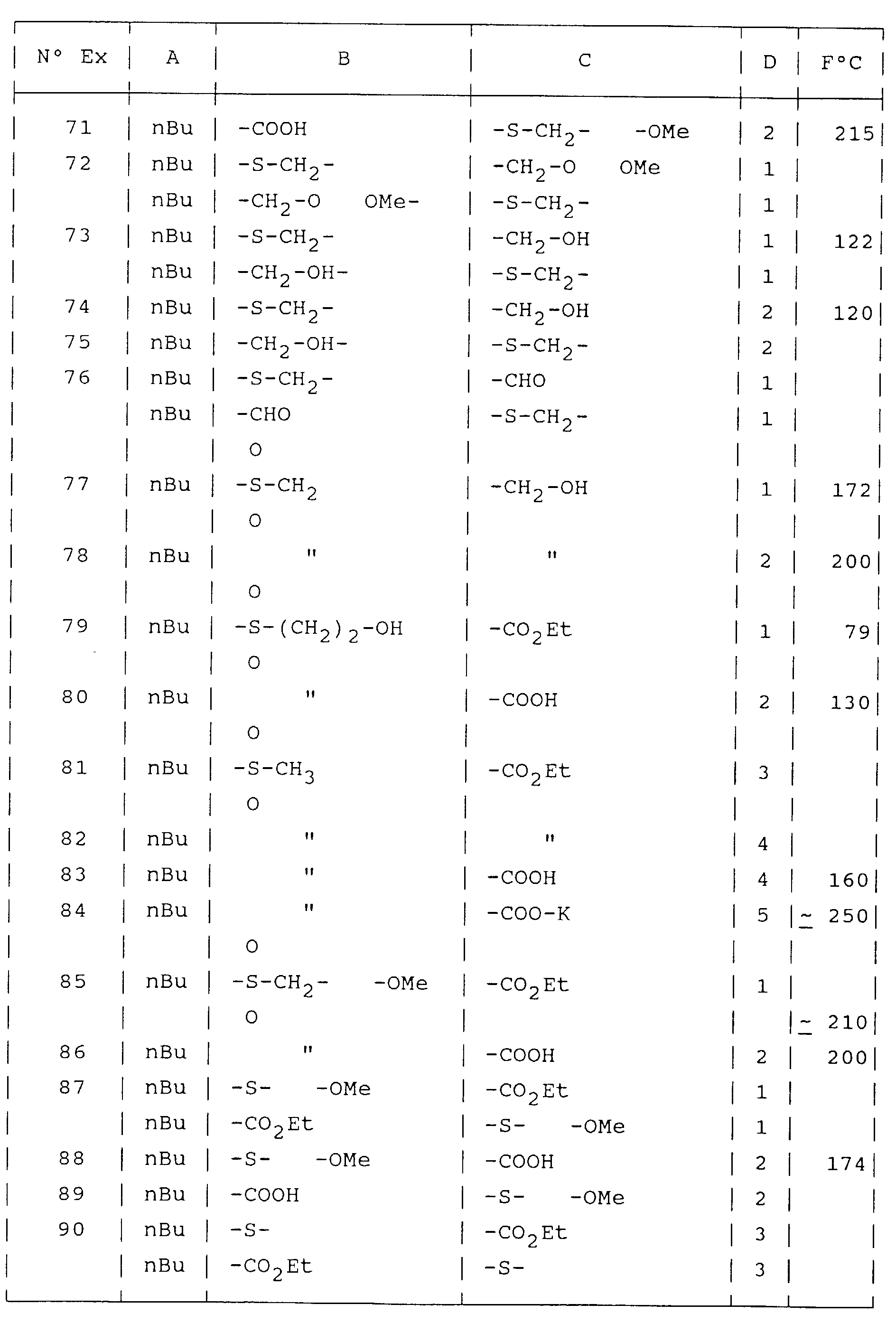

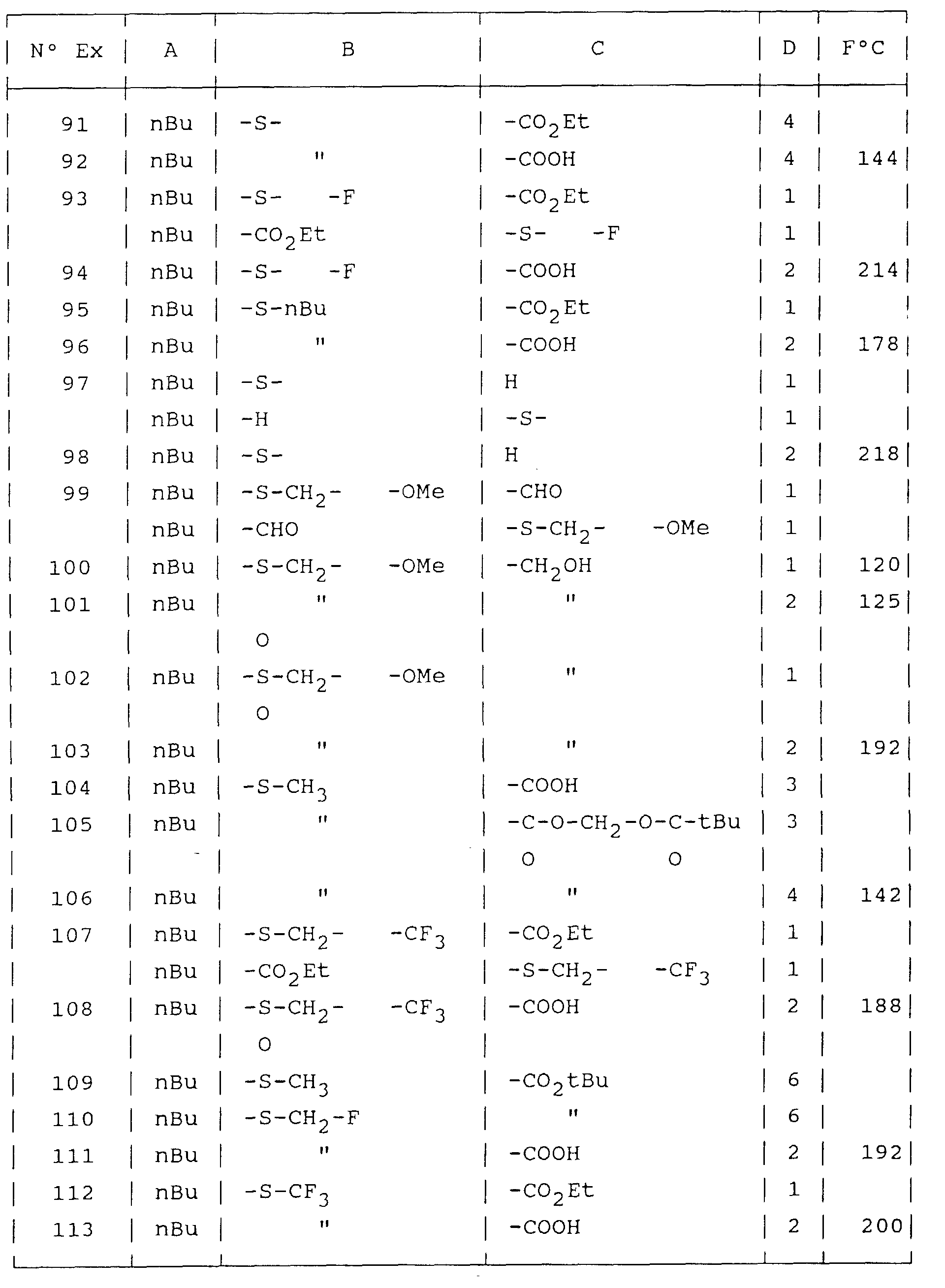

De plus les produits suivants qui constituent les exemples 114 à 143 peuvent être obtenus par les procédés indiqués ci-dessus.

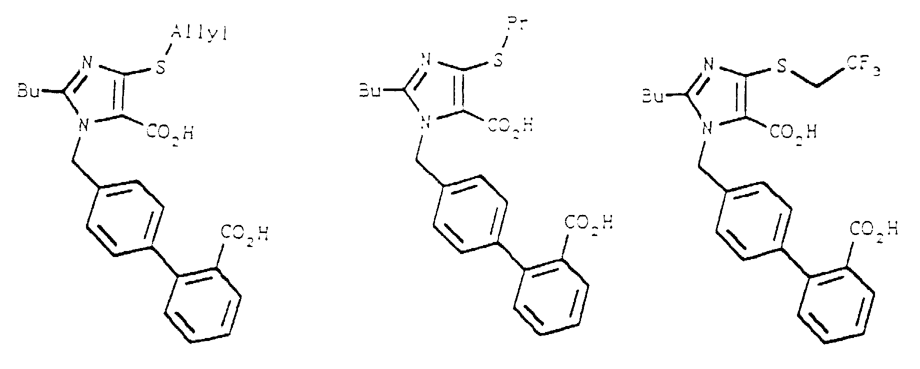

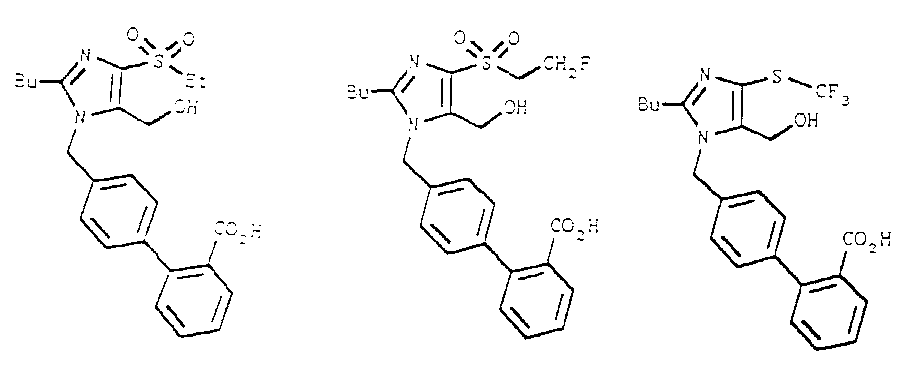

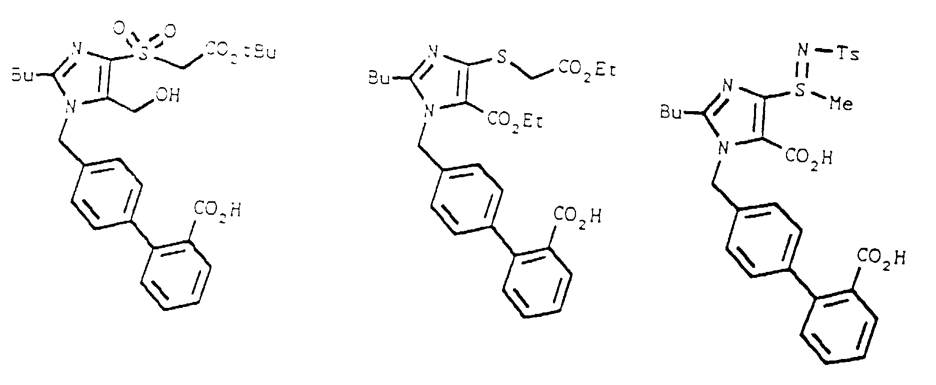

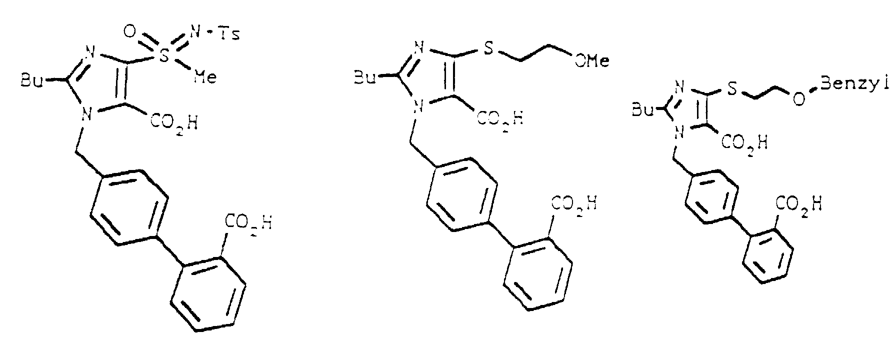

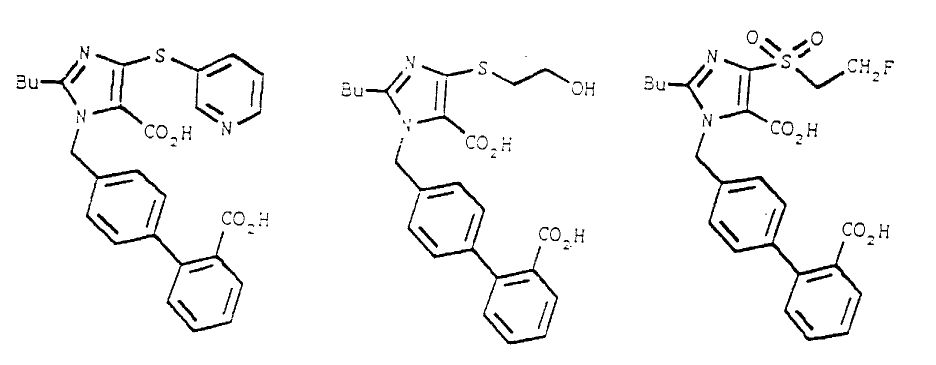

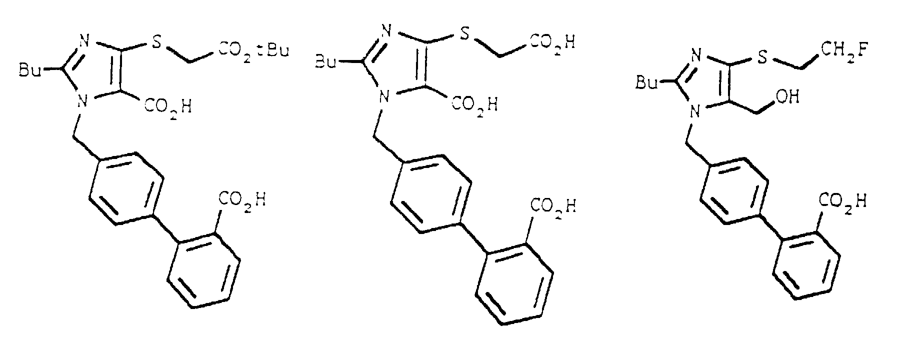

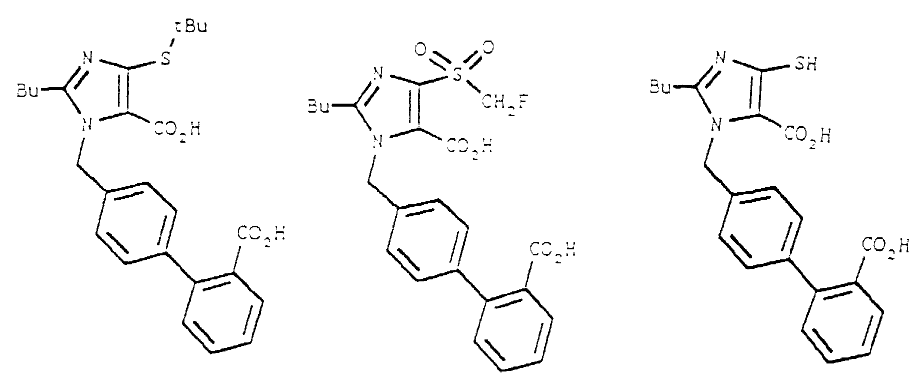

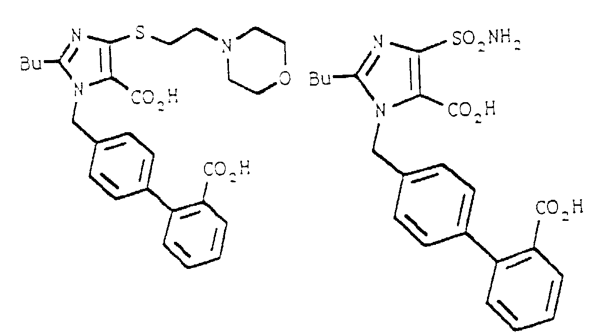

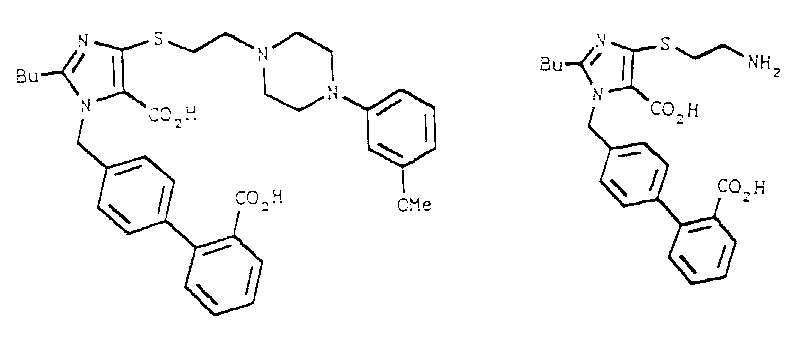

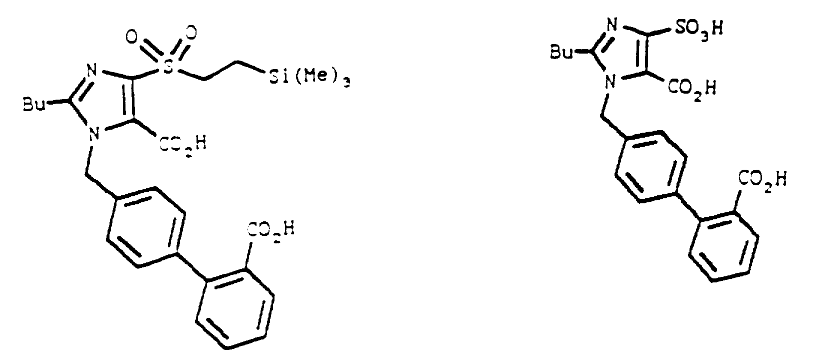

Les produits décrits dans les exemples ci-dessus peuvent également, et notamment à la place du substituant carboxy que porte le radical biphényle lié par un radical méthylène à un atome d'azote du radical imidazole, comporter un radical tétrazole éventuellement salifié ou un radical -(CH₂)ₘ₁-S(O)ₘ₂-X-R₁₀, tel que défini ci-dessus et tout particulièrement les radicaux :
-SO₂-NH-CO-NH-CH=CH-CH₂
-SO₂-NH-CO-NH-CH₂-CH=CH₂

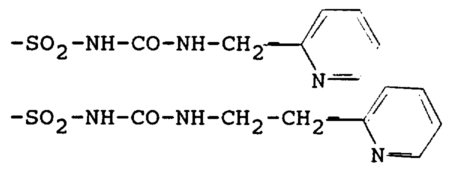

Les 4 produits suivants ont notamment été préparés suivant les conditions expérimentales décrites ci-dessus et constituent les exemples 144 à 147 suivants :

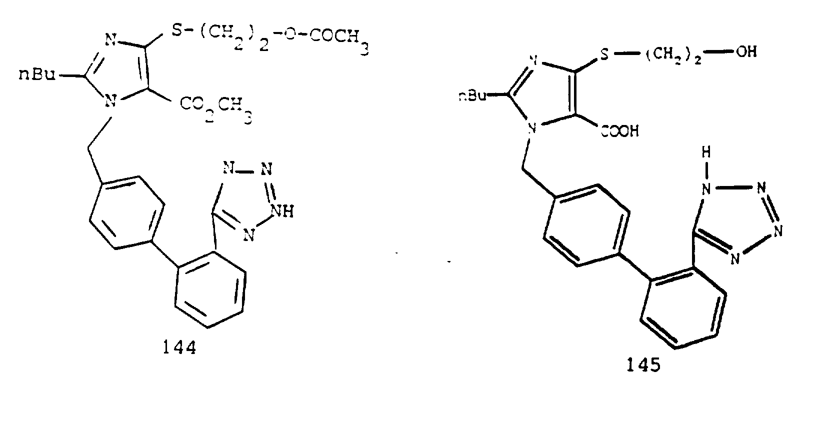

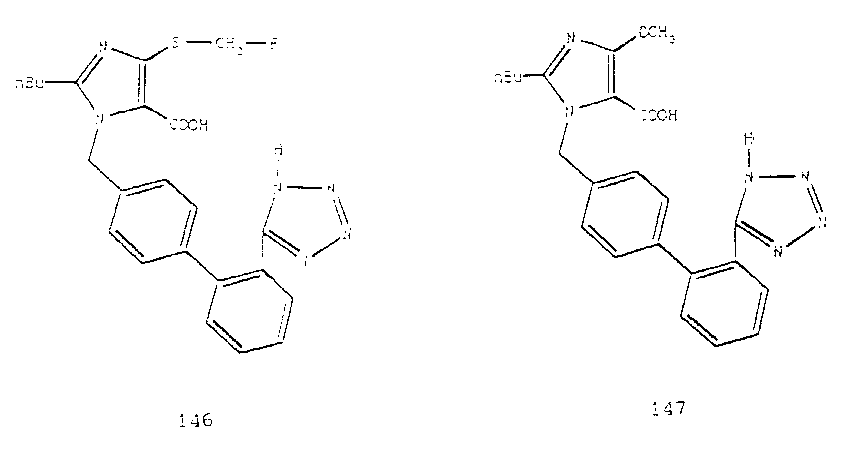

Le produit de l'exemple 145 tel que défini ci-dessus peut être caractérisé par les résultats analytiques suivants :

| IR cm⁻¹ Nujol : | |
|---|---|
| absorption OH/NH | |
| 〉=O | : 1660 cm⁻¹ |
| système conjugué | ¦ 1604 cm⁻¹ |
| + | ¦ 1574 cm⁻¹ |
| aromatiques | ¦ 1520 cm⁻¹ |
| UV dans EtOH pour MM = 478,56 | |
| infl. 225 nm epsilon = 28900 | |
| infl. 246 nm epsilon = 16500 | |
| max. 282 nm epsilon = 14700 | |

Parmi les produits de formule (I) tels que définis ci-dessus qui constituent des produits pouvant être obtenus dans le cadre de la présente invention, les produits préférés sont ceux dans lesquels R₂ représente un radical -Alk et R₃ représente un radical -S(O)Alk, ainsi que ceux dans lesquels R₂ représente un radical soufré, notamment -S-Alk, 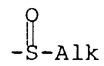 et 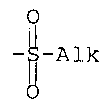 ou un radical O-Alk et R₃ représente un radical -COOH ou -CH₂OH, le radical -Alk dans les radicaux ci-dessus représentant un radical alkyle renfermant au plus 4 atomes de carbones linéaire ou ramifié et éventuellement substitué par un ou plusieurs radicaux choisis parmi les atomes d'halogène ; le radical hydroxyle ; le radical amino lui-même éventuellement substitué par un ou deux radicaux alkyles renfermant au plus 4 atomes de carbone ; le radical phényle lui-même éventuellement substitué par un ou plusieurs radicaux choisis parmi les radicaux alkyle ou alkoxy renfermant au plus 4 atomes de carbone, les atomes d'halogène, les radicaux cyano, nitro, carboxy libre, salifié ou estérifié et tétrazolyle.

### EXEMPLE 144 de composition pharmaceutique.

On a préparé des comprimés répondant à la formule suivante :

| | |
|---|---|
| Produit de l'exemple 51 | 50 mg |
| Excipient pour un comprimé terminé à | 200 mg |
| (détail de l'excipient : lactose, talc, amidon, stéarate de magnésium). | |

### RESULTATS PHARMACOLOGIQUES :

### 1 - Test sur le récepteur de l'anqiotensine II

On utilise une préparation membranaire fraîche obtenue à partir de foie de rat. Le tissu est broyé au polytron dans un tampon Tris 50 mM pH 7,4, le broyage est suivi de 3 centrifugations à 30 000 g 15' avec reprises intermédiaires des culots dans le tampon Tris pH 7,4.

Les derniers culots sont remis en suspension dans un tampon d'incubation (Tris 20 mM, NaCl 135 mM, KCl 10 mM, glucose 5 mM, MgCl₂ 135 mM, KCl 10 mM, glucose 5 mM, MgCl₂ 10 m PMSF 0,3 mM, bacitracine 0,1 mM, BSA 0,2 %).

On répartit des fractions aliquotées de 2 ml dans des tubes à hémolyse et ajoute de la ¹²⁵ I angiotensine II (25 000 DPM/tube) et le produit à étudier. (Le produit est d'abord testé à 3 x 10⁻⁵M en triple). Lorsque le produit testé déplace de plus de 50 % la radioactivité liée spécifiquement au récepteur, il est testé à nouveau selon une gamme de 7 doses afin de déterminer la dose qui inhibe de 50 % la radioactivité liée spécifiquement au récepteur. On détermine ainsi la concentration inhibitrice 50 %).

La liaison non spécifique est déterminée par addition du produit de l'exemple 94 du brevet européen 0253310, à 10⁻⁵M (en triple). On incube à 25°C pendant 150 minutes, remet au bain-marie à 0°C, 5 minutes, filtre sous vide, rince au tampon Tris pH 7,4 et compte la radioactivité en présence du scintillant Triton.

Le résultat est exprimé directement en concentration inhibitrice 50 % (CI₅₀), c'est-à-dire en concentration de produit étudié, exprimée en nM, nécessaire pour déplacer 50 % de la radioactivité spécifique fixée sur le récepteur étudié.

| Résultats : | |
|---|---|
| Produit de l'exemple | CI₅₀ en nanomoles |
| 28 | 10,2 |
| 20 | 17,0 |
| 26 | 47,0 |
| 22 | 49,0 |
| 48 | 5,7 |
| 70 | 3,6 |
| 74 | 5,8 |
| 51 | 1,9 |
| 83 | 1,3 |
| 92 | 0,5 |
| 84 | 0,8 |

### 2 - Mise en évidence de l'activité antaqoniste de l'anqiotensine II sur la veine porte isolée

La veine porte est prélevée, sur des rats mâles Wistar (350 g environ) (IFFA Crédo France) après dislocation cervicale, et mise rapidement dans une solution physiologique (voir ci-dessous) à température ambiante. Un anneau de 1 mm environ est monté dans un bain à organe isolé contenant 20 ml de la solution physiologique suivante (composition en mM : NaCl 118,3 - KCl 4,7 - MgSO₄ 1,2 - KH₂PO₄ 1,2 - NaHCO₃ 25 - glucose 11,1 - CaCl₂ 2,5) le milieu est maintenu à 37°C et oxygéné par un mélange oxygène 95 %, gaz carbonique 5 %. La tension initiale imposée est de 1 g, les anneaux sont laissés au repos pendant 60 à 90 minutes. Afin d'éviter les contractions spontanées, du vérapamil est ajouté au bain d'incubation (1.10⁻⁶M).

A la fin de la période de repos l'angiotensine II (hypertensine liba) 3.10⁻⁸M est ajoutée dans le bain d'incubation et laissée au contact de la préparation pendant 1 minute. Cette opération est répétée chaque 30 minutes, le tissu étant lavé 3 ou 4 fois entre deux stimulations à l'angiotensine. Le composé à étudier est introduit dans le bain 15 minutes avant une nouvelle stimulation par l'angiotensine. On utilise des concentrations croissantes du produit à étudier et on calcule la CI₅₀ (dose qui produit une inhibition de 50 % de la réponse à l'angiotensine) du produit à étudier, celle-ci est exprimée en nanomoles.

| Résultats : | |
|---|---|
| Produit de l'exemple | CI₅₀ en nanomoles |
| 20 | 5 |
| 22 | 8 |
| 14 | 10 |
| 6 | 13 |
| 48 | 0,9 |
| 70 | 0,56 |
| 74 | 2 |
| 51 | 0,14 |
| 83 | 0,21 |

### 3 - Test d'activité antagoniste de l'angiotensine II chez le rat démédullé

Des rats mâles Sprague-Dawley (250 à 350 g) sont anesthésiés par une injection intra-péritonéale de pentobarbital sodique (60 mg/kg). La pression artérielle diastolique est enregistrée grâce à un cathéter (PE50) hépariné introduit dans la carotide gauche de l'animal, et relié à un calculateur de pression (Gould, Pressure Processor) par l'intermédiaire d'un capteur de pression Gould.

Un cathéter est introduit dans la jugulaire droite de l'animal afin de permettre l'injection des molécules à étudier.

L'animal est placé sous respiration assistée. Une section bilatérale des nerfs vagues est effectuée. Le rat est alors démédullé.

Après une période de stabilisation suffisante, l'étude de l'antagonisme des molécules vis-à-vis de l'angiotensine II (Hypertensine, CIBA) est abordée de la façon suivante :
1 - Trois injections consécutives d'angiotensine II (0,75 microgrammes/kg) espacées de 15 minutes permettent d'obtenir une réponse pressive reproductible et stable.
2 - Tout en gardant une périodicité de 15 minutes pour l'administration d'angiotensine II, les molécules (0,01 à 10 mg/kg) sont injectées 5 minutes avant l'angiotensine II.
Les effets presseurs de l'angiotensine II en présence de l'antagoniste sont exprimés en pourcentage des effets presseurs de l'angiotensine II administrée seule. La dose inhibitrice à 50 % de l'effet étudié est ainsi déterminée.
Chaque animal est considéré comme son propre témoin.

| Résultats : | |
|---|---|
| Produit de l'exemple | CI₅₀ en mg/kg |
| 16 | 0,29 |
| 22 | 0,47 |
| 14 | 0,78 |
| 18 | 0,79 |
| 70 | 0,33 |
| 74 | 0,34 |
| 51 | 0,054 |
| 83 | 0,022 |
| 92 | 0,08 |
| 106 | 0,04 |

## Revendications (Revendications pour l'(les) Etat(s) contractant(s) suivant(s): AT, BE, CH, DE, DK, FR, GB, IT, LI, LU, NL, SE)

1. Produits de formule (I_{B}) : 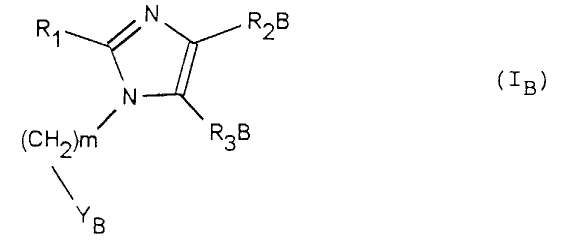 dans laquelle :
R₁ représente un radical alkyle renfermant au plus 4 atomes de carbone,
R_{2B} et R_{3B} identiques ou différents sont choisis parmi :
- l'atome d'hydrogène,
- un radical carboxy libre, salifié ou estérifié par un radical alkyle,
- formyle, acyloxy cnoisi dans le groupe consistant en formyloxy, acétyloxy, propionyloxy, butyryloxy et benzoyloxy, sulfo,
- alkyle et alcoxy éventuellement substitués,
- phénylthio, phénylsulfonyle, phénylsulfinyle, alkylthio, alkylsulfonyle et alkylsulfinyle, éventuellement substitués, tel que dans tous ces radicaux que peuvent représenter R_{2B} et R_{3B}, les radicaux alkyle et alcoxy renferment au plus 6 atomes de carbone, et les radicaux alkyle, alcoxy et phényle sont éventuellement substitués par un ou plusieurs radicaux choisis parmi les atomes d'halogène et les radicaux hydroxyle, trifluorométhyle, acyloxy, carboxy libre salifié ou estérifié, phényle, pyridyle, tétrazolyle, alkyle et alcoxy renfermant au plus 4 atomes de carbone et eux-mêmes éventuellement substitués par un radical alcoxy renfermant au plus 4 atomes de carbone,
étant entendu que l'un au moins des groupes R_{2B} et R_{3B} représente un groupe choisi parmi phénylthio, phénylsulfonyle, phénylsulfinyle, alkylthio, alkylsulfonyle et alkylsulfinyle, éventuellement substitués,
et Y_{B} représente le radical -Y_{1B}-B-Y_{2B} dans lequel Y_{1B} représente un radical phényle, B représente une liaison simple carbone-carbone et Y_{2B} représente un radical carboxy libre ou estérifié par un radical alkyle linéaire ou ramifié renfermant au plus 4 atomes de carbone ou un radical phényle éventuellement substitué par un radical carboxy libre ou estérifié par un radical alkyle linéaire ou ramifié renfermant au plus 4 atomes de carbone, un radical cyano ou un radical tétrazolyle éventuellement salifié,
lesdits produits de formule (I_{B}) étant sous toutes les formes isomères possibles racémiques, énantiomères et diastéréoisomères, ainsi que les sels d'addition avec les acides minéraux et organiques ou avec les bases minérales et organiques desdits produits de formule (I_{B}).

2. Produits de formule (I_{B}) telle que définie à la revendication 1 et répondant à la formule (Iₐ) : 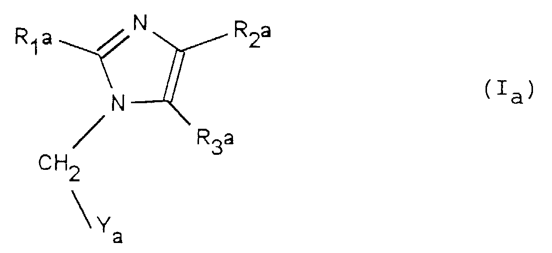 dans laquelle :
R₁ₐ représente un radical alkyle linéaire ou ramifié renfermant au plus 4 atomes de carbone,
R₂ₐ et R₃ₐ, identiques ou différents, sont choisis parmi :
a) l'atome d'hydrogène, le radical mercapto, les radicaux formyle, carboxy libre, salifié ou estérifié,
b) les radicaux R₄ₐ et -S(O)ₙR₄ₐ tel que n représente la valeur 0, 1 ou 2,
dans lesquels R₄ₐ représente un radical alkyle ou alkényle linéaire ou ramifié renfermant au plus 4 atomes de carbone, un radical cyclohexyle, un radical phényle, pyridyle, pyrimidinyle ou imidazolyle, tous ces radicaux étant éventuellement substitués par un ou plusieurs radicaux identiques ou différents choisis parmi :
-- les atomes d'halogène,
-- les radicaux hydroxyle, mercapto, acylthio, trifluorométhyle, trifluorométhylthio, trifluorométhoxy, cyano, azido, nitro,
-- le radical phényle,
-- les radicaux alkyle et alcoxy renfermant au plus 4 atomes de carbone, carboxy libre, salifié ou estérifié, tétrazolyle, acyle, acyloxy, et
-- les radicaux 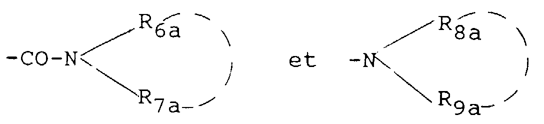
dans lesquels :
**ou bien** R₆ₐ, R₇ₐ, R₈ₐ et R₉ₐ, identiques ou différents, sont choisis parmi l'atome d'hydrogène, le radical hydroxyle, les radicaux alkyle, alcoxy, acyloxy ou acyle, ces radicaux renfermant au plus 6 atomes de carbone, carboxy libre, salifié ou estérifié, phényle, benzyle, phénéthyle, azépine, pipéridyle, morpholine, pyrrolidinyle, pipérazinyle,
**ou bien** d'une part R₆ₐ et R₇ₐ et d'autre part R₈ₐ et R₉ₐ forment respectivement avec l'atome d'azote auxquels ils sont liés un radical, ces radicaux identiques ou différents étant choisis parmi les radicaux imidazolyle, pyrrolyle, pyrrolinyle, pyrrolidinyle, pyridyle, pipéridinyle, pyrimidinyle, pyridazinyle, pyrazinyle, pipérazinyle, phénylpipérazinyle, pipéridyle, oxazolyle, morpholinyle et thiomorpholinyle, azépine, indolyle, ces radicaux étant éventuellement substitués par un ou plusieurs radicaux identiques ou différents choisis parmi les atomes d'halogène, les radicaux hydroxyle, trifluorométhyle, alkyle et alcoxy, ces radicaux renfermant au plus 6 atomes de carbone, étant entendu que l'un au moins de R₂ₐ ou R₃ₐ
représente le radical -S(O)ₙR₄ₐ,
Yₐ représente le radical -Y₁ₐ-Bₐ-Y₂ₐ dans lequel :
- Y₁ₐ représente un radical phényle,
- Bₐ représente une simple liaison ou le radical -CO-NH-,
- Y₂ₐ est tel que :
**soit**, si Bₐ représente une simple liaison ou un radical -CO-NH-, Y₂ₐ représente un radical phényle substitué en ortho par un radical carboxy libre, salifié ou estérifié ou un radical tétrazolyle,
**soit**, si Bₐ représente une simple liaison, Y₂ₐ représente un radical cyano ou carboxy libre, salifié ou estérifié ou un radical tétrazolyle,
lesdits produits de formule (Iₐ) étant sous toutes les formes isomères possibles racémiques, énantiomères et diastéréoisomères, ainsi que les sels d'addition avec les acides minéraux et organiques ou avec les bases minérales et organiques desdits produits de formule (Iₐ).

3. Produits de formule (I_{B}) telle que définie à la revendication 1 dans laquelle :
R₁ représente un radical alkyle renfermant au plus 4 atomes de carbone,
R_{2B} et R_{3B} identiques ou différents sont choisis parmi les radicaux :
- carboxy libre, salifié ou estérifié par un radical alkyle linéaire ou ramifié renfermant au plus 4 atomes de carbone, formyle, acyloxy choisi dans le groupe consistant en formyloxy, acétyloxy, propionyloxy, butyryloxy et benzoyloxy,
- alkyle renfermant au plus 4 atomes de carbone éventuellement substitués par un radical hydroxyle,
- phénylthio, phénylsulfonyle, phénylsulfinyle,
- alkylthio, alkylsulfonyle et alkylsulfinyle,
dans lesquels le radical alkyle renferme au plus 4 atomes de carbone,
et Y_{B} représente le radical -Y_{1B}-B-Y_{2B} dans lequel Y_{1B} représente un radical phényle, B représente une liaison simple carbone-carbone et Y_{2B} représente un radical carboxy libre ou estérifié par un radical alkyle linéaire ou ramifié renfermant au plus 4 atomes de carbone ou un radical phényle éventuellement substitué par un radical carboxy libre ou estérifié par un radical alkyle linéaire ou ramifié renfermant au plus 4 atomes de carbone,
lesdits produits de formule (I_{B}) étant sous toutes les formes isomères possibles racémiques, énantiomères et diastéréoisomères, ainsi que les sels d'addition avec les acides minéraux et organiques ou avec les bases minérales et organiques desdits produits de formule (I_{B})

4. Produits de formule (I_{B}) telle que définie à la revendication 1, dans laquelle l'un de R_{2B} ou R_{3B} représente un radical alcoxy, alkylthio ou phénylthio ces deux derniers étant éventuellement oxydés sous forme de sulfoxyde ou de sulfone et ces trois radicaux étant éventuellement substitués et l'autre représente un radical carboxy libre salifié ou estérifié, lesdits produits de formule (I_{B}) étant sous toutes les formes isomères possibles racémiques, énantiomères et diastéréoisomères, ainsi que les sels d'addition avec les acides minéraux et organiques ou avec les bases minérales et organiques desdits produits de formule (I_{B}).

5. Les produits de formule (I_{B}) répondant aux formules suivantes :
- l'acide 2-butyl 1-[[2'-carboxy (1,1'-biphényl)-4-yl] méthyl] 4-(phénylthio) 1H-imidazole-5-carboxylique
- l'acide 2-butyl 1-[[2'-carboxy (1,1'-biphényl)-4-yl] méthyl] 4-(méthylthio) 1H-imidazole-5-carboxylique
- l'acide 4'-[[2-butyl 4-(éthylthio) 5-(hydroxyméthyl) 1H-imidazol-1-yl] méthyl] (1,1'-biphényl)-2-carboxylique
- l'acide 2-butyl 1-[[2'-carboxy (1,1'-biphényl)-4-yl] méthyl] 4-(éthylsulfonyl) 1H-imidazole-5-carboxylique
- l'acide 2-butyl 1-[[2'-carboxy (1,1'-biphényl)-4-yl] méthyl] 4-(éthylsulfinyl) 1H-imidazole-5-carboxylique
- l'acide 2-butyl 1-[[2'-carboxy (1,1'-biphényl)-4-yl] méthyl] 4-(éthylthio) 1H-imidazole-5-carboxylique
- l'acide 2-butyl 1-[[2'-carboxy (1,1'-biphényl)-4-yl] méthyl] 4-(phénylsulfonyl) 1H-imidazole-5-carboxylique
- l'acide 2-bùtyl 1-[[2'-carboxy (1,1'-biphényl)-4-yl] méthyl] 4-(phénylsulfinyl) 1H-imidazole-5-carboxylique
- l'acide 2-butyl 1-[[2'-tétrazolyl (1,1'-biphényl)-4-yl] méthyl] 4-(méthylthio) 1H-imidazole-5-carboxylique.

6. Procédé de préparation de produits de formule (I_{B}) telle que définie à la revendication 1 caractérisé en ce que l'on fait réagir un composé de formule (II) : 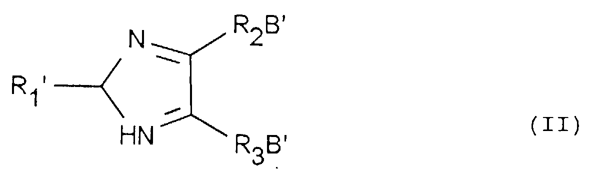 dans laquelle R₁', R_{2B}' et R_{3B}' ont les significations indiquées à la revendication 1 respectivement pour R₁, R_{2B} et R_{3B} et dans lesquelles les éventuelles fonctions réactives sont éventuellement protégées par des groupements protecteurs, avec un composé de formule (III) :
Z-(CH₂)ₘ-Y_{B}' (III)
dans laquelle Z représente un atome d'halogène ou un sulfonate et Y_{B}' a la signification indiquée à la revendication 1 pour Y_{B} dans laquelle les éventuelles fonctions réactives sont éventuellement protégées par des groupements protecteurs, pour obtenir un produit de formule (IV) : 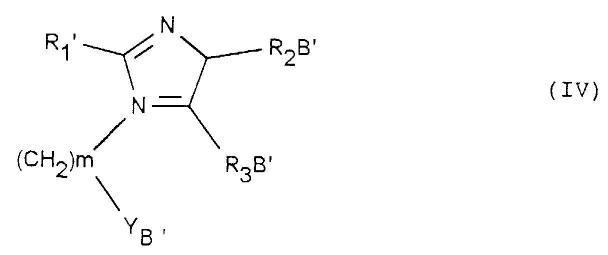 dans laquelle R₁', R_{2B}', R_{3B}' et Y_{B}' ont les significations indiquées ci-dessus,
produit de formule (IV) que l'on soumet, si désiré et si nécessaire, à l'une ou plusieurs des réactions suivantes, dans un ordre quelconque :
a) une réaction d'élimination des groupements protecteurs que peuvent porter les fonctions réactives protégées,
b) une réaction de salification par un acide minéral ou organique ou par une base pour obtenir le sel correspondant,
c) une réaction d'estérification de fonction acide,
d) une réaction de saponification de fonction ester en fonction acide,
e) une réaction de transformation de la fonction cyano en fonction acide,
f) une réaction de réduction de la fonction carboxy en fonction alcool,
g) une réaction de transformation de fonction alcoxy en fonction hydroxyle,
h) une réaction d'oxydation de groupement alkylthio ou arylthio en sulfoxyde ou sulfone correspondant,
i) une réaction de transformation de fonction sulfoxyde ou sulfone en fonction sulfoximine correspondante,
j) une réaction d'oxydation de fonction alcool en fonction aldéhyde ou acide,
k) une réaction de transformation de radical nitrile en tétrazole,
l) une réaction de dédoublement des formes racémiques en produits dédoublés,
m) une réaction de transformation du radical formyle en radical carbamoyle,
n) une réaction de transformation du radical carbamoyle en radical nitrile,
lesdits produits de formule (I_{B}) ainsi obtenus étant sous toutes les formes isomères possibles racémiques, énantiomères et diastéréoisomères.

7. A titre de médicaments, les produits tels que définis par la formule (I_{B}) de la revendication 1, lesdits produits de formule (I_{B}) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (I_{B}).

8. A titre de médicaments, les produits de formule (I_{B}) et (Iₐ) tels que définis aux revendications 2 à 4, ainsi que les sels d'addition avec les acides minéraux et organiques ou avec les bases minérales et organiques pharmaceutiquement acceptables desdits produits de formules (I_{B}) et (Iₐ).

9. Les compositions pharmaceutiques contenant à titre de principe actif, l'un au moins des médicaments tels que définis à l'une quelconque des revendications 7 et 8.

10. Procédé de préparation des produits de départ de formule (II) telle que définie à la revendication 6, caractérisé en ce que l'on soumet un composé de formule (IIₐ) : 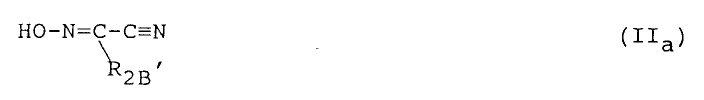 dans laquelle R_{2B}' a la signification indiquée à la revendication 1 pour R_{2B} dans laquelle les éventuelles fonctions réactives sont éventuellement protégées par des groupements protecteurs, que l'on soumet à l'action d'un agent réducteur, pour obtenir l'amine correspondante de formule (II_{b}) : 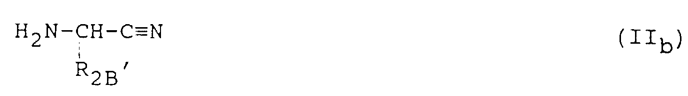 dans laquelle R_{2B}' la signification indiquée précédemment, que l'on soumet à l'action d'un composé de formule (II_{c}) : 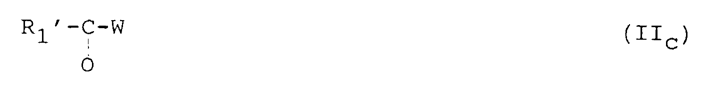 dans laquelle R₁' a la signification indiquée à la revendication 1 pour R₁ dans laquelle les éventuelles fonctions réactives sont éventuellement protégées par des groupements protecteurs et W représente un radical hydroxyle ou un atome d'halogène, pour obtenir un produit de formule (II_{d}) : 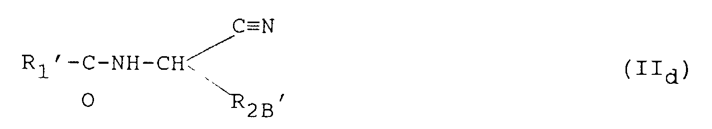 dans laquelle R₁' et R_{2B}' ont les significations indiquées précédemment, que l'on fait réagir avec un composé de formule (IIₑ) :
R_{3B}'-SH (IIₑ)
dans laquelle R_{3B}' a la signification indiquée à la revendication 1 pour R_{3B} dans laquelle les éventuelles fonctions réactives sont éventuellement protégées par des groupements protecteurs, pour obtenir un produit de formule (II_{f}) : 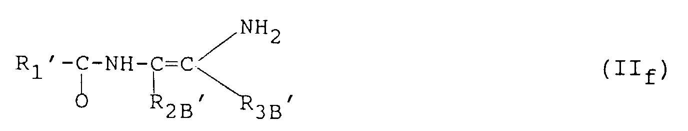 dans laquelle R₁', R_{2B}' et R_{3B}' ont les significations indiquées précédemment, que l'on soumet à une réaction de cyclisation pour obtenir un produit de formule (II), produit de formule (II) que l'on soumet, si désiré et si nécessaire, à l'une ou plusieurs des réactions suivantes, dans un ordre quelconque :
a) une réaction d'élimination des groupements protecteurs que peuvent porter les fonctions réactives protégées,
b) une réaction de salification par un acide minéral ou organique ou par une base pour obtenir le sel correspondant,
c) une réaction d'estérification de fonction acide,
d) une réaction de saponification de fonction ester en fonction acide,
e) une réaction de transformation de la fonction cyano en fonction acide,
f) une réaction de réduction de la fonction carboxy en fonction alcool,
g) une réaction de transformation de fonction alcoxy en fonction hydroxyle,
h) une réaction d'oxydation de groupement alkylthio ou arylthio en sulfoxyde ou sulfone correspondant,
i) une réaction de transformation de fonction sulfoxyde ou sulfone en fonction sulfoximine correspondante,
j) une réaction d'oxydation de fonction alcool en fonction aldéhyde ou acide,
k) une réaction de transformation de fonction nitrile en tétrazole,
l) une réaction de dédoublement des formes racémiques en produits dédoublés,
m) une réaction de transformation du radical formyle en radical carbamoyle,
n) une réaction de transformation du radical carbamoyle en radical nitrile,
lesdits produits de formule (II) ainsi obtenus étant sous toutes les formes isomères possibles racémiques, énantiomères et diastéréoisomères.

11. Composés de formule (II) dans laquelle R₁' ne représente pas le radical méthyle.

## Revendications (Revendications pour l'(les) Etat(s) contractant(s) suivant(s): ES)

1. Procédé de préparation de produits de formule (I_{B}) : 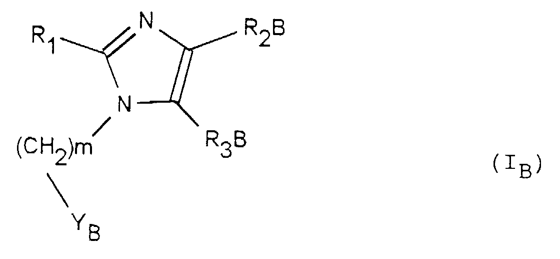 dans laquelle :
R₁ représente un radical alkyle renfermant au plus 4 atomes de carbone, R_{2B} et R_{3B}, identiques ou différents sont choisis parmi :
- l'atome d'hydrogène,
- un radical carboxy libre, salifié ou estérifié par un radical alkyle,
- formyle, acyloxy choisi dans le groupe consistant en formyloxy, acétyloxy, propionyloxy, butyryloxy et benzoyloxy, sulfo,
- alkyle et alcoxy éventuellement substitués,
- phénylthio, phénylsulfonyle, phénylsulfinyle, alkylthio, alkylsulfonyle et alkylsulfinyle, éventuellement substitués, tel que dans tous ces radicaux que peuvent représenter R_{2B} et R_{3B}, les radicaux alkyle et alcoxy renferment au plus 6 atomes de carbone, et les radicaux alkyle, alcoxy et phényle sont éventuellement substitués par un ou plusieurs radicaux choisis parmi les atomes d'halogène et les radicaux hydroxyle, trifluorométhyle, acyloxy, carboxy libre salifié ou estérifié, phényle, pyridyle, tétrazolyle, alkyle et alcoxy renfermant au plus 4 atomes de carbone et eux-mêmes éventuellement substitués par un radical alcoxy renfermant au plus 4 atomes de carbone, étant entendu que l'un au moins des groupes R_{2B} et R_{3B} représente un groupe choisi parmi phénylthio, phénylsulfonyle, phénylsulfinyle, alkylthio, alkylsulfonyle et alkylsulfinyle, éventuellement substitués, et Y_{B} représente le radical -Y_{1B}-B-Y_{2B} dans lequel Y_{1B} représente un radical phényle, B représente une liaison simple carbone-carbone et Y_{2B} représente un radical carboxy libre ou estérifié par un radical alkyle linéaire ou ramifié renfermant au plus 4 atomes de carbone ou un radical phényle éventuellement substitué par un radical carboxy libre ou estérifié par un radical alkyle linéaire ou ramifié renfermant au plus 4 atomes de carbone, un radical cyano ou un radical tétrazolyle éventuellement salifié,lesdits produits de formule (I_{B}) étant sous toutes les formes isomères racémiques, énantiomères et diastéréoisomères possibles, ainsi que les sels d'addition avec les acides minéraux et organiques ou avec les bases minérales et organiques desdits produits de formule (I_{B}), caractérisé en ce que l'on fait réagir un composé de formule (II) : 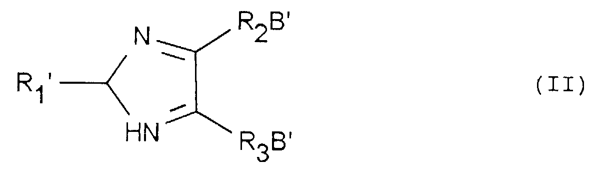
dans laquelle R₁', R_{2B}' et R_{3B}' ont les significations indiquées à la revendication 1 respectivement pour R₁, R_{2B} et R_{3B} et dans lesquelles les éventuelles fonctions réactives sont éventuellement protégées par des groupements protecteurs, avec un composé de formule (III) :
Z-(CH₂)ₘ-Y_{B}' (III)
dans laquelle Z représente un atome d'halogène ou un sulfonate et Y_{B}' a la signification indiquée à la revendication 1 pour Y_{B} dans laquelle les éventuelles fonctions réactives sont éventuellement protégées par des groupements protecteurs, pour obtenir un produit de formule (IV) : 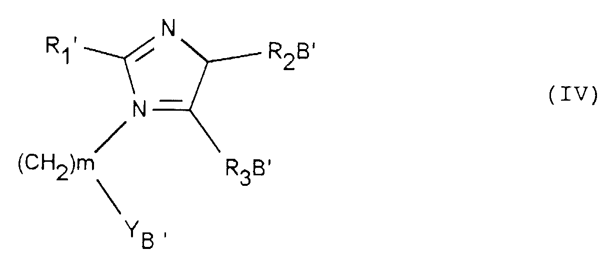 dans laquelle R₁', R_{2B}', R_{3B}' et Y_{B}' ont les significations indiquées ci-dessus,
produit de formule (IV) que l'on soumet, si désiré et si nécessaire, à l'une ou plusieurs des réactions suivantes, dans un ordre quelconque :
a) une réaction d'élimination des groupements protecteurs que peuvent porter les fonctions réactives protégées,
b) une réaction de salification par un acide minéral ou organique ou par une base pour obtenir le sel correspondant,
c) une réaction d'estérification de fonction acide,
d) une réaction de saponification de fonction ester en fonction acide,
e) une réaction de transformation de la fonction cyano en fonction acide,
f) une réaction de réduction de la fonction carboxy en fonction alcool,
g) une réaction de transformation de fonction alcoxy en fonction hydroxyle,
h) une réaction d'oxydation de groupement alkylthio ou arylthio en sulfoxyde ou sulfone correspondant,
i) une réaction de transformation de fonction sulfoxyde ou sulfone en fonction sulfoximine correspondante,
j) une réaction d'oxydation de fonction alcool en fonction aldéhyde ou acide,
k) une réaction de transformation de radical nitrile en tétrazole,
l) une réaction de dédoublement des formes racémiques en produits dédoublés,
m) une réaction de transformation du radical formyle en radical carbamoyle,
n) une réaction de transformation du radical carbamoyle en radical nitrile,
lesdits produits de formule (I_{B}) ainsi obtenus étant sous toutes les formes isomères possibles racémiques, énantiomères et diastéréoisomères.

2. Procédé selon la revendication 1 pour la préparation des produits de formule (I_{B}) répondant à la formule (Iₐ) : 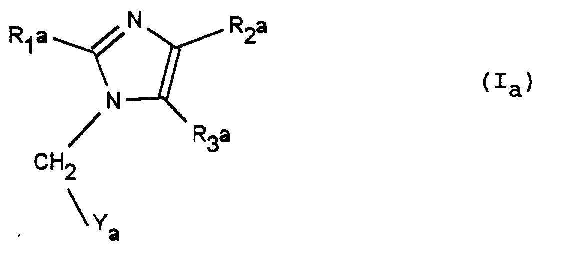 dans laquelle :
R₁ₐ représente un radical alkyle linéaire ou ramifié renfermant au plus 4 atomes de carbone,
R₂ₐ et R₃ₐ, identiques ou différents, sont choisis parmi :
a) l'atome d'hydrogène, le radical mercapto, les radicaux formyle, carboxy libre, salifié ou estérifié,
b) les radicaux R₄ₐ et -S(O)ₙR₄ₐ tel que n représente la valeur 0, 1 ou 2,
dans lesquels R₄ₐ représente un radical alkyle ou alkényle linéaire ou ramifié renfermant au plus 4 atomes de carbone, un radical cyclohexyle, un radical phényle, pyridyle, pyrimidinyle ou imidazolyle, tous ces radicaux étant éventuellement substitués par un ou plusieurs radicaux identiques ou différents choisis parmi :
-- les atomes d'halogène,
-- les radicaux hydroxyle, mercapto, acylthio, trifluorométhyle, trifluorométhylthio, trifluorométhoxy, cyano, azido, nitro,
-- le radical phényle,
-- les radicaux alkyle et alcoxy renfermant au plus 4 atomes de carbone, carboxy libre, salifié ou estérifié, tétrazolyle, acyle, acyloxy, et
-- les radicaux 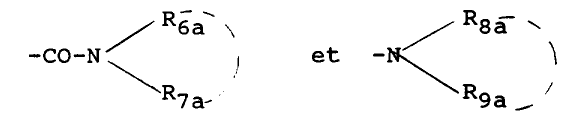
dans lesquels :
**ou bien** R₆ₐ, R₇ₐ, R₈ₐ et R₉ₐ, identiques ou différents, sont choisis parmi l'atome d'hydrogène, le radical hydroxyle, les radicaux alkyle, alcoxy, acyloxy ou acyle, ces radicaux renfermant au plus 6 atomes de carbone, carboxy libre, salifié ou estérifié, phényle, benzyle, phénéthyle, azépine, pipéridyle, morpholine, pyrrolidinyle, pipérazinyle,
**ou bien** d'une part R₆ₐ et R₇ₐ et d'autre part R₈ₐ et R₉ₐ forment respectivement avec l'atome d'azote auxquels ils sont liés un radical, ces radicaux identiques ou différents étant choisis parmi les radicaux imidazolyle, pyrrolyle, pyrrolinyle, pyrrolidinyle, pyridyle, pipéridinyle, pyrimidinyle, pyridazinyle, pyrazinyle, pipérazinyle, phénylpipérazinyle, pipéridyle, oxazolyle, morpholinyle et thiomorpholinyle, azépine, indolyle, ces radicaux étant éventuellement substitués par un ou plusieurs radicaux identiques ou différents choisis parmi les atomes d'halogène, les radicaux hydroxyle, trifluorométhyle, alkyle et alcoxy, ces radicaux renfermant au plus 6 atomes de carbone, étant entendu que l'un au moins de R₂ₐ ou R₃ₐ représente le radical -S(O)ₙR₄ₐ,
Yₐ représente le radical -Y₁ₐ-Bₐ-Y₂ₐ dans lequel :
- Y₁ₐ représente un radical phényle,
- Bₐ représente une simple liaison ou le radical -CO-NH-,
- Y₂ₐ est tel que :
**soit**, si Bₐ représente une simple liaison ou un radical -CO-NH-, Y₂ₐ représente un radical phényle substitué en ortho par un radical carboxy libre, salifié ou estérifié ou un radical tétrazolyle,
**soit**, si Bₐ représente une simple liaison, Y₂ₐ représente un radical cyano ou carboxy libre, salifié ou estérifié ou un radical tétrazolyle,
lesdits produits de formule (Iₐ) étant sous toutes les formes isomères possibles racémiques, énantiomères et diastéréoisomères, ainsi que les sels d'addition avec les acides minéraux et organiques ou avec les bases minérales et organiques desdits produits de formule (Iₐ), caractérisé en ce que l'on utilise au départ un produit de formule (II) et (III) dans lesquels R'₁, R'_{2B}, R'_{3B} et Y'_{B} ont les valeurs indiquées ci-dessus respectivement pour R₁ₐ, R₂ₐ, R₃ₐ et Yₐ dans lesquelles les fonctions réactives sont éventuellement protégées.

3. Procédé selon la revendication 2, caractérisé en ce que l'on utilise au départ un produit de formule (II) dans laquelle R'₁ représente un radical alkyle renfermant au plus 4 atomes de carbone, R'_{2B} et R'_{3B} identiques ou différents sont choisis parmi les radicaux :
- carboxy libre, salifié ou estérifié par un radical alkyle linéaire ou ramifié renfermant au plus 4 atomes de carbone, formyle, acyloxy, choisi dans le groupe consistant en formyloxy, acétyloxy, propionyloxy, butyryloxy et benzoyloxy.
- alkyle renfermant au plus 4 atomes de carbone éventuellement substitués par un radical hydroxyle,
- phénylthio, phénylsulfonyle, phénylsulfinyle,
- alkylthio, alkylsulfonyle et alkylsulfinyle,
dans lesquels le radical alkyle renferme au plus 4 atomes de carbone, dans lesquels les fonctions réactives sont éventuellement protégées et un produit de formule (III) dans laquelle Y'_{B} représente le radical -Y₁-B-Y₂ dans lequel Y₁ représente un radical phényle, B représente une liaison simple carbone-carbone et Y₂ représente un radical carboxy libre ou estérifié par un radical alkyle linéaire ou ramifié renfermant au plus 4 atomes de carbone ou un radical phényle éventuellement substitué par un radical carboxy libre ou estérifié par un radical alkyle linéaire ou ramifié renfermant au plus 4 atomes de carbone, dans lesquels les fonctions réactives sont éventuellement protégées.

4. Procédé selon la revendication 1 ou 2, caractérisé en ce que l'on utilise au départ un produit de formule (II) dans laquelle R'_{2B} ou R'_{3B} représente un radical alcoxy, alkylthio ou phénylthio ces deux derniers étant éventuellement oxydés sous forme de sulfoxyde ou de sulfone et ces trois radicaux étant éventuellement substitués et l'autre représente un radical carboxy libre salifié ou estérifié.

5. Procédé de préparation des produits de départ de formule (II) telle que définie à la revendication 1, caractérisé en ce que l'on soumet un composé de formule (IIₐ) : 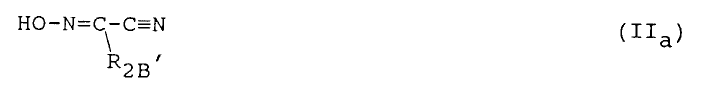 dans laquelle R_{2B}' a la signification indiquée à la revendication 1 pour R_{2B} dans laquelle les éventuelles fonctions réactives sont éventuellement protégées par des groupements protecteurs, que l'on soumet à l'action d'un agent réducteur, pour obtenir l'amine correspondante de formule (II_{b}) : 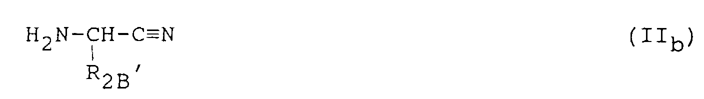 dans laquelle R_{2B}' a la signification indiquée précédemment, que l'on soumet à l'action d'un composé de formule (II_{c}) : 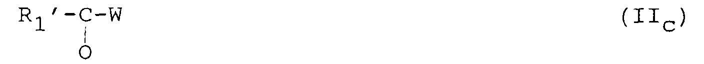 dans laquelle R₁' a la signification indiquée à la revendication 1 pour R₁ dans laquelle les éventuelles fonctions réactives sont éventuellement protégées par des groupements protecteurs et W représente un radical hydroxyle ou un atome d'halogène, pour obtenir un produit de formule (II_{d}) : 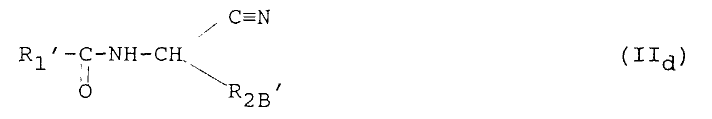 dans laquelle R₁' et R_{2B}' ont les significations indiquées précédemment, que l'on fait réagir avec un composé de formule (IIₑ) :
R_{3B}'-SH (IIₑ)
dans laquelle R_{3B}' a la signification indiquée à la revendication 1 pour R_{3B} dans laquelle les éventuelles fonctions réactives sont éventuellement protégées par des groupements protecteurs, pour obtenir un produit de formule (II_{f}) : 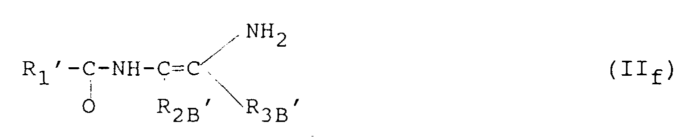 dans laquelle R₁', R_{2B}' et R_{3B}' ont les significations indiquées précédemment, que l'on soumet à une réaction de cyclisation pour obtenir un produit de formule (II), produit de formule (II) que l'on soumet, si désiré et si nécessaire, à l'une ou plusieurs des réactions suivantes, dans un ordre quelconque :
a) une réaction d'élimination des groupements protecteurs que peuvent porter les fonctions réactives protégées,
b) une réaction de salification par un acide minéral ou organique ou par une base pour obtenir le sel correspondant,
c) une réaction d'estérification de fonction acide,
d) une réaction de saponification de fonction ester en fonction acide,
e) une réaction de transformation de la fonction cyano en fonction acide,
f) une réaction de réduction de la fonction carboxy en fonction alcool,
g) une réaction de transformation de fonction alcoxy en fonction hydroxyle,
h) une réaction d'oxydation de groupement alkylthio ou arylthio en sulfoxyde ou sulfone correspondant,
i) une réaction de transformation de fonction sulfoxyde ou sulfone en fonction sulfoximine correspondante,
j) une réaction d'oxydation de fonction alcool en fonction aldéhyde ou acide,
k) une réaction de transformation de fonction nitrile en tétrazole,
l) une réaction de dédoublement des formes racémiques en produits dédoublés,
m) une réaction de transformation du radical formyle en radical carbamoyle,
n) une réaction de transformation du radical carbamoyle en radical nitrile,
lesdits produits de formule (II) ainsi obtenus étant sous toutes les formes isomères possibles racémiques, énantiomères et diastéréoisomères.

## Revendications (Revendications pour l'(les) Etat(s) contractant(s) suivant(s): GR)

1. Procédé de préparation de produits de formule (I_{B}) : 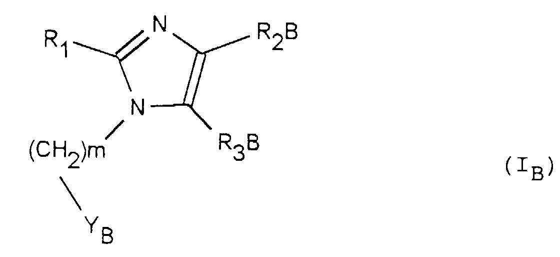 dans laquelle :
R₁ représente un radical alkyle renfermant au plus 4 atomes de carbone, R_{2B} et R_{3B}, identiques ou différents sont choisis parmi :
- l'atome d'hydrogène,
- un radical carboxy libre, salifié ou estérifié par un radical alkyle,
- formyle, acyloxy choisi dans le groupe consistant en formyloxy, acétyloxy, propionyloxy, butyryloxy et benzoyloxy, sulfo,
- alkyle et alcoxy éventuellement substitués,
- phénylthio, phénylsulfonyle, phénylsulfinyle, alkylthio, alkylsulfonyle et alkylsulfinyle, éventuellement substitués, tel que dans tous ces radicaux que peuvent représenter R_{2B} et R_{3B}, les radicaux alkyle et alcoxy renferment au plus 6 atomes de carbone, et les radicaux alkyle, alcoxy et phényle sont éventuellement substitués par un ou plusieurs radicaux choisis parmi les atomes d'halogène et les radicaux hydroxyle, trifluorométhyle, acyloxy, carboxy libre salifié ou estérifié, phényle, pyridyle, tétrazolyle, alkyle et alcoxy renfermant au plus 4 atomes de carbone et eux-mêmes éventuellement substitués par un radical alcoxy renfermant au plus 4 atomes de carbone, étant entendu que l'un au moins des groupes R_{2B} et R_{3B} représente un groupe choisi parmi phénylthio, phénylsulfonyle, phénylsulfinyle, alkylthio, alkylsulfonyle et alkylsulfinyle, éventuellement substitués, et Y_{B} représente le radical -Y_{1B}-B-Y_{2B} dans lequel Y_{1B} représente un radical phényle, B représente une liaison simple carbone-carbone et Y_{2B} représente un radical carboxy libre ou estérifié par un radical alkyle linéaire ou ramifié renfermant au plus 4 atomes de carbone ou un radical phényle éventuellement substitué par un radical carboxy libre ou estérifié par un radical alkyle linéaire ou ramifié renfermant au plus 4 atomes de carbone, un radical cyano ou un radical tétrazolyle éventuellement salifié, lesdits produits de formule (I_{B}) étant sous toutes les formes isomères racémiques, énantiomères et diastéréoisomères possibles, ainsi que les sels d'addition avec les acides minéraux et organiques ou avec les bases minérales et organiques desdits produits de formule (I_{B}), caractérisé en ce que l'on fait réagir un composé de formule (II) : 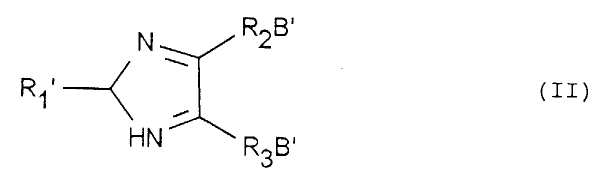
dans laquelle R₁', R_{2B}' et R_{3B}' ont les significations indiquées à la revendication 1 respectivement pour R₁, R_{2B} et R_{3B} et dans lesquelles les éventuelles fonctions réactives sont éventuellement protégées par des groupements protecteurs, avec un composé de formule (III) :
Z-(CH₂)ₘ-Y_{B}' (III)
dans laquelle Z représente un atome d'halogène ou un sulfonate et Y_{B}' a la signification indiquée à la revendication 1 pour Y_{B} dans laquelle les éventuelles fonctions réactives sont éventuellement protégées par des groupements protecteurs, pour obtenir un produit de formule (IV) : 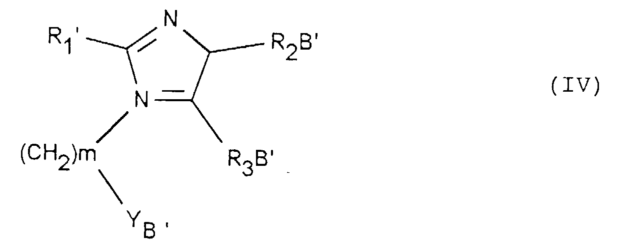 dans laquelle R₁', R_{2B}', R_{3B}' et Y_{B}' ont les significations indiquées ci-dessus,
produit de formule (IV) que l'on soumet, si désiré et si nécessaire, à l'une ou plusieurs des réactions suivantes, dans un ordre quelconque :
a) une réaction d'élimination des groupements protecteurs que peuvent porter les fonctions réactives protégées,
b) une réaction de salification par un acide minéral ou organique ou par une base pour obtenir le sel correspondant,
c) une réaction d'estérification de fonction acide,
d) une réaction de saponification de fonction ester en fonction acide,
e) une réaction de transformation de la fonction cyano en fonction acide,
f) une réaction de réduction de la fonction carboxy en fonction alcool,
g) une réaction de transformation de fonction alcoxy en fonction hydroxyle,
h) une réaction d'oxydation de groupement alkylthio ou arylthio en sulfoxyde ou sulfone correspondant,
i) une réaction de transformation de fonction sulfoxyde ou sulfone en fonction sulfoximine correspondante,
j) une réaction d'oxydation de fonction alcool en fonction aldéhyde ou acide,
k) une réaction de transformation de radical nitrile en tétrazole,
l) une réaction de dédoublement des formes racémiques en produits dédoublés,
m) une réaction de transformation du radical formyle en radical carbamoyle,
n) une réaction de transformation du radical carbamoyle en radical nitrile,
lesdits produits de formule (I_{B}) ainsi obtenus étant sous toutes les formes isomères possibles racémiques, énantiomères et diastéréoisomères.

2. Procédé selon la revendication 1 pour la préparation des produits de formule (I_{B}) répondant à la formule (Iₐ) : 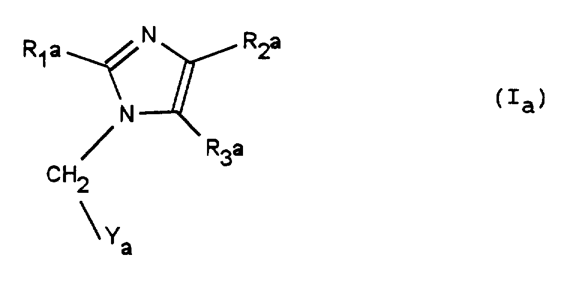 dans laquelle :
R₁ₐ représente un radical alkyle linéaire ou ramifié renfermant au plus 4 atomes de carbone,
R₂ₐ et R₃ₐ, identiques ou différents, sont choisis parmi :
a) l'atome d'hydrogène, le radical mercapto, les radicaux formyle, carboxy libre, salifié ou estérifié,
b) les radicaux R₄ₐ et -S(O)ₙR₄ₐ tel que n représente la valeur 0, 1 ou 2,
dans lesquels R₄ₐ représente un radical alkyle ou alkényle linéaire ou ramifié renfermant au plus 4 atomes de carbone, un radical cyclohexyle, un radical phényle, pyridyle, pyrimidinyle ou imidazolyle, tous ces radicaux étant éventuellement substitués par un ou plusieurs radicaux identiques ou différents choisis-parmi :
-- les atomes d'halogène,
-- les radicaux hydroxyle, mercapto, acylthio, trifluorométhyle, trifluorométhylthio, trifluorométhoxy, cyano, azido, nitro,
-- le radical phényle,
-- les radicaux alkyle et alcoxy renfermant au plus 4 atomes de carbone, carboxy libre, salifié ou estérifié, tétrazolyle, acyle, acyloxy, et
-- les radicaux 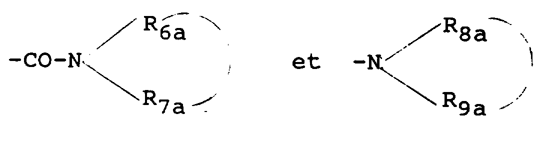
dans lesquels :
**ou bien** R₆ₐ, R₇ₐ, R₈ₐ et R₉ₐ, identiques ou différents, sont choisis parmi l'atome d'hydrogène, le radical hydroxyle, les radicaux alkyle, alcoxy, acyloxy ou acyle, ces radicaux renfermant au plus 6 atomes de carbone, carboxy libre, salifié ou estérifié, phényle, benzyle, phénéthyle, azépine, pipéridyle, morpholine, pyrrolidinyle, pipérazinyle,
**ou bien** d'une part R₆ₐ et R₇ₐ et d'autre part R₈ₐ et R₉ₐ forment respectivement avec l'atome d'azote auxquels ils sont liés un radical, ces radicaux identiques ou différents étant choisis parmi les radicaux imidazolyle, pyrrolyle, pyrrolinyle, pyrrolidinyle, pyridyle, pipéridinyle, pyrimidinyle, pyridazinyle, pyrazinyle, pipérazinyle, phénylpipérazinyle, pipéridyle, oxazolyle, morpholinyle et thiomorpholinyle, azépine, indolyle, ces radicaux étant éventuellement substitués par un ou plusieurs radicaux identiques ou différents choisis parmi les atomes d'halogène, les radicaux hydroxyle, trifluorométhyle, alkyle et alcoxy, ces radicaux renfermant au plus 6 atomes de carbone, étant entendu que l'un au moins de R₂ₐ ou R₃ₐ représente le radical -S(O)ₙR₄ₐ,
Yₐ représente le radical -Y₁ₐ-Bₐ-Y₂ₐ dans lequel :
- Y₁ₐ représente un radical phényle,
- Bₐ représente une simple liaison ou le radical -CO-NH-,
- Y₂ₐ est tel que :
**soit**, si Bₐ représente une simple liaison ou un radical -CO-NH-, Y₂ₐ représente un radical phényle substitué en ortho par un radical carboxy libre, salifié ou estérifié ou un radical tétrazolyle,
**soit**, si Bₐ représente une simple liaison, Y₂ₐ représente un radical cyano ou carboxy libre, salifié ou estérifié ou un radical tétrazolyle,
lesdits produits de formule (Iₐ) étant sous toutes les formes isomères possibles racémiques, énantiomères et diastéréoisomères, ainsi que les sels d'addition avec les acides minéraux et organiques ou avec les bases minérales et organiques desdits produits de formule (Iₐ), caractérisé en ce que l'on utilise au départ un produit de formule (II) et (III) dans lesquels R'₁, R'_{2B}, R'_{3B} et Y'_{B} ont les valeurs indiquées ci-dessus respectivement pour R₁ₐ, R₂ₐ, R₃ₐ et Yₐ dans lesquelles les fonctions réactives sont éventuellement protégées.

3. Procédé selon la revendication 2, caractérisé en ce que l'on utilise au départ un produit de formule (II) dans laquelle R'₁ représente un radical alkyle renfermant au plus 4 atomes de carbone, R'_{2B} et R'_{3B} identiques ou différents sont choisis parmi les radicaux :
- carboxy libre, salifié ou estérifié par un radical alkyle linéaire ou ramifié renfermant au plus 4 atomes de carbone, formyle, acyloxy, choisi dans le groupe consistant en formyloxy, acétyloxy, propionyloxy, butyryloxy et benzoyloxy.
- alkyle renfermant au plus 4 atomes de carbone éventuellement substitués par un radical hydroxyle,
- phénylthio, phénylsulfonyle, phénylsulfinyle,
- alkylthio, alkylsulfonyle et alkylsulfinyle,
dans lesquels le radical alkyle renferme au plus 4 atomes de carbone, dans lesquels les fonctions réactives sont éventuellement protégées et un produit de formule (III) dans laquelle Y'_{B} représente le radical -Y₁-B-Y₂ dans lequel Y₁ représente un radical phényle, B représente une liaison simple carbone-carbone et Y₂ représente un radical carboxy libre ou estérifié par un radical alkyle linéaire ou ramifié renfermant au plus 4 atomes de carbone ou un radical phényle éventuellement substitué par un radical carboxy libre ou estérifié par un radical alkyle linéaire ou ramifié renfermant au plus 4 atomes de carbone, dans lesquels les fonctions réactives sont éventuellement protégées.

4. Procédé selon la revendication 1 ou 2, caractérisé en ce que l'on utilise au départ un produit de formule (II) dans laquelle R'_{2B} ou R'_{3B} représente un radical alcoxy, alkylthio ou phénylthio ces deux derniers étant éventuellement oxydés sous forme de sulfoxyde ou de sulfone et ces trois radicaux étant éventuellement substitués et l'autre représente un radical carboxy libre salifié ou estérifié.

5. Procédé de préparation des produits de départ de formule (II) telle que définie à la revendication 1, caractérisé en ce que l'on soumet un composé de formule (IIₐ) : 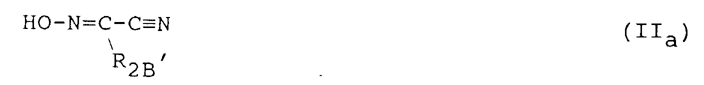 dans laquelle R_{2B}' a la signification indiquée à la revendication 1 pour R_{2B} dans laquelle les éventuelles fonctions réactives sont éventuellement protégées par des groupements protecteurs, que l'on soumet à l'action d'un agent réducteur, pour obtenir l'amine correspondante de formule (II_{b}) : 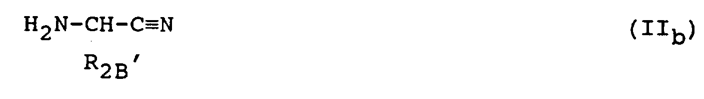 dans laquelle R_{2B}' a la signification indiquée précédemment, que l'on soumet à l'action d'un composé de formule (II_{c}) : 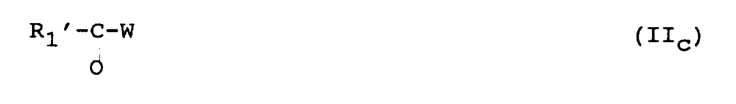 dans laquelle R₁' a la signification indiquée à la revendication 1 pour R₁ dans laquelle les éventuelles fonctions réactives sont éventuellement protégées par des groupements protecteurs et W représente un radical hydroxyle ou un atome d'halogène, pour obtenir un produit de formule (II_{d}) : 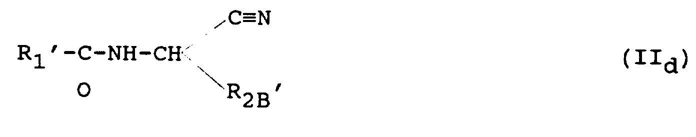 dans laquelle R₁' et R_{2B}' ont les significations indiquées précédemment, que l'on fait réagir avec un composé de formule (IIₑ) :
R_{3B}'-SH (IIₑ)
dans laquelle R_{3B}' a la signification indiquée à la revendication 1 pour R_{3B} dans laquelle les éventuelles fonctions réactives sont éventuellement protégées par des groupements protecteurs, pour obtenir un produit de formule (II_{f}) : 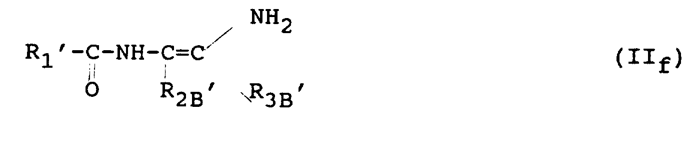 dans laquelle R₁', R_{2B}' et R_{3B}' ont les significations indiquées précédemment, que l'on soumet à une réaction de cyclisation pour obtenir un produit de formule (II), produit de formule (II) que l'on soumet, si désiré et si nécessaire, à l'une ou plusieurs des réactions suivantes, dans un ordre quelconque :
a) une réaction d'élimination des groupements protecteurs que peuvent porter les fonctions réactives protégées,
b) une réaction de salification par un acide minéral ou organique ou par une base pour obtenir le sel correspondant,
c) une réaction d'estérification de fonction acide,
d) une réaction de saponification de fonction ester en fonction acide,
e) une réaction de transformation de la fonction cyano en fonction acide,
f) une réaction de réduction de la fonction carboxy en fonction alcool,
g) une réaction de transformation de fonction alcoxy en fonction hydroxyle,
h) une réaction d'oxydation de groupement alkylthio ou arylthio en sulfoxyde ou sulfone correspondant,
i) une réaction de transformation de fonction sulfoxyde ou sulfone en fonction sulfoximine correspondante,
j) une réaction d'oxydation de fonction alcool en fonction aldéhyde ou acide,
k) une réaction de transformation de fonction nitrile en tétrazole,
l) une réaction de dédoublement des formes racémiques en produits dédoublés,
m) une réaction de transformation du radical formyle en radical carbamoyle,
n) une réaction de transformation du radical carbamoyle en radical nitrile,
lesdits produits de formule (II) ainsi obtenus étant sous toutes les formes isomères possibles racémiques, énantiomères et diastéréoisomères.

6. Procédé selon la revendication 1, caractérisé en ce que l'on utilise au départ des composés de formule (II) et (III) choisis de manière telle que l'on prépare les produits répondant aux formules suivantes :
- l'acide 2-butyl 1-[[2'-carboxy (1,1'-biphényl)-4-yl] méthyl] 4-(phénylthio) 1H-imidazole-5-carboxylique
- l'acide 2-butyl 1-[[2'-carboxy (1,1'-biphényl)-4-yl] méthyl] 4-(méthylthio) 1H-imidazole-5-carboxylique
- l'acide 4'-[[2-butyl 4-(éthylthio) 5-(hydroxyméthyl) 1H-imidazol-1-yl] méthyl] (1,1'-biphényl)-2-carboxylique
- l'acide 2-butyl 1-[[2'-carboxy (1,1'-biphényl)-4-yl] méthyl] 4-(éthylsulfonyl) 1H-imidazole-5-carboxylique
- l'acide 2-butyl 1-[[2'-carboxy (1,1'-biphényl)-4-yl] méthyl] 4-(éthylsulfinyl) 1H-imidazole-5-carboxylique
- l'acide 2-butyl 1-[[2'-carboxy (1,1'-biphényl)-4-yl] méthyl] 4-(éthylthio) 1H-imidazole-5-carboxylique
- l'acide 2-butyl 1-[[2'-carboxy (1,1'-biphényl)-4-yl] méthyl] 4-(phénylsulfonyl) lH-imidazole-5-carboxylique
- l'acide 2-butyl 1-[[2'-carboxy (1,1'-biphényl)-4-yl] méthyl] 4-(phénylsulfinyl) lH-imidazole-5-carboxylique
- l'acide 2-butyl 1-[[2'-tétrazolyl (1,1'-biphényl)-4-yl] méthyl] 4-(méthylthio) 1H-imidazole-5-carboxylique.

7. Procédé de préparation de compositions pharmaceutiques caractérisé en ce que l'on met à titre de principe actif l'un au moins des produits de formule (I_{B}) telle que définie à la revendication 1 ou l'un au moins de leurs sels d'addition avec les acides minéraux ou organiques ou avec les bases minérales ou organiques pharmaceutiquement acceptables, sous une forme destinée à cet usage.

8. Procédé de préparation de compositions pharmaceutiques caractérisé en ce que l'on met à titre de principe actif l'un au moins des produits de formule (I_{B}) telle que définie à l'une quelconque des revendication 2 à 4 ou l'un au moins de leurs sels d'addition avec les acides minéraux ou organiques ou avec les bases minérales ou organiques pharmaceutiquement acceptables, sous une forme destinée à cet usage.
au moins des produits de formule (I_{B}) telle que définie à l'une quelconque des revendication 2 à 4 ou l'un au moins de leurs sels d'addition avec les acides minéraux ou organiques ou avec les bases minérales ou organiques pharmaceutiquement acceptables, sous une forme destinée à cet usage.

9. Procédé de préparation de compositions pharmaceutiques caractérisé en ce que l'on met à titre de principe actif l'un au moins des produits de formule (I_{B}) telle que définie à la revendication 6 ou l'un au moins de leurs sels d'addition avec les acides minéraux ou organiques ou avec les bases minérales ou organiques pharmaceutiquement acceptables, sous une forme destinée à cet usage.

10. A titre de produits industriels nouveaux, les composés de formule (II) dans laquelle R₁' ne représente pas le radical méthyle.

## Patentansprüche (Patentansprüche für folgende(n) Vertragsstaat(en): AT, BE, CH, DE, DK, FR, GB, IT, LI, LU, NL)

1. Verbindungen der Formel (I_{B}): 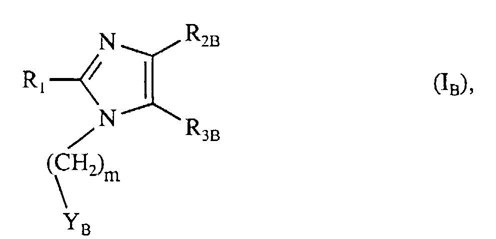 in der:
• R₁ einen Alkylrest darstellt, der maximal 4 Kohlenstoffatome enthält,
• R_{2B} und R_{3B}, die gleich oder verschieden sein können, ausgewählt sind unter:
- dem Wasserstoffatom,
- den freien, als Salz vorliegenden oder mit einem Alkylrest veresterten Carboxylresten,
- dem Formylrest, den Acyloxyresten, die unter den folgenden Resten ausgewählt sind: Formyloxy, Acetyloxy, Propionyloxy, Butyryloxy und Benzoyloxy, sowie dem Sulforest,
- den Alkyl- und Alkoxyresten, die gegebenenfalls substituiert sind, und
- den Phenylthio-, Phenylsulfonyl-, Phenylsulfinyl- sowie Alkylthio-, Alkylsulfonyl- und Alkylsulfinylresten, die gegebenenfalls substituiert sind,
wobei in all diesen Resten, die durch R_{2B} und R_{3B} dargestellt werden können, die Alkyl- und Alkoxyreste maximal 6 Kohlenstoffatome enthalten und die Alkyl-, Alkoxy- und Phenylreste gegebenenfalls mit einem oder mehreren Resten substituiert sind, die unter den Halogenatomen, der Hydroxylgruppe, dem Trifluormethyl- und den Acyloxyresten, den freien, als Salz vorliegenden oder veresterten Carboxylresten, dem Phenyl-, dem Pyridyl- und dem Tetrazolylrest sowie den Alkyl- und Alkoxyresten mit maximal 4 Kohlenstoffatomen ausgewählt sind, die wiederum gegebenenfalls mit einem Alkoxyrest substituiert sind, der maximal 4 Kohlenstoffatome enthält, mit der Maßgabe, daß zumindest eine der beiden Gruppen R_{2B} und R_{3B} eine Gruppe darstellt, die unter den folgenden Gruppen ausgewählt ist: Phenylthio-, Phenylsulfonyl-, Phenylsulfinyl- sowie Alkylthio-, Alkylsulfonyl- und Alkylsulfinylresten, die gegebenenfalls substituiert sind, und
• Y_{B} den Rest -Y_{1B}-B-Y_{2B} darstellt, in dem:
- Y_{1B} einen Phenylrest bezeichnet,
- B eine Kohlenstoff-Kohlenstoff-Einfachbindung bezeichnet und
- Y_{2B} einen der folgenden Reste bezeichnet:
* einen freien oder mit einem geradkettigen oder verzweigten Alkylrest mit maximal 4 Kohlenstoffatomen veresterten Carboxylrest,
* einen Phenylrest, der gegebenenfalls mit einem freien oder mit einem geradkettigen oder verzweigten Alkylrest mit maximal 4 Kohlenstoffatomen veresterten Carboxylrest substituiert ist,
* eine Nitrilgruppe oder
* einen gegebenenfalls als Salz vorliegenden Tetrazolylrest,
wobei diese Verbindungen der Formel (I_{B}) sowohl in allen möglichen racemischen, enantiomeren und diastereomeren isomeren Formen als auch in Form von Salzen vorliegen können, die durch eine Addition dieser Verbindungen der Formel (I_{B}) an mineralische oder organische Säuren beziehungsweise mineralische oder organische Basen gebildet werden.

2. Verbindungen der Formel (I_{B}) wie in Anspruch 1 definiert, die der Formel (Iₐ) entsprechen: 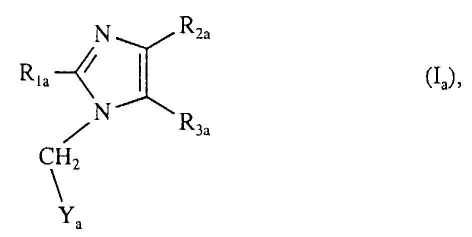 in der:
• R₁ₐ einen geradkettigen oder verzweigten Alkylrest mit maximal 4 Kohlenstoffatomen darstellt,
• R₂ₐ und R₃ₐ, die gleich oder verschieden sein können, ausgewählt sind unter:
a) dem Wasserstoffatom, dem Mercaptorest, dem Formylrest und den freien, als Salz vorliegenden oder veresterten Carboxylresten,
b) den Resten R₄ₐ und -S(O)ₙR₄ₐ, in denen n gleich 0, 1 oder 2 ist,
wobei R₄ₐ in diesen Resten einen geradkettigen oder verzweigten Alkyl- oder Alkenylrest mit maximal 4 Kohlenstoffatomen, einen Cyclohexylrest, einen Phenyl-, Pyridyl-, Pyrimidinyl- oder Imidazolylrest darstellt, wobei all diese Reste gegebenenfalls mit einem oder mehreren Resten substituiert sind, die gleich oder verschieden sein können und die ausgewählt sind unter:
-- den Halogenatomen,
-- der Hydroxylgruppe, dem Mercaptorest, den Acylthioresten, dem Trifluormethyl-, dem Trifluormethylthio- und dem Trifluormethoxyrest sowie der Nitril-, der Azid- und der Nitrogruppe,
-- dem Phenylrest,
-- den Alkyl- und Alkoxyresten mit maximal 4 Kohlenstoffatomen, den freien, als Salz vorliegenden oder veresterten Carboxylresten, dem Tetrazolylrest, sowie den Acyl- und Acyloxyresten und
-- den Resten: 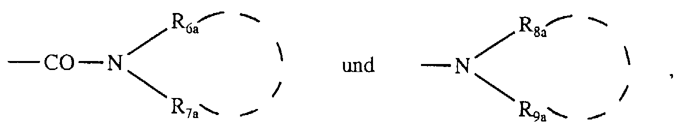 in denen:
**entweder**
R₆ₐ, R₇ₐ, R₈ₐ und R₉ₐ, die gleich oder verschieden sein können, ausgewählt sind unter dem Wasserstoffatom, der Hydroxylgruppe, den Alkyl-, Alkoxy-, Acyloxy- und Acylgruppen mit maximal 6 Kohlenstoffatomen, den freien, als Salz vorliegenden oder veresterten Carboxylresten sowie den folgenden Resten: Phenyl, Benzyl, Phenylethyl, Azepin, Piperidyl, Morpholin, Pyrrolidinyl und Piperazinyl,
**oder**
R₆ₐ und R₇ₐ beziehungsweise R₈ₐ und R₉ₐ zusammen mit dem Stickstoffatom, an das sie gebunden sind, einen Rest bilden, wobei diese Reste, die gleich oder verschieden sein können, unter folgenden Resten ausgewählt sind: Imidazolyl, Pyrrolyl, Pyrrolinyl, Pyrrolidinyl, Pyridyl, Piperidinyl, Pyrimidinyl, Pyridazinyl, Pyrazinyl, Piperazinyl, Phenylpiperazinyl, Piperidyl, Oxazolyl, Morpholinyl und Thiomorpholinyl, Azepin und Indolyl, wobei diese Reste gegebenenfalls mit einem oder mehreren Resten substituiert sind, die gleich oder verschieden sein können und die unter den Halogenatomen, der Hydroxylgruppe, dem Trifluormethylrest sowie den Alkyl- und Alkoxyresten mit maximal 6 Kohlenstoffatomen ausgewählt sind,
mit der Maßgabe, daß zumindest einer der beiden Reste R₂ₐ und R₃ₐ den Rest -S(O)ₙR₄ₐ darstellt,
• Yₐ den Rest -Y₁ₐ-Bₐ-Y₂ₐ darstellt, in dem:
- Y₁ₐ einen Phenylrest bezeichnet,
- Bₐ eine Einfachbindung oder den Rest -CO-NH- bezeichnet und
- Y₂ₐ:
**entweder**, wenn Bₐ eine Einfachbindung oder den Rest -CO-NH- bezeichnet, einen Phenylrest, der in ortho-Stellung mit einem freien, als Salz vorliegenden oder veresterten Carboxylrest substituiert ist, oder einen Tetrazolylrest bezeichnet,
**oder,** wenn Bₐ eine Einfachbindung darstellt, eine Nitrilgruppe, einen freien, als Salz vorliegenden oder veresterten Carboxylrest oder einen Tetrazolylrest bezeichnet,
wobei diese Verbindungen der Formel (Iₐ) sowohl in allen möglichen racemischen, enantiomeren und diastereomeren isomeren Formen als auch in Form von Salzen vorliegen können, die durch eine Addition dieser Verbindungen der Formel (Iₐ) an mineralische oder organische Säuren beziehungsweise mineralische oder organische Basen gebildet werden.

3. Verbindungen der Formel (I_{B}) wie in Anspruch 1 definiert, wobei in dieser Formel:
• R₁ einen Alkylrest mit maximal 4 Kohlenstoffatomen darstellt,
• R_{2B} und R_{3B}, die gleich oder verschieden sein können, unter den folgenden Resten ausgewählt sind:
- den freien, als Salz vorliegenden oder mit einem geradkettigen oder verzweigten Alkylrest mit maximal 4 Kohlenstoffatomen veresterten Carboxylresten, dem Formylrest, den Acyloxyresten, die ausgewählt sind unter dem Formyloxy-, dem Acetyloxy-, dem Propionyloxy-, dem Butyryloxy- und dem Benzoyloxyrest,
- den Alkylresten mit maximal 4 Kohlenstoffatomen, die gegebenenfalls mit einer Hydroxylgruppe substituiert sind,
- dem Phenylthio-, dem Phenylsulfonyl- und dem Phenylsulfinylrest sowie
- den Alkylthio-, Alkylsulfonyl- und Alkylsulfinylresten, deren Alkylreste maximal 4 Kohlenstoffatome enthalten,
und
• Y_{B} den Rest -Y_{1B}-B-Y_{2B} darstellt, in dem
- Y_{1B} einen Phenylrest bezeichnet,
- B eine Kohlenstoff-Kohlenstoff-Einfachbindung bezeichnet und
- Y_{2B} einen freien oder mit einem geradkettigen oder verzweigten Alkylrest mit maximal 4 Kohlenstoffatomen veresterten Carboxylrest oder einen gegebenenfalls mit einem freien oder mit einem geradkettigen oder verzweigten Alkylrest mit maximal 4 Kohlenstoffatomen veresterten Carboxylrest substituierten Phenylrest bezeichnet,
wobei diese Verbindungen der Formel (I_{B}) sowohl in allen möglichen racemischen, enantiomeren und diastereomeren isomeren Formen als auch in Form von Salzen vorliegen können, die durch eine Addition dieser Verbindungen der Formel (I_{B}) an mineralische oder organische Säuren beziehungsweise mineralische oder organische Basen gebildet werden.

4. Verbindungen der Formel (I_{B}) wie in Anspruch 1 definiert, wobei in dieser Formel (I_{B}) entweder R_{2B} oder R_{3B} einen Alkoxy-, Alkylthio- oder Phenylthiorest darstellt, wobei der Alkylthiorest und der Phenylthiorest gegebenenfalls in oxidierter Form als Sulfoxid oder Sulfon vorliegen und diese drei Reste gegebenenfalls substituiert sind, und der andere der beiden Reste R_{2B} und R_{3B} einen freien, als Salz vorliegenden oder veresterten Carboxylrest darstellt, wobei diese Verbindungen der Formel (I_{B}) sowohl in allen möglichen racemischen, enantiomeren und diastereomeren isomeren Formen als auch in Form von Salzen vorliegen können, die durch eine Addition dieser Verbindungen der Formel (I_{B}) an mineralische oder organische Säuren beziehungsweise mineralische oder organische Basen gebildet werden.

5. Verbindungen der Formel (I_{B}), die den folgenden chemischen Bezeichnungen entsprechen:
- 2-Butyl-1-[[2'-carboxy-(1,1'-biphenyl)-4-yl]-methyl]-4-(phenylthio)-1H-imidazol-5-carbonsäure,
- 2-Butyl-1-[[2'-carboxy-(1,1'-biphenyl)-4-yl]-methyl]-4-(methylthio)-1H-imidazol-5-carbonsäure,
- 4'-[[2-Butyl-4-(ethylthio)-5-hydroxylmethyl)-1H-imidazol-1-yl]-methyl]-(1,1'-biphenyl)-2-carbonsäure,
- 2-Butyl-1-[[2'-carboxy-(1,1'-biphenyl)-4-yl]-methyl] -4-(ethylsulfonyl)-1H-imidazol-5-carbonsäure,
- 2-Butyl-1-[[2'-carboxy-(1,1'-biphenyl)-4-yl]-methyl]-4-(ethylsulfinyl)-1H-imidazol-5-carbonsäure,
- 2-Butyl-1-[[2'-carboxy-(1,1'-biphenyl)-4-yl]-methyl]-4-(ethylthio)-1H-imidazol-5-carbonsäure,
- 2-Butyl-1-[[2'-carboxy-(1,1'-biphenyl)-4-yl]-methyl]-4-(phenylsulfonyl)-1H-imidazol-5-carbonsäure,
- 2-Butyl-1-[[2'-carboxy-(1,1'-biphenyl)-4-yl]-methyl]-4-(phenylsulfinyl)-1H-imidazol-5-carbonsäure,
- 2-Butyl-1-[[2'-tetrazolyl-(1,1'-biphenyl)-4-yl]-methyl]-4-(methylthio)-1H-imidazol-5-carbonsäure.

6. Verfahren zur Herstellung von Verbindungen der Formel (I_{B}) wie in Anspruch 1 definiert, dadurch gekennzeichnet, daß eine Verbindung der Formel (II): 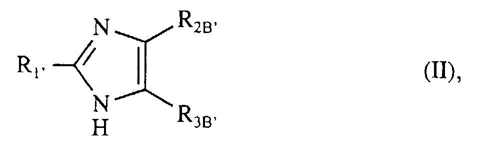 in der R_{1'}, R_{2B'} und R_{3B}, die in Anspruch 1 für R₁ beziehungsweise R_{2B} oder R_{3B} angegebene Bedeutung besitzen, wobei die in diesen Gruppen gegebenenfalls vorhandenen reaktiven Gruppen gegebenenfalls durch Schutzgruppen geschützt sind,
mit einer Verbindung der folgenden Formel (III) umgesetzt wird:
Z-(CH₂)ₘ-Y_{B'} (III),
in der Z ein Halogenatom oder eine Sulfonatgruppe darstellt und Y_{B'} die in Anspruch 1 für Y_{B} angegebene Bedeutung besitzt, wobei die in dieser Gruppe gegebenenfalls vorhandenen reaktiven Gruppen gegebenenfalls durch Schutzgruppen geschützt sind,
wobei eine Verbindung der Formel (IV) erhalten wird: 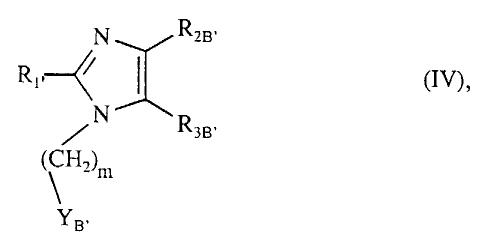 in der R_{1'}, R_{2B'}, R_{3B}, und Y_{B'} die oben angegebene Bedeutung besitzen, wobei die Verbindung der Formel (IV), sofern dies gewünscht wird beziehungsweise notwendig ist, einer oder mehreren der folgenden Reaktionen unterworfen wird, die in beliebiger Reihenfolge durchgeführt werden können:
a) einer Eliminierung der Schutzgruppen, die die geschützten reaktiven Gruppen tragen können,
b) einer Salzbildung mit einer mineralischen oder organischen Säure oder mit einer Base, bei der das entsprechende Salz gebildet wird,
c) einer Veresterung der Säurefunktion,
d) einer Verseifung der Esterfunktion zur Säurefunktion,
e) einer Umsetzung der Nitrilfunktion zur Säurefunktion,
f) einer Reduktion der Carboxylfunktion zur Alkoholfunktion,
g) einer Umsetzung der Alkoxyfunktion zur Hydroxylfunktion,
h) einer Oxidation der Alkylthio- beziehungsweise Arylthiogruppe zum entsprechenden Sulfoxid oder Sulfon,
i) einer Umsetzung der Sulfoxid- beziehungsweise Sulfonfunktion zur entsprechenden Sulfoximinfunktion,
j) einer Oxidation der Alkoholfunktion zur Aldehyd- oder Säurefunktion,
k) einer Umsetzung der Nitrilgruppe zum Tetrazol,
l) einer Spaltung der Racemformen zu den enantiomerenreinen Verbindungen,
m) einer Umsetzung des Formylrests zum Carbamoylrest und
n) einer Umsetzung des Carbamoylrests zur Nitrilgruppe,
wobei die so erhaltenen Verbindungen der Formel (I_{B}) in allen möglichen racemischen, enantiomeren und diastereomeren isomeren Formen vorliegen können.

7. Verbindungen, die durch die Formel (I_{B}) des Anspruchs 1 definiert sind und die sowohl in allen möglichen racemischen, enantiomeren und diastereomeren isomeren Formen als auch in Form von Salzen vorliegen können, die durch eine Addition dieser Verbindungen der Formel (I_{B}) an pharmazeutisch akzeptable mineralische oder organische Säuren beziehungsweise Basen gebildet werden, in ihrer Eigenschaft als Arzneimittel.

8. Verbindungen der Formel (I_{B}) und der Formel (Iₐ), die in den Ansprüchen 2 bis 4 definiert sind, sowie Salze, die durch eine Addition dieser Verbindungen der Formeln (I_{B}) und (Iₐ) an pharmazeutisch akzeptable mineralische oder organische Säuren beziehungsweise Basen gebildet werden, in ihrer Eigenschaft als Arzneimittel.

9. Pharmazeutische Zusammensetzungen, die als Wirkstoff mindestens eines der in den Ansprüchen 7 und 8 definierten Arzneimittel enthalten.

10. Verfahren zur Herstellung der Ausgangsverbindungen der Formel (II) wie in Anspruch 6 definiert, dadurch gekennzeichnet, daß eine Verbindung der Formel (IIₐ): 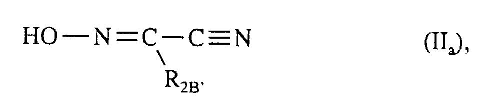 in der R_{2B'} die in Anspruch 1 für R_{2B} angegebene Bedeutung besitzt und in der die gegebenenfalls vorhandenen reaktiven Gruppen gegebenenfalls mit Schutzgruppen geschützt sind,
mit einem Reduktionsmittel umgesetzt wird, wobei das entsprechende Amin der folgenden Formel (II_{b}) erhalten wird: 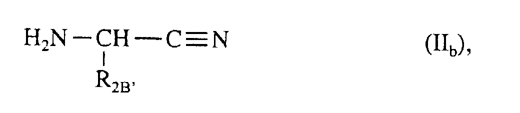 in der R_{2B'} die oben angegebene Bedeutung besitzt;
diese Verbindung der Formel (II_{b}) wird mit einer Verbindung der Formel (II_{c}) umgesetzt: 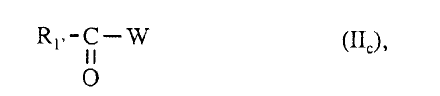 in der:
• R_{1'} die in Anspruch 1 für R₁ angegebene Bedeutung besitzt, wobei die in diesem Rest gegebenenfalls vorhandenen reaktiven Gruppen gegebenenfalls mit Schutzgruppen geschützt sind,
und
• W eine Hydroxylgruppe oder ein Halogenatom darstellt,
wobei eine Verbindung der folgenden Formel (II_{d}) erhalten wird: 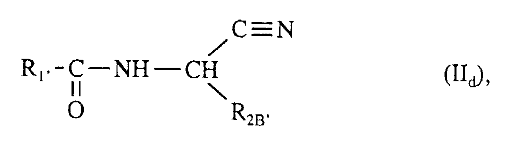 in der R_{1'} und R_{2B'} die oben angegebene Bedeutung besitzen und
die mit einer Verbindung der Formel (IIₑ) umgesetzt wird:
R_{3B'}―SH (IIₑ),
in der R_{3B'} die in Anspruch 1 für R_{3B} angegebene Bedeutung besitzt und in der die gegebenenfalls vorhandenen reaktiven Gruppen gegebenenfalls mit Schutzgruppen geschützt sind,
wobei eine Verbindung der Formel (II_{f}) erhalten wird: 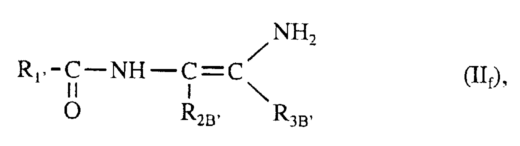 in der R_{1'}, R_{2B'} und R_{3B'} die oben angegebene Bedeutung besitzen und
die einer Cyclisierungsreaktion unterworfen wird, bei der eine Verbindung der Formel (II) erhalten wird, welche man, sofern dies gewünscht wird beziehungsweise notwendig ist, einer oder mehreren der folgenden Reaktionen unterwirft, die in beliebiger Reihenfolge durchgeführt werden können:
a) einer Eliminierung der Schutzgruppen, die die geschützten reaktiven Gruppen tragen können,
b) einer Salzbildung mit einer mineralischen oder organischen Säure oder mit einer Base, bei der das entsprechende Salz gebildet wird,
c) einer Veresterung der Säurefunktion,
d) einer Verseifung der Esterfunktion zur Säurefunktion,
e) einer Umsetzung der Nitrilfunktion zur Säurefunktion,
f) einer Reduktion der Carboxylfunktion zur Alkoholfunktion,
g) einer Umsetzung der Alkoxyfunktion zur Hydroxyfunktion,
h) einer Oxidation der Alkylthio- beziehungsweise Arylthiogruppe zum entsprechenden Sulfoxid oder Sulfon,
i) einer Umsetzung der Sulfoxid- beziehungsweise Sulfonfunktion zur entsprechenden Sulfoximinfunktion,
j) einer Oxidation der Alkoholfunktion zur Aldehyd- oder Säurefunktion,
k) einer Umsetzung der Nitrilfunktion zum Tetrazol,
l) einer Spaltung der Racemformen zu den enantiomerenreinen Verbindungen,
m) einer Umsetzung des Formylrests zum Carbamoylrest und
n) einer Umsetzung des Carbamoylrests zur Nitrilgruppe,
wobei die so erhaltenen Verbindungen der Formel (II) in allen möglichen racemischen, enantiomeren und diastereomeren isomeren Formen vorliegen können.

11. Verbindungen der Formel (II), in denen R_{1'} keine Methylgruppe darstellt.

## Patentansprüche (Patentansprüche für folgende(n) Vertragsstaat(en): ES)

1. Verfahren zur Herstellung von Verbindungen der Formel (I_{B}): 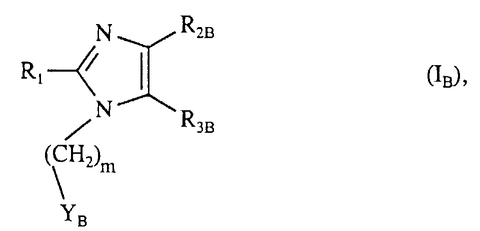 in der:
• R₁ einen Alkylrest darstellt, der maximal 4 Kohlenstoffatome enthält,
• R_{2B} und R_{3B}, die gleich oder verschieden sein können, ausgewählt sind unter:
- dem Wasserstoffatom,
- den freien, als Salz vorliegenden oder mit einem Alkylrest veresterten Carboxylresten,
- dem Formylrest, den Acyloxyresten, die unter den folgenden Resten ausgewählt sind: Formyloxy, Acetyloxy, Propionyloxy, Butyryloxy und Benzoyloxy, sowie dem Sulforest,
- den Alkyl- und Alkoxyresten, die gegebenenfalls substituiert sind,
und
- den Phenylthio-, Phenylsulfonyl-, Phenylsulfinyl- sowie Alkylthio-, Alkylsulfonyl- und Alkylsulfinylresten, die gegebenenfalls substituiert sind,
wobei in all diesen Resten, die durch R_{2B} und R_{3B} dargestellt werden können, die Alkyl- und Alkoxyreste maximal 6 Kohlenstoffatome enthalten und die Alkyl-, Alkoxy- und Phenylreste gegebenenfalls mit einem oder mehreren Resten substituiert sind, die unter den Halogenatomen, der Hydroxylgruppe, dem Trifluormethyl- und den Acyloxyresten, den freien, als Salz vorliegenden oder veresterten Carboxylresten, dem Phenyl-, dem Pyridyl- und dem Tetrazolylrest sowie den Alkyl- und Alkoxyresten mit maximal 4 Kohlenstoffatomen ausgewählt sind, die wiederum gegebenenfalls mit einem Alkoxyrest substituiert sind, der maximal 4 Kohlenstoffatome enthält,
mit der Maßgabe, daß zumindest eine der beiden Gruppen R_{2B} und R_{3B} eine Gruppe darstellt, die unter den folgenden Gruppen ausgewählt ist: Phenylthio-, Phenylsulfonyl-, Phenylsulfinyl- sowie Alkylthio-, Alkylsulfonyl- und Alkylsulfinylresten, die gegebenenfalls substituiert sind,
und
• Y_{B} den Rest -Y_{1B}-B-Y_{2B} darstellt, in dem:
- Y_{1B} einen Phenylrest bezeichnet,
- B eine Kohlenstoff-Kohlenstoff-Einfachbindung bezeichnet und
- Y_{2B} einen der folgenden Reste bezeichnet:
* einen freien oder mit einem geradkettigen oder verzweigten Alkylrest mit maximal 4 Kohlenstoffatomen veresterten Carboxylrest,
* einen Phenylrest, der gegebenenfalls mit einem freien oder mit einem geradkettigen oder verzweigten Alkylrest mit maximal 4 Kohlenstoffatomen veresterten Carboxylrest substituiert ist,
* eine Nitrilgruppe oder
* einen gegebenenfalls als Salz vorliegenden Tetrazolylrest,
wobei diese Verbindungen der Formel (I_{B}) sowohl in allen möglichen racemischen, enantiomeren und diastereomeren isomeren Formen als auch in Form von Salzen vorliegen können, die durch eine Addition dieser Verbindungen der Formel (I_{B}) an mineralische oder organische Säuren beziehungsweise mineralische oder organische Basen gebildet werden, dadurch gekennzeichnet, daß eine Verbindung der Formel (II): 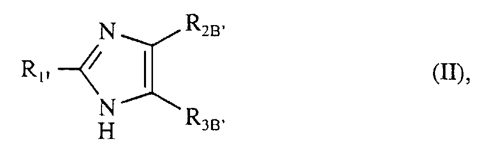 in der R_{1'}, R_{2B'} und R_{3B'} die in Anspruch 1 für R₁ beziehungsweise R_{2B} oder R_{3B} angegebene Bedeutung besitzen, wobei die in diesen Gruppen gegebenenfalls vorhandenen reaktiven Gruppen gegebenenfalls durch Schutzgruppen geschützt sind,
mit einer Verbindung der folgenden Formel (III) umgesetzt wird:
Z-(CH₂)ₘ-Y_{B'} (III),
in der Z ein Halogenatom oder eine Sulfonatgruppe darstellt und Y_{B}, die in Anspruch 1 für Y_{B} angegebene Bedeutung besitzt, wobei die in dieser Gruppe gegebenenfalls
vorhandenen reaktiven Gruppen gegebenenfalls durch Schutzgruppen geschützt sind, wobei eine Verbindung der Formel (IV) erhalten wird: 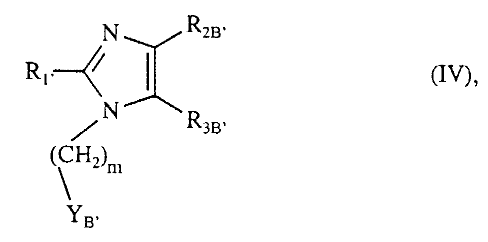 in der R_{1'}, R_{2B'}, R_{3B'} und Y_{B'} die oben angegebene Bedeutung besitzen,
wobei die Verbindung der Formel (IV), sofern dies gewünscht wird beziehungsweise notwendig ist, einer oder mehreren der folgenden Reaktionen unterworfen wird, die in beliebiger Reihenfolge durchgeführt werden können:
a) einer Eliminierung der Schutzgruppen, die die geschützten reaktiven Gruppen tragen können,
b) einer Salzbildung mit einer mineralischen oder organischen Säure oder mit einer Base, bei der das entsprechende Salz gebildet wird,
c) einer Veresterung der Säurefunktion,
d) einer Verseifung der Esterfunktion zur Säurefunktion,
e) einer Umsetzung der Nitrilfunktion zur Säurefunktion,
f) einer Reduktion der Carboxylfunktion zur Alkoholfunktion,
g) einer Umsetzung der Alkoxyfunktion zur Hydroxylfunktion,
h) einer Oxidation der Alkylthio- beziehungsweise Arylthiogruppe zum entsprechenden Sulfoxid oder Sulfon,
i) einer Umsetzung der Sulfoxid- beziehungsweise Sulfonfunktion zur entsprechenden Sulfoximinfunktion,
j) einer Oxidation der Alkoholfunktion zur Aldehyd- oder Säurefunktion,
k) einer Umsetzung der Nitrilgruppe zum Tetrazol,
l) einer Spaltung der Racemformen zu den enantiomerenreinen Verbindungen
m) einer Umsetzung des Formylrests zum Carbamoylrest und
n) einer Umsetzung des Carbamoylrests zur Nitrilgruppe,
wobei die so erhaltenen Verbindungen der Formel (I_{B}) in allen möglichen racemischen, enantiomeren und diastereomeren isomeren Formen vorliegen können.

2. Verfahren nach Anspruch 1 zur Herstellung der Verbindungen der Formel (I_{B}), die der Formel (Iₐ) entsprechen: 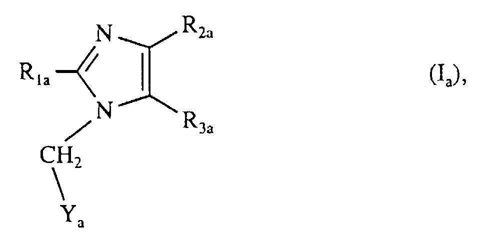 in der:
• R₁ₐ einen geradkettigen oder verzweigten Alkylrest mit maximal 4 Kohlenstoffatomen darstellt,
• R₂ₐ und R₃ₐ, die gleich oder verschieden sein können, ausgewählt sind unter:
a) dem Wasserstoffatom, dem Mercaptorest, dem Formylrest und den freien, als Salz vorliegenden oder veresterten Carboxylresten,
b) den Resten R₄ₐ und -S(O)ₙR₄ₐ, in denen n gleich 0, 1 oder ist,
wobei R₄ₐ in diesen Resten einen geradkettigen oder verzweigten Alkyl- oder Alkenylrest mit maximal 4 Kohlenstoffatomen, einen Cyclohexylrest, einen Phenyl-, Pyridyl-, Pyrimidinyl- oder Imidazolylrest darstellt, wobei all diese Reste gegebenenfalls mit einem oder mehreren Resten substituiert sind, die gleich oder verschieden sein können und die ausgewählt sind unter:
-- den Halogenatomen,
-- der Hydroxylgruppe, dem Mercaptorest, den Acylthioresten, dem Trifluormethyl-, dem Trifluormethylthio- und dem Trifluormethoxyrest sowie der Nitril-, der Azid- und der Nitrogruppe,
-- dem Phenylrest,
-- den Alkyl- und Alkoxyresten mit maximal 4 Kohlenstoffatomen, den freien, als Salz vorliegenden oder veresterten Carboxylresten, dem Tetrazolylrest, sowie den Acyl- und Acyloxyresten und
-- den Resten: 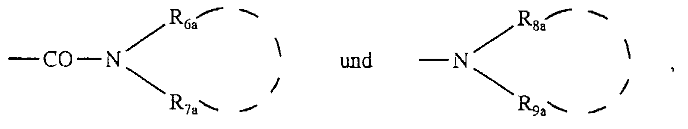 in denen:
**entweder**
R₆ₐ, R₇ₐ, R₈ₐ und R₉ₐ, die gleich oder verschieden sein können, ausgewählt sind unter dem Wasserstoffatom, der Hydroxylgruppe, den Alkyl-, Alkoxy-, Acyloxy- und Acylgruppen mit maximal 6 Kohlenstoffatomen, den freien, als Salz vorliegenden oder veresterten Carboxylresten sowie den folgenden Resten: Phenyl, Benzyl, Phenylethyl, Azepin, Piperidyl, Morpholin, Pyrrolidinyl und Piperazinyl,
**oder**
R₆ₐ und R₇ₐ beziehungsweise R₈ₐ und R₉ₐ zusammen mit dem Stickstoffatom, an das sie gebunden sind, einen Rest bilden, wobei diese Reste, die gleich oder verschieden sein können, unter folgenden Resten ausgewählt sind:
Imidazolyl, Pyrrolyl, Pyrrolinyl, Pyrrolidinyl, Pyridyl, Piperidinyl, Pyrimidinyl, Pyridazinyl, Pyrazinyl, Piperazinyl, Phenylpiperazinyl, Piperidyl, Oxazolyl, Morpholinyl und Thiomorpholinyl, Azepin und Indolyl, wobei diese Reste gegebenenfalls mit einem oder mehreren Resten substituiert sind, die gleich oder verschieden sein können und die unter den Halogenatomen, der Hydroxylgruppe, dem Trifluormethylrest sowie den Alkyl- und Alkoxyresten mit maximal 6 Kohlenstoffatomen ausgewählt sind,
mit der Maßgabe, daß zumindest einer der beiden Reste R₂ₐ und R₃ₐ den Rest -S(O)ₙR₄ₐ darstelit,
• Yₐ den Rest -Y₁ₐ-Bₐ-Y₂ₐ darstellt, in dem:
- Y₁ₐ einen Phenylrest bezeichnet,
- Bₐ eine Einfachbindung oder den Rest -CO-NH- bezeichnet und
- Y₂ₐ:
**entweder**, wenn Bₐ eine Einfachbindung oder den Rest -CO-NH- bezeichnet, einen Phenylrest, der in ortho-Stellung mit einem freien, als Salz vorliegenden oder veresterten Carboxylrest substituiert ist, oder einen Tetrazolylrest bezeichnet,
**oder,** wenn Bₐ eine Einfachbindung darstellt, eine Nitrilgruppe, einen freien, als Salz vorliegenden oder veresterten Carboxylrest oder einen Tetrazolylrest bezeichnet,
wobei diese Verbindungen der Formel (Iₐ) sowohl in allen möglichen racemischen, enantiomeren und diastereomeren isomeren Formen als auch in Form von Salzen vorliegen können, die durch eine Addition dieser Verbindungen der Formel (Iₐ) an mineralische oder organische Säuren beziehungsweise mineralische oder organische Basen gebildet werden, dadurch gekennzeichnet, daß von Verbindungen der Formel (II) und der Formel (III) ausgegangen wird, in denen R_{1'}, R_{2B'}, R_{3B'} und Y_{B'} die oben für R₁ₐ, R₂ₐ, R₃ₐ und Yₐ angegebene Bedeutung besitzen, wobei in diesen Resten die reaktiven Gruppen gegebenenfalls geschützt sind.

3. Verfahren nach Anspruch 2, dadurch gekennzeichnet, daß von einer Verbindung der Formel (II), in der:
• R_{1'} einen Alkylrest mit maximal 4 Kohlenstoffatomen darstellt und
• R_{2B'} und R_{3B'}, die gleich oder verschieden sein können, unter folgenden Resten ausgewählt sind:
- den freien, als Salz vorliegenden oder mit einem geradkettigen oder verzweigten Alkylrest mit maximal 4 Kohlenstoffatomen veresterten Carboxylresten, dem Formylrest, den Acyloxyresten, die ausgewählt sind unter dem Formyloxy-, dem Acetyloxy-, dem Propionyloxy-, dem Butyryloxy- und dem Benzoyloxyrest,
- Alkylresten mit maximal 4 Kohlenstoffatomen, die gegebenenfalls mit einer Hydroxylgruppe substituiert sind,
- dem Phenylthio-, dem Phenylsulfonyl- und dem Phenylsulfinylrest sowie
- den Alkylthio-, Alkylsulfonyl- und Alkylsulfinylresten, deren Alkylreste maximal 4 Kohlenstoffatome enthalten,
wobei in diesen Resten die reaktiven Gruppen gegebenenfalls geschützt sind,
und von einer Verbindung der Formel (III) ausgegangen wird, in der:
• Y_{B'} den Rest -Y₁-B-Y₂ darstellt, in dem
- Y₁ einen Phenylrest bezeichnet,
- B eine Kohlenstoff-Kohlenstoff-Einfachbindung bezeichnet und
- Y₂ einen freien oder mit einem geradkettigen oder verzweigten Alkylrest mit maximal 4 Kohlenstoffatomen veresterten Carboxylrest oder einen gegebenenfalls mit einem freien oder mit einem geradkettigen oder verzweigten Alkylrest mit maximal 4 Kohlenstoffatomen veresterten Carboxylrest substituierten Phenylrest bezeichnet, wobei in diesen Resten die reaktiven Gruppen gegebenenfalls geschützt sind.

4. Verfahren nach Anspruch 1 oder 2, dadurch gekennzeichnet, daß von einer Verbindung der Formel (II) ausgegangen wird, in der R_{2B'} oder R_{3B'} einen gegebenenfalls substituierten Alkoxy-, Alkylthio- oder Phenylthiorest darstellt, wobei der Alkylthiorest und der Phenylthiorest in oxidierter Form als Sulfoxid oder Sulfon vorliegen können, und der andere der beiden Reste R_{2B} und R_{3B} einen freien, als Salz vorliegenden oder veresterten Carboxylrest darstellt.

5. Verfahren zur Herstellung der Ausgangsverbindungen der Formel (II) wie in Anspruch 1 definiert, dadurch gekennzeichnet, daß eine Verbindung der Formel (IIₐ): 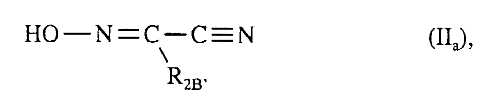 in der R_{2B'} die in Anspruch 1 für R_{2B} angegebene Bedeutung besitzt und in der die gegebenenfalls vorhandenen reaktiven Gruppen gegebenenfalls mit Schutzgruppen geschützt sind,
mit einem Reduktionsmittel umgesetzt wird, wobei das entsprechende Amin der folgenden Formel (II_{b}) erhalten wird: 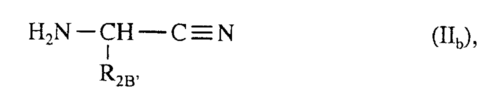 in der R_{2B'} die oben angegebene Bedeutung besitzt;
diese Verbindung der Formel (II_{b}) wird mit einer Verbindung der Formel (II_{c}) umgesetzt: 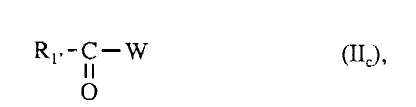 in der:
• R_{1'}, die in Anspruch 1 für R₁ angegebene Bedeutung besitzt, wobei die in diesem Rest gegebenenfalls vorhandenen reaktiven Gruppen gegebenenfalls mit Schutzgruppen geschützt sind, und
• W eine Hydroxylgruppe oder ein Halogenatom darstellt,
wobei eine Verbindung der folgenden Formel (II_{d}) erhalten wird: 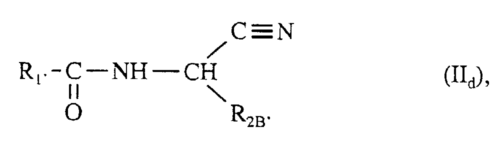 in der R_{1'} und R_{2B'} die oben angegebene Bedeutung besitzen und
die mit einer Verbindung der Formel (IIₑ) umgesetzt wird:
R_{3B'}―SH (IIₑ),
in der R_{3B'} die in Anspruch 1 für R_{3B} angegebene Bedeutung besitzt und in der die gegebenenfalls vorhandenen reaktiven Gruppen gegebenenfalls mit Schutzgruppen geschützt sind,
wobei eine Verbindung der Formel (II_{f}) erhalten wird: 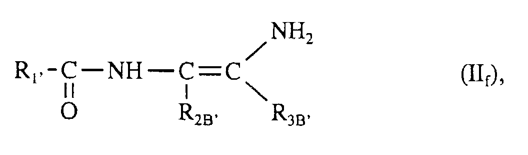 in der R_{1'}, R_{2B'} und R_{3B'} die oben angegebene Bedeutung besitzen und
die einer Cyclisierungsreaktion unterworfen wird, bei der eine Verbindung der Formel (II) erhalten wird, welche man, sofern dies gewünscht wird beziehungsweise notwendig ist, einer oder mehreren der folgenden Reaktionen unterwirft, die in beliebiger Reihenfolge durchgeführt werden können:
a) einer Eliminierung der Schutzgruppen, die die geschützten reaktiven Gruppen tragen können,
b) einer Salzbildung mit einer mineralischen oder organischen Säure oder mit einer Base, bei der das entsprechende Salz gebildet wird,
c) einer Veresterung der Säurefunktion,
d) einer Verseifung der Esterfunktion zur Säurefunktion,
e) einer Umsetzung der Nitrilfunktion zur Säurefunktion,
f) einer Reduktion der Carboxylfunktion zur Alkoholfunktion,
g) einer Umsetzung der Alkoxyfunktion zur Hydroxyfunktion,
h) einer Oxidation der Alkylthio- beziehungsweise Arylthiogruppe zum entsprechenden Sulfoxid oder Sulfon,
i) einer Umsetzung der Sulfoxid- beziehungsweise Sulfonfunktion zur entsprechenden Sulfoximinfunktion,
j) einer Oxidation der Alkoholfunktion zur Aldehyd- oder Säurefunktion,
k) einer Umsetzung der Nitrilfunktion zum Tetrazol,
l) einer Spaltung der Racemformen zu den enantiomerenreinen Verbindungen,
m) einer Umsetzung des Formylrests zum Carbamoylrest und
n) einer Umsetzung des Carbamoylrests zur Nitrilgruppe,
wobei die so erhaltenen Verbindungen der Formel (II) in allen möglichen racemischen, enantiomeren und diastereomeren isomeren Formen vorliegen können.

## Patentansprüche (Patentansprüche für folgende(n) Vertragsstaat(en): GR)

1. Verfahren zur Herstellung von Verbindungen der Formel (I_{B}): 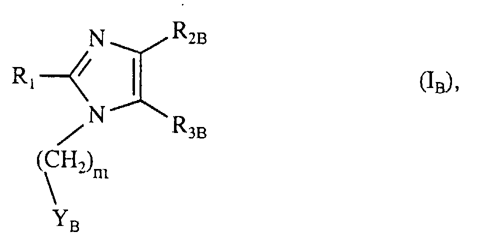 in der:
• R₁ einen Alkylrest darstellt, der maximal 4 Kohlenstoffatome enthält,
• R_{2B} und R_{3B}, die gleich oder verschieden sein können, ausgewählt sind unter:
- dem Wasserstoffatom,
- den freien, als Salz vorliegenden oder mit einem Alkylrest veresterten Carboxylresten,
- dem Formylrest, den Acyloxyresten, die unter den folgenden Resten ausgewählt sind: Formyloxy, Acetyloxy, Propionyloxy, Butyryloxy und Benzoyloxy, sowie dem Sulforest,
- den Alkyl- und Alkoxyresten, die gegebenenfalls substituiert sind,
und
- den Phenylthio-, Phenylsulfonyl-, Phenylsulfinyl- sowie Alkylthio-, Alkylsulfonyl- und Alkylsulfinylresten, die gegebenenfalls substituiert sind,
wobei in all diesen Resten, die durch R_{2B} und R_{3B} dargestellt werden können, die Alkyl- und Alkoxyreste maximal 6 Kohlenstoffatome enthalten und die Alkyl-, Alkoxy- und Phenylreste gegebenenfalls mit einem oder mehreren Resten substituiert sind, die unter den Halogenatomen, der Hydroxylgruppe, dem Trifluormethyl- und den Acyloxyresten, den freien, als Salz vorliegenden oder veresterten Carboxylresten, dem Phenyl-, dem Pyridyl- und dem Tetrazolylrest sowie den Alkyl- und Alkoxyresten mit maximal 4 Kohlenstoffatomen ausgewählt sind, die wiederum gegebenenfalls mit einem Alkoxyrest substituiert sind, der maximal 4 Kohlenstoffatome enthält,
mit der Maßgabe, daß zumindest eine der beiden Gruppen R_{2B} und R_{3B} eine Gruppe darstellt, die unter den folgenden Gruppen ausgewählt ist: Phenylthio-, Phenylsulfonyl-, Phenylsulfinyl- sowie Alkylthio-, Alkylsulfonyl- und Alkylsulfinylresten, die gegebenenfalls substituiert sind,
und
• Y_{B} den Rest -Y_{1B}-B-Y_{2B} darstellt, in dem:
- Y_{1B} einen Phenylrest bezeichnet,
- B eine Kohlenstoff-Kohlenstoff-Einfachbindung bezeichnet und
- Y_{2B} einen der folgenden Reste bezeichnet:
* einen freien oder mit einem geradkettigen oder verzweigten Alkylrest mit maximal 4 Kohlenstoffatomen veresterten Carboxylrest,
* einen Phenylrest, der gegebenenfalls mit einem freien oder mit einem geradkettigen oder verzweigten Alkylrest mit maximal 4 Kohlenstoffatomen veresterten Carboxylrest substituiert ist,
* eine Nitrilgruppe oder
* einen gegebenenfalls als Salz vorliegenden Tetrazolylrest,
wobei diese Verbindungen der Formel (I_{B}) sowohl in allen möglichen racemischen, enantiomeren und diastereomeren isomeren Formen als auch in Form von Salzen vorliegen können, die durch eine Addition dieser Verbindungen der Formel (I_{B}) an mineralische oder organische Säuren beziehungsweise mineralische oder organische Basen gebildet werden, dadurch gekennzeichnet, daß eine Verbindung der Formel (II): 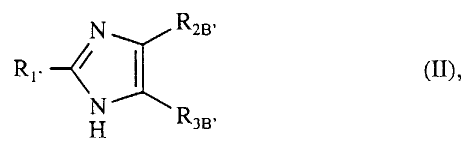 in der R_{1'}, R_{2B'} und R_{3B'} die in Anspruch 1 für R₁ beziehungsweise R_{2B} oder R_{3B} angegebene Bedeutung besitzen, wobei die in diesen Gruppen gegebenenfalls vorhandenen reaktiven Gruppen gegebenenfalls durch Schutzgruppen geschützt sind,
mit einer Verbindung der folgenden Formel (III) umgesetzt wird:
Z-(CH₂)ₘ-Y_{B'} (III),
in der Z ein Halogenatom oder eine Sulfonatgruppe darstellt und Y_{B'} die in Anspruch 1 für Y_{B} angegebene Bedeutung besitzt, wobei die in dieser Gruppe gegebenenfalls vorhandenen reaktiven Gruppen gegebenenfalls durch Schutzgruppen geschützt sind, wobei eine Verbindung der Formel (IV) erhalten wird: 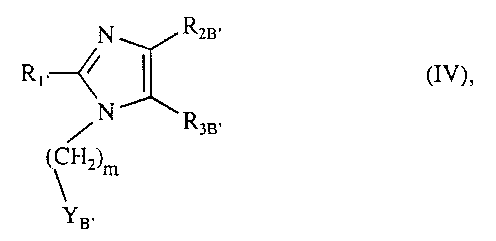 in der R_{1'}, R_{2B'}, R_{3B'} und Y_{B'} die oben angegebene Bedeutung besitzen,
wobei die Verbindung der Formel (IV), sofern dies gewünscht wird beziehungsweise notwendig ist, einer oder mehreren der folgenden Reaktionen unterworfen wird, die in beliebiger Reihenfolge durchgeführt werden können:
a) einer Eliminierung der Schutzgruppen, die die geschützten reaktiven Gruppen tragen können,
b) einer Salzbildung mit einer mineralischen oder organischen Säure oder mit einer Base, bei der das entsprechende Salz gebildet wird,
c) einer Veresterung der Säurefunktion,
d) einer Verseifung der Esterfunktion zur Säurefunktion,
e) einer Umsetzung der Nitrilfunktion zur Säurefunktion,
f) einer Reduktion der Carboxylfunktion zur Alkoholfunktion,
g) einer Umsetzung der Alkoxyfunktion zur Hydroxyfunktion,
h) einer Oxidation der Alkylthio- beziehungsweise Arylthiogruppe zum entsprechenden Sulfoxid oder Sulfon,
i) einer Umsetzung der Sulfoxid- beziehungsweise Sulfonfunktion zur entsprechenden Sulfoximinfunktion,
j) einer Oxidation der Alkoholfunktion zur Aldehyd- oder Säurefunktion,
k) einer Umsetzung der Nitrilgruppe zum Tetrazol,
l) einer Spaltung der Racemformen zu den enantiomerenreinen Verbindungen
m) einer Umsetzung des Formylrests zum Carbamoylrest und
n) einer Umsetzung des Carbamoylrests zur Nitrilgruppe,
wobei die so erhaltenen Verbindungen der Formel (I_{B}) in allen möglichen racemischen, enantiomeren und diastereomeren isomeren Formen vorliegen können.

2. Verfahren nach Anspruch 1 zur Herstellung der Verbindungen der Formel (I_{B}), die der Formel (Iₐ) entsprechen: 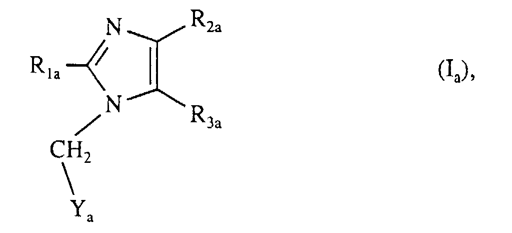 in der:
• R₁ₐ einen geradkettigen oder verzweigten Alkylrest mit maximal 4 Kohlenstoffatomen darstellt,
• R₂ₐ und R₃ₐ, die gleich oder verschieden sein können, ausgewählt sind unter:
a) dem Wasserstoffatom, dem Mercaptorest, dem Formylrest und den freien, als Salz vorliegenden oder veresterten Carboxylresten,
b) den Resten R₄ₐ und -S(O)ₙR₄ₐ, in denen n gleich 0, 1 oder 2 ist,
wobei R₄ₐ in diesen Resten einen geradkettigen oder verzweigten Alkyl- oder Alkenylrest mit maximal 4 Kohlenstoffatomen, einen Cyclohexylrest, einen Phenyl-, Pyridyl-, Pyrimidinyl- oder Imidazolylrest darstellt, wobei all diese Reste gegebenenfalls mit einem oder mehreren Resten substituiert sind, die gleich oder verschieden sein können und die ausgewählt sind unter:
-- den Halogenatomen,
-- der Hydroxylgruppe, dem Mercaptorest, den Acylthioresten, dem Trifluormethyl-, dem Trifluormethylthio- und dem Trifluormethoxyrest sowie der Nitril-, der Azid- und der Nitrogruppe,
-- dem Phenylrest,
-- den Alkyl- und Alkoxyresten mit maximal 4 Kohlenstoffatomen, den freien, als Salz vorliegenden oder veresterten Carboxylresten, dem Tetrazolylrest, sowie den Acyl- und Acyloxyresten und
-- den Resten: 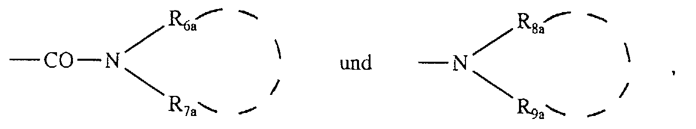 in denen:
**entweder**
R₆ₐ, R₇ₐ, R₈ₐ und R₉ₐ, die gleich oder verschieden sein können, ausgewählt sind unter dem Wasserstoffatom, der Hydroxylgruppe, den Alkyl-, Alkoxy-, Acyloxy- und Acylgruppen mit maximal 6 Kohlenstoffatomen, den freien, als Salz vorliegenden oder veresterten Carboxylresten sowie den folgenden Resten: Phenyl, Benzyl, Phenylethyl, Azepin, Piperidyl, Morpholin, Pyrrolidinyl und Piperazinyl,
**oder**
R₆ₐ und R₇ₐ beziehungsweise R₈ₐ und R₉ₐ zusammen mit dem Stickstoffatom, an das sie gebunden sind, einen Rest bilden, wobei diese Reste, die gleich oder verschieden sein können, unter folgenden Resten ausgewählt sind:
Imidazolyl, Pyrrolyl, Pyrrolinyl, Pyrrolidinyl, Pyridyl, Piperidinyl, Pyrimidinyl, Pyridazinyl, Pyrazinyl, Piperazinyl, Phenylpiperazinyl, Piperidyl, Oxazolyl, Morpholinyl und Thiomorpholinyl, Azepin und Indolyl, wobei diese Reste gegebenenfalls mit einem oder mehreren Resten substituiert sind, die gleich oder verschieden sein können und die unter den Halogenatomen, der Hydroxylgruppe, dem Trifluormethylrest sowie den Alkyl- und Alkoxyresten mit maximal 6 Kohlenstoffatomen ausgewählt sind,
mit der Maßgabe, daß zumindest einer der beiden Reste R₂ₐ und R₃ₐ den Rest -S(O)ₙR₄ₐ darstellt,
• Yₐ den Rest -Y₁ₐ-Bₐ-Y₂ₐ darstellt, in dem:
- Y₁ₐ einen Phenylrest bezeichnet,
- Bₐ eine Einfachbindung oder den Rest -CO-NH- bezeichnet und
- Y₂ₐ:
**entweder**, wenn Bₐ eine Einfachbindung oder den Rest -CO-NH- bezeichnet, einen Phenylrest, der in ortho-Stellung mit einem freien, als Salz vorliegenden oder veresterten Carboxylrest substituiert ist, oder einen Tetrazolylrest bezeichnet,
**oder,** wenn Bₐ eine Einfachbindung darstellt, eine Nitrilgruppe, einen freien, als Salz vorliegenden oder veresterten Carboxylrest oder einen Tetrazolylrest bezeichnet,
wobei diese Verbindungen der Formel (Iₐ) sowohl in allen möglichen racemischen, enantiomeren und diastereomeren isomeren Formen als auch in Form von Salzen vorliegen können, die durch eine Addition dieser Verbindungen der Formel (Iₐ) an mineralische oder organische Säuren beziehungsweise mineralische oder organische Basen gebildet werden, dadurch gekennzeichnet, daß von Verbindungen der Formel (II) und der Formel (III) ausgegangen wird, in denen R_{1'}, R_{2B'}, R_{3B'} und Y_{B'} die oben für R₁ₐ, R₂ₐ, R₃ₐ und Yₐ angegebene Bedeutung besitzen, wobei in diesen Resten die reaktiven Gruppen gegebenenfalls geschützt sind.

3. Verfahren nach Anspruch 2, dadurch gekennzeichnet, daß von einer Verbindung der Formel (II), in der:
• R_{1'} einen Alkylrest mit maximal 4 Kohlenstoffatomen darstellt und
• R_{2B'} und R_{3B'}, die gleich oder verschieden sein können, unter folgenden Resten ausgewählt sind:
- den freien, als Salz vorliegenden oder mit einem geradkettigen oder verzweigten Alkylrest mit maximal 4 Kohlenstoffatomen veresterten Carboxylresten, dem Formylrest, den Acyloxyresten, die ausgewählt sind unter dem Formyloxy-, dem Acetyloxy-, dem Propionyloxy-, dem Butyryloxy- und dem Benzoyloxyrest,
- Alkylresten mit maximal 4 Kohlenstoffatomen, die gegebenenfalls mit einer Hydroxylgruppe substituiert sind,
- dem Phenylthio-, dem Phenylsulfonyl- und dem Phenylsulfinylrest sowie
- den Alkylthio-, Alkylsulfonyl- und Alkylsulfinylresten, deren Alkylreste maximal 4 Kohlenstoffatome enthalten,
wobei in diesen Resten die reaktiven Gruppen gegebenenfalls geschützt sind,
und von einer Verbindung der Formel (III) ausgegangen wird, in der:
• Y_{B'} den Rest -Y₁-B-Y₂ darstellt, in dem
- Y₁ einen Phenylrest bezeichnet,
- B eine Kohlenstoff-Kohlenstoff-Einfachbindung bezeichnet und
- Y₂ einen freien oder mit einem geradkettigen oder verzweigten Alkylrest mit maximal 4 Kohlenstoffatomen veresterten Carboxylrest oder einen gegebenenfalls mit einem freien oder mit einem geradkettigen oder verzweigten Alkylrest mit maximal 4 Kohlenstoffatomen veresterten Carboxylrest substituierten Phenylrest bezeichnet, wobei in diesen Resten die reaktiven Gruppen gegebenenfalls geschützt sind.

4. Verfahren nach Anspruch 1 oder 2, dadurch gekennzeichnet, daß von einer Verbindung der Formel (II) ausgegangen wird, in der R_{2B'} oder R_{3B'} einen gegebenenfalls substituierten Alkoxy-, Alkylthio- oder Phenylthiorest darstellt, wobei der Alkylthiorest und der Phenylthiorest in oxidierter Form als Sulfoxid oder Sulfon vorliegen können, und der andere der beiden Reste R_{2B} und R_{3B} einen freien, als Salz vorliegenden oder veresterten Carboxylrest darstellt.

5. Verfahren zur Herstellung der Ausgangsverbindungen der Formel (II) wie in Anspruch 1 definiert, dadurch gekennzeichnet, daß eine Verbindung der Formel (IIₐ): 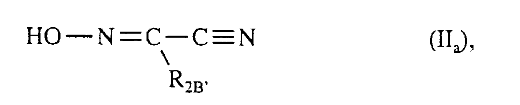 in der R_{2B'} die in Anspruch 1 für R_{2B} angegebene Bedeutung besitzt und in der die gegebenenfalls vorhandenen reaktiven Gruppen gegebenenfalls mit Schutzgruppen geschützt sind,
mit einem Reduktionsmittel umgesetzt wird, wobei das entsprechende Amin der folgenden Formel (II_{b}) erhalten wird: 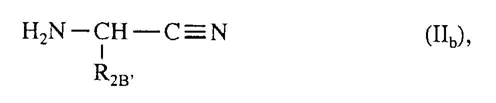 in der R_{2B'} die oben angegebene Bedeutung besitzt;
diese Verbindung der Formel (II_{b}) wird mit einer Verbindung der Formel (II_{c}) umgesetzt: 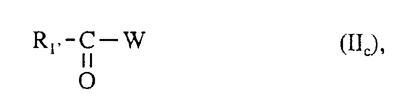 in der:
• R_{1'} die in Anspruch 1 für R₁ angegebene Bedeutung besitzt, wobei die in diesem Rest gegebenenfalls vorhandenen reaktiven Gruppen gegebenenfalls mit Schutzgruppen geschützt sind,
und
• W eine Hydroxylgruppe oder ein Halogenatom darstellt,
wobei eine Verbindung der folgenden Formel (II_{d}) erhalten wird: 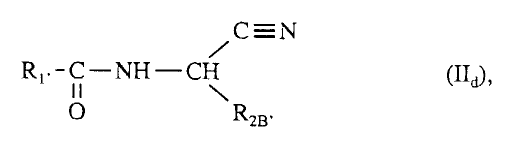 in der R_{1'} und R_{2B'} die oben angegebene Bedeutung besitzen und
die mit einer Verbindung der Formel (IIₑ) umgesetzt wird:
R_{3B'}―SH (IIₑ),
in der R_{3B'} die in Anspruch 1 für R_{3B} angegebene Bedeutung besitzt und in der die gegebenenfalls vorhandenen reaktiven Gruppen gegebenenfalls mit Schutzgruppen geschützt sind,
wobei eine Verbindung der Formel (II_{f}) erhalten wird: 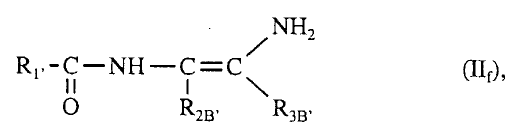 in der R_{1'}, R_{2B'} und R_{3B'} die oben angegebene Bedeutung besitzen und
die einer Cyclisierungsreaktion unterworfen wird, bei der eine Verbindung der Formel (II) erhalten wird, welche man, sofern dies gewünscht wird beziehungsweise notwendig ist, einer oder mehreren der folgenden Reaktionen unterwirft, die in beliebiger Reihenfolge durchgeführt werden können:
a) einer Eliminierung der Schutzgruppen, die die geschützten reaktiven Gruppen tragen können,
b) einer Salzbildung mit einer mineralischen oder organischen Säure oder mit einer Base, bei der das entsprechende Salz gebildet wird,
c) einer Veresterung der Säurefunktion,
d) einer Verseifung der Esterfunktion zur Säurefunktion,
e) einer Umsetzung der Nitrilfunktion zur Säurefunktion,
f) einer Reduktion der Carboxylfunktion zur Alkoholfunktion,
g) einer Umsetzung der Alkoxyfunktion zur Hydroxylfunktion,
h) einer Oxidation der Alkylthio- beziehungsweise Arylthiogruppe zum entsprechenden Sulfoxid oder Sulfon,
i) einer Umsetzung der Sulfoxid- beziehungsweise Sulfonfunktion zur entsprechenden Sulfoximinfunktion,
j) einer Oxidation der Alkoholfunktion zur Aldehyd- oder Säurefunktion,
k) einer Umsetzung der Nitrilfunktion zum Tetrazol,
l) einer Spaltung der Racemformen zu den enantiomerenreinen Verbindungen,
m) einer Umsetzung des Formylrests zum Carbamoylrest und
n) einer Umsetzung des Carbamoylrests zur Nitrilgruppe,
wobei die so erhaltenen Verbindungen der Formel (II) in allen möglichen racemischen, enantiomeren und diastereomeren isomeren Formen vorliegen können.

6. Verfahren nach Anspruch 1, dadurch gekennzeichnet, daß von Verbindungen der Formeln (II) und (III) ausgegangen wird, die so ausgewählt werden, daß Verbindungen hergestellt werden, die den folgenden chemischen Bezeichnungen entsprechen:
- 2-Butyl-1-[[2'-carboxy-(1,1'-biphenyl)-4-yl]-methyl]-4-(phenylthio)-1H-imidazol-5-carbonsäure,
- 2-Butyl-1-[[2'-carboxy-(1,1'-biphenyl)-4-yl]-methyl]-4-(methylthio)-1H-imidazol-5-carbonsäure,
- 4'-[[2-Butyl-4-(ethylthio)-5-hydroxylmethyl)-1H-imidazol-1-yl]-methyl]-(1,1'-biphenyl)-2-carbonsäure,
- 2-Butyl-1-[[2'-carboxy-(1,1'-biphenyl)-4-yl]-methyl]-4-(ethylsulfonyl)-1H-imidazol-5-carbonsäure,
- 2-Butyl-1-[[2'-carboxy-(1,1'-biphenyl)-4-yl]-methyl]-4-(ethylsulfinyl)-1H-imidazol-5-carbonsäure,
- 2-Butyl-1-[[2'-carboxy-(1,1'-biphenyl)-4-yl]-methyl]-4-(ethylthio)-1H-imidazol-5-carbonsäure,
- 2-Butyl-1-[[2'-carboxy-(1,1'-biphenyl)-4-yl]-methyl]-4-(phenylsulfonyl)-1H-imidazol-5-carbonsäure,
- 2-Butyl-1-[[2'-carboxy-(1,1'-biphenyl)-4-yl]-methyl]-4-(phenylsulfinyl)-1H-imidazol-5-carbonsäure,
- 2-Butyl-1-[[2'-tetrazolyl-(1,1'-biphenyl)-4-yl]-methyl]-4-(methylthio)-1H-imidazol-5-carbonsäure.

7. Verfahren zur Herstellung pharmazeutischer Zusammensetzungen, dadurch gekennzeichnet, daß als Wirkstoff mindestens eine der Verbindungen der Formel (I_{B}) wie in Anspruch 1 definiert oder mindestens eines der Salze, die durch eine Addition dieser Verbindungen an pharmazeutisch akzeptable mineralische oder organische Säuren beziehungsweise Basen gebildet werden, in einer für diese Anwendung bestimmten Form zugegeben wird.

8. Verfahren zur Herstellung pharmazeutischer Zusammensetzungen, dadurch gekennzeichnet, daß als Wirkstoff mindestens eine der Verbindungen der Formel (I_{B}) wie in einem der Ansprüche 2 bis 4 definiert oder mindestens eines der Salze, die durch eine Addition dieser Verbindungen an pharmazeutisch akzeptable mineralische oder organische Säuren beziehungsweise Basen gebildet werden, in einer für diese Anwendung bestimmten Form zugegeben wird.

9. Verfahren zur Herstellung pharmazeutischer Zusammensetzungen, dadurch gekennzeichnet, daß als Wirkstoff mindestens eine der Verbindungen der Formel (I_{B}) wie in Anspruch 6 definiert oder mindestens eines der Salze, die durch eine Addition dieser Verbindungen an pharmazeutisch akzeptable mineralische oder organische Säuren beziehungsweise Basen gebildet werden, in einer für diese Anwendung bestimmten Form zugegeben wird.

10. Verbindungen der Formel (II), in der R_{1'} keinen Methylrest darstellt, in ihrer Eigenschaft als neue industrielle Produkte.

## Claims (Claims for the following Contracting State(s): AT, BE, CH, DE, DK, FR, GB, IT, LI, LU, NL, SE)

1. Products of formula (I_{B}): 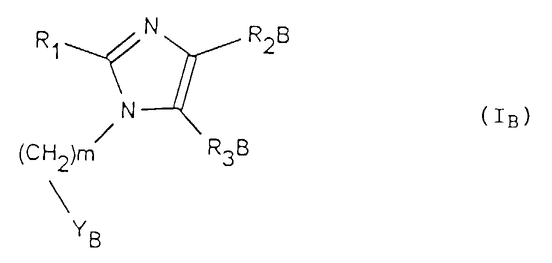 in which:
R₁ represents an alkyl radical containing at most 4 carbon atoms,
R_{2B} and R_{3B}, identical or different, are chosen from the following:
- the hydrogen atom,
- a carboxy radical, free salified or esterified by an alkyl radical,
- formyl, acyloxy selected from the group constituted by formyloxy, acetyloxy, propionyloxy, butyryloxy and benzoyloxy, sulpho,
- optionally substituted alkyl and alkoxy,
- phenylthio, phenylsulphonyl, phenylsulphinyl, alkylthio, alkylsulphonyl and alkylsulphinyl, optionally substituted, as in all those radicals that can be represented by R_{2B} and R_{3B}, the alkyl and alkoxy radicals contain at most 6 carbon atoms, and the alkyl, alkoxy and phenyl radicals are optionally substituted by one or more radicals chosen from halogen atoms and hydroxyl radicals, trifluoromethyl, acyloxy radicals, free, salified or esterified carboxy radicals, phenyl, pyridyl, tetrazolyl, alkyl and alkoxy radicals containing at most 4 carbon atoms and themselves optionally substituted by an alkoxy radical containing at most 4 carbon atoms, it being understood that at least one of the R_{2B} and R_{3B} groups represents a group chosen from phenylthio,
phenylsulfonyl, phenylsulfinyl, alkythio, alkylsulfonyl and alkylsulfinyl, optionally substituted,
and Y_{B} represents the -Y_{1B}-B-Y_{2B} radical in which Y_{1B} represents a phenyl radical, B represents a single carbon-carbon bond and Y_{2B} represents a carboxy radical, free or esterified by a linear or branched alkyl radical containing at most 4 carbon atoms or a phenyl radical optionally substituted by a carboxy radical, free or esterified by a linear or branched alkyl radical containing at most 4 carbon atoms, a cyano radical or a tetrazolyl radical optionally salified, the said products of formula (I_{B}) being in all the possible racemic, enantiomeric and diastereoisomeric isomer forms, as well as the addition salts with mineral and organic acids or with mineral and organic bases of the said products of formula (I_{B}).

2. Products of formula (I_{B}) as defined in claim 1 and corresponding to formula (Iₐ): 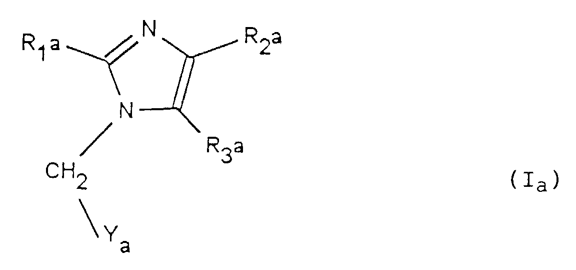 in which:
R₁ₐ represents a linear or branched alkyl radical containing at most 4 carbon atoms,
R₂ₐ and R₃ₐ, identical or different, are chosen from:
a) the hydrogen atom, the mercapto radical, the formyl radical, the free, salified or esterified carboxy radical,
b) the R₄ₐ and -S(O)ₙR₄ₐ radicals such that n represents the value 0, 1 or 2,
in which R₄ₐ represents a linear or branched alkyl or alkenyl radical containing at most 4 carbon atoms, a cyclohexyl radical, a phenyl, pyridyl, pyrimidinyl or imidazolyl radical, all these radicals being optionally substituted by one or more identical or different radicals chosen from:
- halogen atoms,
- hydroxyl, mercapto, acylthio, trifluoromethyl, trifluoromethylthio, trifluoromethoxy, cyano, azido or nitro radicals,
- the phenyl radical,
- alkyl and alkoxy radicals containing at most 4 carbon atoms, free, salified or esterified carboxy radicals, tetrazolyl, acyl, acyloxy radicals, and
- the radicals 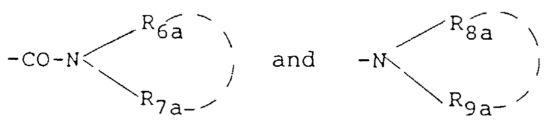 in which:
**either** R₆ₐ, R₇ₐ, R₈ₐ and R₉ₐ, identical or different, are chosen from the hydrogen atom, the hydroxyl radical, alkyl, alkoxy, acyloxy or acyl radicals, these radicals containing at most 6 carbon atoms, free, salified or esterified carboxy radicals, phenyl, benzyl, phenethyl, azepine, piperidyl, morpholine, pyrrolidinyl or piperazinyl radicals,
**or** on the one hand R₆ₐ and R₇ₐ and on the other hand R₈ₐ and R₉ₐ form respectively, with the nitrogen atom to which they are linked, a radical, these identical or different radicals being chosen from the following radicals: imidazolyl, pyrrolyl, pyrrolinyl, pyrrolidinyl, pyridyl, piperidinyl, pyrimidinyl, pyridazinyl, pyrazinyl, piperazinyl, phenylpiperazinyl, piperidyl, oxazolyl, morpholinyl and thiomorpholinyl, azepine, indolyl, these radicals being optionally substituted by one or more identical or different radicals chosen from halogen atoms, hydroxyl, trifluoromethyl, alkyl and alkoxy radicals, these radicals containing at most 6 carbon atoms, it being understood that at least one of R₂ₐ or R₃ₐ
represents the -S(O)ₙR₄ₐ radical,
Yₐ represents the -Y₁ₐ-Bₐ-Y₂ₐ radical in which:
- Y₁ₐ represents a phenyl radical,
- Bₐ represents a single bond or the -CO-NH- radical,
- Y₂ₐ is such that:
**either,** if Bₐ represents a single bond or a -CO-NH- radical, Y₂ₐ represents a phenyl radical substituted in the ortho position by a free, salified or esterified carboxy radical or a tetrazolyl radical,
**or,** if Bₐ represents a single bond, Y₂ₐ represents a cyano radical or a free, salified or esterified carboxy radical or a tetrazolyl radical,
the said products of formula (Iₐ) being in all the possible racemic, enantiomeric and diastereoisomeric isomer forms, as well as the addition salts with mineral and organic acids or with mineral and organic bases of the said products of formula (Iₐ).

3. Products of formula (I_{B}) as defined in claim 2 in which:
R₁ represents an alkyl radical containing at most 4 carbon atoms,
R_{2B} and R_{3B}, identical or different, are chosen from the following radicals:
- carboxy free, salified or esterified by a linear or branched alkyl radical containing at most 4 carbon atoms, formyl, acyloxy chosen from the group consisting of formyloxy, acetyloxy, propionyloxy, butyryloxy and benzoyloxy,
- alkyl containing at most 4 carbon atoms optionally substituted by a hydroxyl radical,
- phenylthio, phenylsulphonyl, phenylsulphinyl,
- alkylthio, alkylsulphonyl and alkylsulphinyl,
in which the alkyl radical contains at most 4 carbon atoms, and Y_{B} represents the -Y_{1B}-B-Y_{2B} radical in which Y_{1B} represents a phenyl radical, B represents a single carbon-carbon bond and Y_{2B} represents a carboxy radical, free or esterified by a linear or branched alkyl radical containing at most 4 carbon atoms or a phenyl radical optionally substituted by a carboxy radical free or esterified by a linear or branched alkyl radical containing at most 4 carbon atoms, the said products of formula (I_{B}) being in all the possible racemic, enantiomeric and diastereoisomeric isomer forms, as well as the addition salts with mineral and organic acids or with mineral and organic bases of the said products of formula (I_{B}).

4. Products of formula (I_{B}) as defined in claim 1, in which one of R_{2B} or R_{3B} represents an alkoxy, alkylthio or phenylthio radical, these two latter radicals being optionally oxidized in the form of the sulphoxide or sulphone and these three radicals being optionally substituted and the other represents a free, salified or esterified carboxy radical, the said products of formula (I_{B}) being in all the possible racemic, enantiomeric and diastereoisomeric isomer forms, as well as the addition salts with mineral and organic acids or with mineral and organic bases of the said products of formula (I_{B}).

5. The products of formula (I_{B}) corresponding to the following formulae:
- 2-butyl-1-[[2'-carboxy-(1,1'-biphenyl)-4-yl]-methyl]-4-(phenylthio)-1H-imidazole-5-carboxylic acid
- 2-butyl-1-[[2'-carboxy-(1,1'-biphenyl)-4-yl]-methyl]-4-(methylthio)-1H-imidazole-5-carboxylic acid
- 4'-[[2-butyl-4-(ethylthio)-5-(hydroxymethyl)-1H-imidazol-1-yl]-methyl]-(1,1'-biphenyl)-2-carboxylic acid
- 2-butyl 1-[[2'-carboxy-(1,1'-biphenyl)-4-yl]-methyl]-4-(ethylsulphonyl)-1H-imidazole-5-carboxylic acid
- 2-butyl-1-[[2'-carboxy-(1,1'-biphenyl)-4-yl]-methyl]-4-(ethylsulphinyl)-1H-imidazole-5-carboxylic acid
- 2-butyl-1-[[2'-carboxy-(1,1'-biphenyl)-4-yl]-methyl]-4-(ethylthio)-1H-imidazole-5-carboxylic acid
- 2-butyl-1-[[2'-carboxy-(1,1'-biphenyl)-4-yl]-methyl]-4-(phenylsulphonyl)-1H-imidazole-5-carboxylic acid
- 2-butyl-1-[[2'-carboxy-(1,1'-biphenyl)-4-yl]-methyl]-4-(phenylsulphinyl)-1H-imidazole-5-carboxylic acid
- 2-butyl-1-[[2'-tetrazolyl-(1,1'-biphenyl)-4-yl]-methyl]-4-(methylthio)-1H-imidazole-5-carboxylic acid.

6. Preparation process for the products of formula (I_{B}) as defined in claim 1, characterized in that a compound of formula (II): 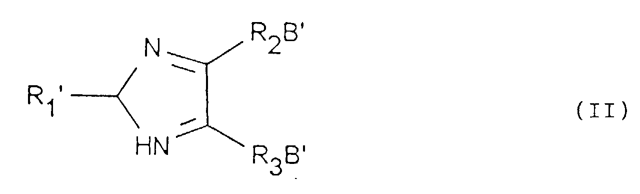 in which R₁', R_{2B}' and R_{3B}' have the meanings indicated in claim 1 for R₁, R_{2B} and R_{3B} respectively and in which the optional reactive functions are optionally protected by protective groups, is reacted with a compound of formula (III):
Z-(CH₂)ₘ-Y_{B}' (III)
in which Z represents a halogen atom or a sulphonate and Y_{B}' has the meaning indicated in claim 1 for Y_{B} in which the optional reactive functions are optionally protected by protective groups, in order to obtain a product of formula (IV): 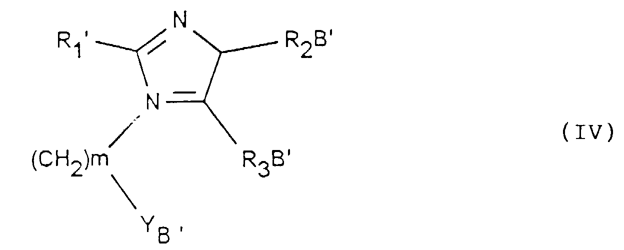 in which R₁', R_{2B}', R_{3B}' and Y_{B}' have the meanings indicated above,
which product of formula (IV) is subjected, if necessary and if desired, to one or more of the following reactions, in any order:
a) an elimination reaction of the protective groups that can be carried by the protected reactive functions,
b) a salification reaction by a mineral or organic acid or by a base in order to obtain the corresponding salt,
c) an esterification reaction of the acid function,
d) a saponification reaction of the ester function into an acid function,
e) a conversion reaction of the cyano function into an acid function,
f) a reduction reaction of the carboxy function to an alcohol function,
g) a conversion reaction of the alkoxy function into a hydroxyl function,
h) an oxidation reaction of the alkylthio or arylthio group into a corresponding sulphoxide or sulphone,
i) a conversion reaction of the sulphoxide or sulphone function into a corresponding sulphoximine function,
j) an oxidation reaction of the alcohol function into an aldehyde or acid function,
k) a conversion reaction of the nitrile function into tetrazole,
l) a resolution reaction of the racemic forms into resolved products,
m) a conversion reaction of the formyl radical into a carbamoyl radical,
n) a conversion reaction of the carbamoyl radical into a nitrile radical,
the said products of formula (I_{B}) thus obtained being in all the possible racemic, enantiomeric and diastereoisomeric isomer forms.

7. As medicaments, the products as defined by formula (I_{B}) of claim 1, the said products of formula (I_{B}) being in all the possible racemic, enantiomeric and diastereoisomeric isomer forms, as well as the addition salts with pharmaceutically acceptable mineral and organic acids or mineral and organic bases of the said products of formula (I_{B}).

8. As medicaments, the products of formulae (I_{B}) and (Iₐ) as defined in claims 2 to 4, as well as the addition salts with pharmaceutically acceptable mineral and organic acids or mineral and organic bases of the said products of formulae (I_{B}) and (Iₐ).

9. Pharmaceutical compositions containing as active ingredient, at least one of the medicaments as defined in any one of claims 7 and 8.

10. Preparation process for the starting products of formula (II) as defined in claim 6, characterized in that a compound of formula (IIₐ): 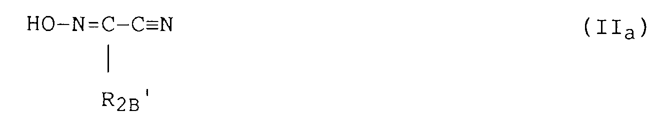 in which R_{2B} has the meaning indicated in claim 1 for R_{2B} in which the optional reactive functions are optionally protected by protective groups, is subjected to the action of a reducing agent, in order to obtain the corresponding amine of formula (II_{b}): 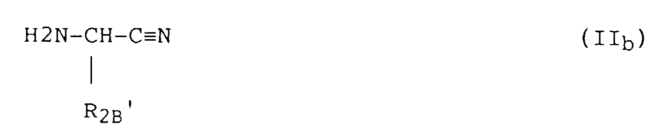 in which R_{2B}' has the meaning indicated previously, which is subjected to the action of a compound of formula (II_{c}): 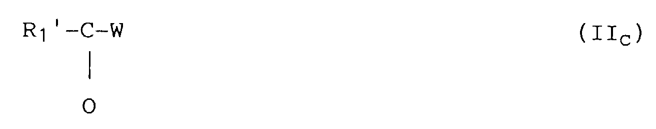 in which R₁' has the meaning indicated in claim 1 for R₁ in which the optional reactive functions are optionally protected by protective groups and W represents a hydroxyl radical or a halogen atom, in order to obtain a product of formula (II_{d}): 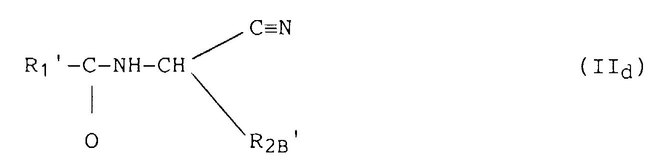 in which R₁' and R_{2B}' have the meanings indicated previously, which is reacted with a compound of formula (IIₑ):
R_{3B}'-SH (IIₑ)
in which R_{3B}' has the meaning indicated in claim 1 for R_{3B} in which the optional reactive functions are optionally protected by protective groups, in order to obtain a product of formula (II_{f}): 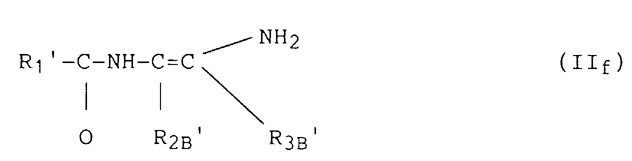 in which R₁', R_{2B}' and R_{3B}' have the meanings indicated previously, which is subjected to a cyclization reaction in order to obtain a product of formula (II), which product of formula (II) is subjected, if desired and if necessary, to one or more of the following reactions, in any order:
a) an elimination reaction of the protective groups that can be carried by the protected reactive functions,
b) a salification reaction by a mineral or organic acid or by a base in order to obtain the corresponding salt,
c) an esterification reaction of the acid function,
d) a saponification reaction of the ester function into an acid function,
e) a conversion reaction of the cyano function into an acid function,
f) a reduction reaction of the carboxy function to an alcohol function,
g) a conversion reaction of the alkoxy function into a hydroxyl function,
h) an oxidation reaction of the alkylthio or arylthio group into a corresponding sulphoxide or sulphone,
i) a conversion reaction of the sulphoxide or sulphone function into a corresponding sulphoximine function,
j) an oxidation reaction of the alcohol function into an aldehyde or acid function,
k) a conversion reaction of the nitrile function into tetrazole,
l) a resolution reaction of the racemic forms into resolved products,
m) a conversion reaction of the formyl radical into a carbamoyl radical,
n) a conversion reaction of the carbamoyl radical into a nitrile radical,
the said products of formula (II) thus obtained being in all the possible racemic, enantiomeric and diastereoisomeric isomer forms.

11. Compounds of formula (II) in which R₁' does not represent the methyl radical.

## Claims (Claims for the following Contracting State(s): ES)

1. Preparation process for products of formula (I_{B}): 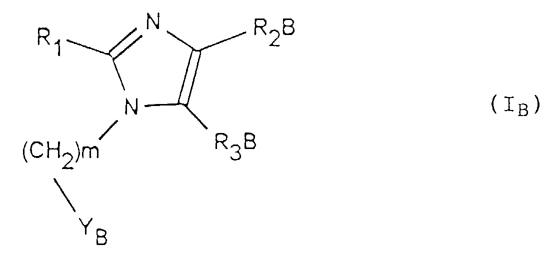 in which:
R₁ represents an alkyl radical containing at most 4 carbon atoms, R_{2B} and R_{3B} identical or different, are chosen from the following:
- the hydrogen atom,
- a carboxy radical, free salified or esterified by an alkyl radical,
- formyl, acyloxy selected from the group constituted by formyloxy, acetyloxy, propionyloxy, butyryloxy and benzoyloxy, sulpho,
- optionally substituted alkyl and alkoxy,
- phenylthio, phenylsulphonyl, phenylsulphinyl, alkylthio, alkylsulphonyl and alkylsulphinyl, optionally substituted, as in all those radicals that can be represented by R_{2B} and R_{3B}, the alkyl and alkoxy radicals contain at most 6 carbon atoms, and the alkyl, alkoxy and phenyl radicals are optionally substituted by one or more radicals chosen from halogen atoms and hydroxyl radicals, trifluoromethyl, acyloxy radicals, free, salified or esterified carboxy radicals, phenyl, pyridyl, tetrazolyl, alkyl and alkoxy radicals containing at most 4 carbon atoms and themselves optionally substituted by an alkoxy radical containing at most 4 carbon atoms, it being understood that at least one of the R_{2B} and R_{3B} groups represents a group chosen from phenylthio, phenylsulfonyl, phenylsulfinyl, alkythio, alkylsulfonyl and alkylsulfinyl, optionally substituted, and Y_{B} represents the -Y_{1B}-B-Y_{2B} radical in which Y_{1B} represents a phenyl radical, B represents a single carbon-carbon bond and Y_{2B} represents a carboxy radical, free or esterified by a linear or branched alkyl radical containing at most 4 carbon atoms or a phenyl radical optionally substituted by a carboxy radical, free or esterified by a linear or branched alkyl radical containing at most 4 carbon atoms, a cyano radical or a tetrazolyl radical optionally salified, the said products of formula (I_{B}) being in all the possible racemic, enantiomeric and diastereoisomeric isomer forms, as well as the addition salts with mineral and organic acids or with mineral and organic bases of the said products of formula (I_{B}), characterized in that a compound of formula (II): 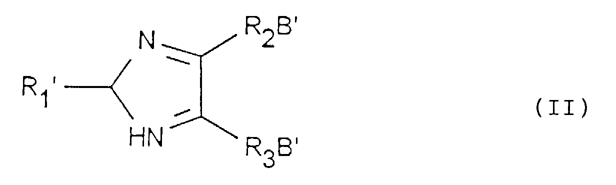
in which R₁', R_{2B}' and R_{3B}' have the meanings indicated in claim 1 for R₁, R_{2B} and R_{3B} respectively and in which the optional reactive functions are optionally protected by protective groups, is reacted with a compound of formula (III):
Z-(CH₂)ₘ-Y_{B}' (III)
in which Z represents a halogen atom or a sulphonate and Y_{B}' has the meaning indicated in claim 1 for Y_{B} in which the optional reactive functions are optionally protected by protective groups, in order to obtain a product of formula (IV): 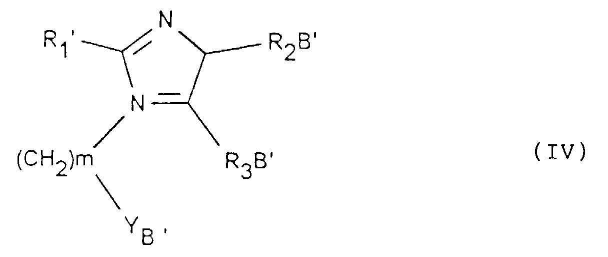 in which R₁', R_{2B}', R_{3B}' and Y_{B}^{,} have the meanings indicated above,
which product of formula (IV) is subjected, if necessary and if desired, to one or more of the following reactions, in any order:
a) an elimination reaction of the protective groups that can be carried by the protected reactive functions,
b) a salification reaction by a mineral or organic acid or by a base in order to obtain the corresponding salt,
c) an esterification reaction of the acid function,
d) a saponification reaction of the ester function into an acid function,
e) a conversion reaction of the cyano function into an acid function,
f) a reduction reaction of the carboxy function to an alcohol function,
g) a conversion reaction of the alkoxy function into a hydroxyl function,
h) an oxidation reaction of the alkylthio or arylthio group into a corresponding sulphoxide or sulphone,
i) a conversion reaction of the sulphoxide or sulphone function into a corresponding sulphoximine function,
j) an oxidation reaction of the alcohol function into an aldehyde or acid function,
k) a conversion reaction of the nitrile function into tetrazole,
l) a resolution reaction of the racemic forms into resolved products,
m) a conversion reaction of the formyl radical into a carbamoyl radical,
n) a conversion reaction of the carbamoyl radical into a nitrile radical,
the said products of formula (I_{B}) thus obtained being in all the possible racemic, enantiomeric and diastereoisomeric isomer forms.

2. Process according to claim 1 for the preparation of the products of formula (I_{B}) corresponding to formula (Iₐ): 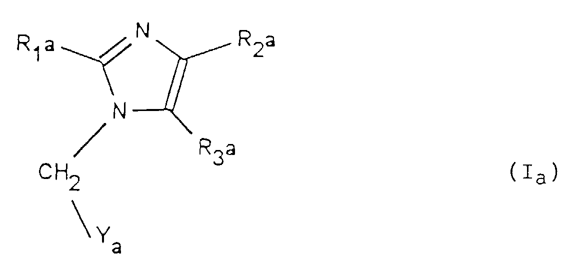 in which:
R₁ₐ represents a linear or branched alkyl radical containing at most 4 carbon atoms,
R₂ₐ and R₃ₐ, identical or different, are chosen from:
a) the hydrogen atom, the mercapto radical, the formyl radical, the free, salified or esterified carboxy radical,
b) the R₄ₐ and -S(O)ₙR₄ₐ radicals such that n represents the value 0, 1 or 2,
in which R₄ₐ represents a linear or branched alkyl or alkenyl radical containing at most 4 carbon atoms, a cyclohexyl radical, a phenyl, pyridyl, pyrimidinyl or imidazolyl radical, all these radicals being optionally substituted by one or more identical or different radicals chosen from:
- halogen atoms,
- hydroxyl, mercapto, acylthio, trifluoromethyl, trifluoromethylthio, trifluoromethoxy, cyano, azido or nitro radicals,
- the phenyl radical,
- alkyl and alkoxy radicals containing at most 4 carbon atoms, free, salified or esterified carboxy radicals, tetrazolyl, acyl, acyloxy radicals, and
- the radicals 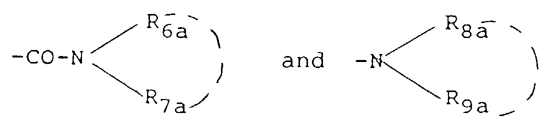 in which:
**either** R₆ₐ, R₇ₐ, R₈ₐ and R₉ₐ, identical or different, are chosen from the hydrogen atom, the hydroxyl radical, alkyl, alkoxy, acyloxy or acyl radicals, these radicals containing at most 6 carbon atoms, free, salified or esterified carboxy radicals, phenyl, benzyl, phenethyl, azepine, piperidyl, morpholine, pyrrolidinyl or piperazinyl radicals,
**or** on the one hand R₆ₐ and R₇ₐ and on the other hand R₈ₐ and R₉ₐ form respectively, with the nitrogen atom to which they are linked, a radical, these identical or different radicals being chosen from the following radicals: imidazolyl, pyrrolyl, pyrrolinyl, pyrrolidinyl, pyridyl, piperidinyl, pyrimidinyl, pyridazinyl, pyrazinyl, piperazinyl, phenylpiperazinyl, piperidyl, oxazolyl, morpholinyl and thiomorpholinyl, azepine, indolyl, these radicals being optionally substituted by one or more identical or different radicals chosen from halogen atoms, hydroxyl, trifluoromethyl, alkyl and alkoxy radicals, these radicals containing at most 6 carbon atoms, it being understood that at least one of R₂ₐ or R₃ₐ represents the -S(O)ₙR₄ₐ radical,
Yₐ represents the -Y₁ₐ-Bₐ-Y₂ₐ radical in which:
- Y₁ₐ represents a phenyl radical,
- Bₐ represents a single bond or the -CO-NH- radical,
- Y₂ₐ is such that:
**either,** if Bₐ represents a single bond or a -CO-NH- radical, Y₂ₐ represents a phenyl radical substituted in the ortho position by a free, salified or esterified carboxy radical or a tetrazolyl radical,
**or,** if Bₐ represents a single bond, Y₂ₐ represents a cyano radical or a free, salified or esterified carboxy radical or a tetrazolyl radical,
the said products of formula (Iₐ) being in all the possible racemic, enantiomeric and diastereoisomeric isomer forms, as well as the addition salts with mineral and organic acids or with mineral and organic bases of the said products of formula (Iₐ), characterized in that the starting product used is a product of formula (II) and (III) in which R'₁, R'_{2B}, R'_{3B} and Y'_{B} have the values indicated above respectively for R₁ₐ, R₂ₐ, R₃ₐ and Yₐ in which the reactive functions are optionally protected.

3. Process according to claim 2, characterized in that the starting product used is a product of formula (II) in which R'₁ represents an alkyl radical containing at most 4 carbon atoms, R'_{2B} and R'_{3B}, identical or different, are chosen from the following radicals:
- carboxy free, salified or esterified by a linear or branched alkyl radical containing at most 4 carbon atoms, formyl, acyloxy, chosen from the group consisting in formyloxy, acetyloxy, propionyloxy, butyryloxy and benzoyloxy,
- alkyl containing at most 4 carbon atoms optionally substituted by a hydroxyl radical,
- phenylthio, phenylsulphonyl, phenylsulphinyl,
- alkylthio, alkylsulphonyl and alkylsulphinyl,
in which the alkyl radical contains at most 4 carbon atoms, in which the reactive functions are optionally protected and a product of formula (III) in which Y'_{B} represents the -Y₁-B-Y₂ radical in which Y₁ represents a phenyl radical, B represents a single carbon-carbon bond and Y₂ represents a carboxy radical, free or esterified by a linear or branched alkyl radical containing at most 4 carbon atoms or a phenyl radical optionally substituted by a carboxy radical free or esterified by a linear or branched alkyl radical containing at most 4 carbon atoms, in which the reactive functions are optionally protected.

4. Process according to claim 1 or 2, characterized in that the starting product used is a product of formula (II) in which R'_{2B} or R'_{3B} represents an alkoxy, alkylthio or phenylthio radical, these two latter radicals being optionally oxidized in the form of the sulphoxide or sulphone and these three radicals being optionally substituted and the other represents a free, salified or esterified carboxy radical. 5) Preparation process for the starting products of formula (II) as defined in claim 1, characterized in that a compound of formula (IIₐ): 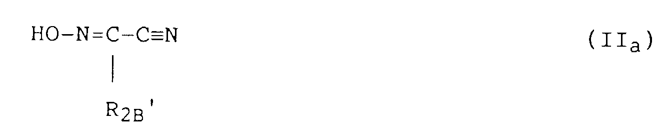 in which R_{2B}' has the meaning indicated in claim 1 for R_{2B} in which the optional reactive functions are optionally protected by protective groups, is subjected to the action of a reducing agent, in order to obtain the corresponding amine of formula (II_{b}): 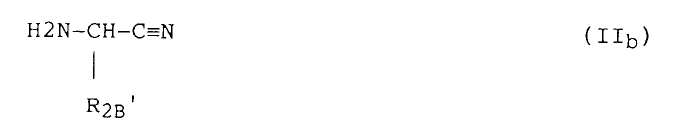 in which R_{2B}' has the meaning indicated previously, which is subjected to the action of a compound of formula (II_{c}): 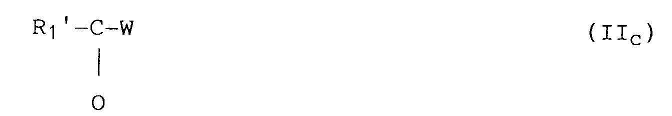 in which R₁' has the meaning indicated in claim 1 for R₁ in which the optional reactive functions are optionally protected by protective groups and W represents a hydroxyl radical or a halogen atom, in order to obtain a product of formula (II_{d}): 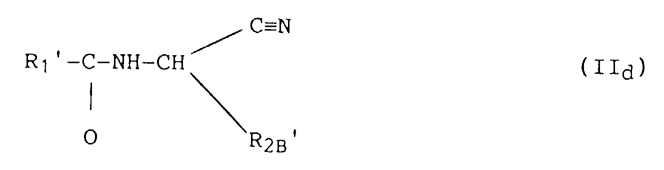 in which R₁' and R_{2B}' have the meanings indicated previously, which is reacted with a compound of formula (IIₑ):
R_{3B}'-SH (IIₑ)
in which R_{3B}' has the meaning indicated in claim 1 for R_{3B} in which the optional reactive functions are optionally protected by protective groups, in order to obtain a product of formula (II_{f}): 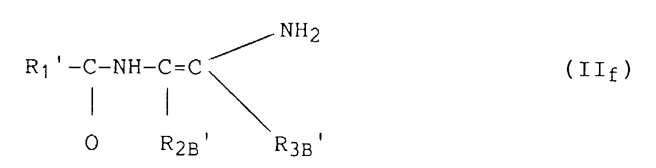 in which R₁', R_{2B}' and R_{3B}' have the meanings indicated previously, which is subjected to a cyclization reaction in order to obtain a product of formula (II), which product of formula (II) is subjected, if desired and if necessary, to one or more of the following reactions, in any order:
a) an elimination reaction of the protective groups that can be carried by the protected reactive functions,
b) a salification reaction by a mineral or organic acid or by a base in order to obtain the corresponding salt,
c) an esterification reaction of the acid function,
d) a saponification reaction of the ester function into an acid function,
e) a conversion reaction of the cyano function into an acid function,
f) a reduction reaction of the carboxy function to an alcohol function,
g) a conversion reaction of the alkoxy function into a hydroxyl function,
h) an oxidation reaction of the alkylthio or arylthio group into a corresponding sulphoxide or sulphone,
i) a conversion reaction of the sulphoxide or sulphone function into a corresponding sulphoximine function,
j) an oxidation reaction of the alcohol function into an aldehyde or acid function,
k) a conversion reaction of the nitrile function into tetrazole,
l) a resolution reaction of the racemic forms into resolved products,
m) a conversion reaction of the formyl radical into a carbamoyl radical,
n) a conversion reaction of the carbamoyl radical into a nitrile radical,
the said products of formula (II) thus obtained being in all the possible racemic, enantiomeric and diastereoisomeric isomer forms.

## Claims (Claims for the following Contracting State(s): GR)

1. Preparation process for products of formula (I_{B}): 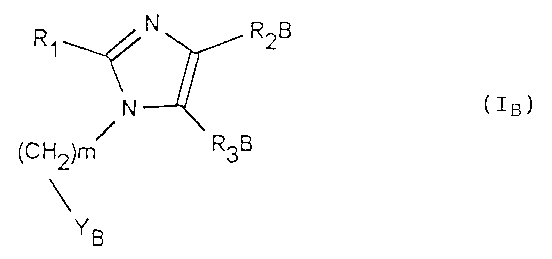 in which:
R₁ represents an alkyl radical containing at most 4 carbon atoms, R_{2B} and R_{3B} identical or different, are chosen from the following:
- the hydrogen atom,
- a carboxy radical, free salified or esterified by an alkyl radical,
- formyl, acyloxy selected from the group constituted by formyloxy, acetyloxy, propionyloxy, butyryloxy and benzoyloxy, sulpho,
- optionally substituted alkyl and alkoxy,
- phenylthio, phenylsulphonyl, phenylsulphinyl, alkylthio, alkylsulphonyl and alkylsulphinyl, optionally substituted, as in all those radicals that can be represented by R_{2B} and R_{3B}, the alkyl and alkoxy radicals contain at most 6 carbon atoms, and the alkyl, alkoxy and phenyl radicals are optionally substituted by one or more radicals chosen from halogen atoms and hydroxyl radicals, trifluoromethyl, acyloxy radicals, free, salified or esterified carboxy radicals, phenyl, pyridyl, tetrazolyl, alkyl and alkoxy radicals containing at most 4 carbon atoms and themselves optionally substituted by an alkoxy radical containing at most 4 carbon atoms, it being understood that at least one of the R_{2B} and R_{3B} groups represents a group chosen from phenylthio, phenylsulfonyl, phenylsulfinyl, alkythio, alkylsulfonyl and alkylsulfinyl, optionally substituted, and Y_{B} represents the -Y_{1B}-B-Y_{2B} radical in which Y_{1B} represents a phenyl radical, B represents a single carbon-carbon bond and Y_{2B} represents a carboxy radical, free or esterified by a linear or branched alkyl radical containing at most 4 carbon atoms or a phenyl radical optionally substituted by a carboxy radical, free or esterified by a linear or branched alkyl radical containing at most 4 carbon atoms, a cyano radical or a tetrazolyl radical optionally salified, the said products of formula (I_{B}) being in all the possible racemic, enantiomeric and diastereoisomeric isomer forms, as well as the addition salts with mineral and organic acids or with mineral and organic bases of the said products of formula (I_{B}), characterized in that a compound of formula (II): 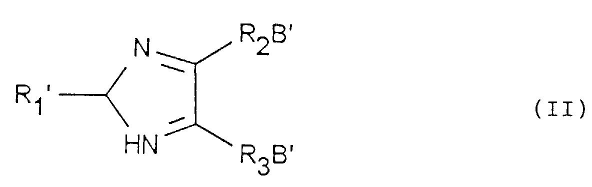
in which R₁', R_{2B}' and R_{3B}' have the meanings indicated in claim 1 for R₁, R_{2B} and R_{3B} respectively and in which the optional reactive functions are optionally protected by protective groups, is reacted with a compound of formula (III):
Z-(CH₂)ₘ-Y_{B}' (III)
in which Z represents a halogen atom or a sulphonate and Y_{B}' has the meaning indicated in claim 1 for Y_{B} in which the optional reactive functions are optionally protected by protective groups, in order to obtain a product of formula (IV): 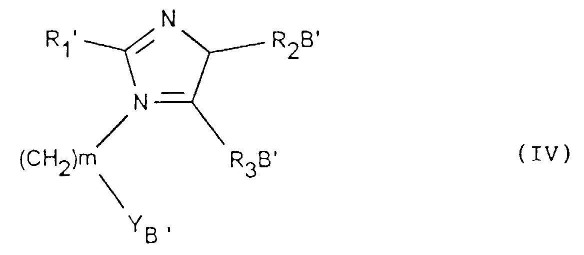 in which R₁', R_{2B}', R_{3B}' and Y_{B}' have the meanings indicated above,
which product of formula (IV) is subjected, if necessary and if desired, to one or more of the following reactions, in any order:
a) an elimination reaction of the protective groups that can be carried by the protected reactive functions,
b) a salification reaction by a mineral or organic acid or by a base in order to obtain the corresponding salt,
c) an esterification reaction of the acid function,
d) a saponification reaction of the ester function into an acid function,
e) a conversion reaction of the cyano function into an acid function,
f) a reduction reaction of the carboxy function to an alcohol function,
g) a conversion reaction of the alkoxy function into a hydroxyl function,
h) an oxidation reaction of the alkylthio or arylthio group into a corresponding sulphoxide or sulphone,
i) a conversion reaction of the sulphoxide or sulphone function into a corresponding sulphoximine function,
j) an oxidation reaction of the alcohol function into an aldehyde or acid function,
k) a conversion reaction of the nitrile function into tetrazole,
l) a resolution reaction of the racemic forms into resolved products,
m) a conversion reaction of the formyl radical into a carbamoyl radical,
n) a conversion reaction of the carbamoyl radical into a nitrile radical,
the said products of formula (I_{B}) thus obtained being in all the possible racemic, enantiomeric and diastereoisomeric isomer forms.

2. Process according to claim 1 for the preparation of the products of formula (I_{B}) corresponding to the formula (Iₐ): 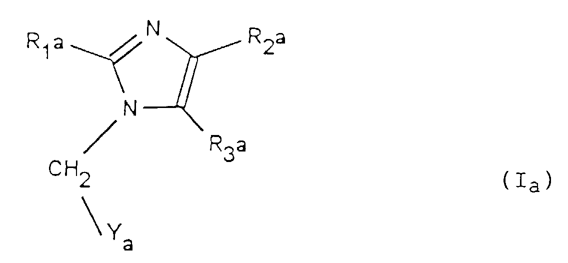 in which:
R₁ₐ represents a linear or branched alkyl radical containing at most 4 carbon atoms,
R₂ₐ and R₃ₐ, identical or different, are chosen from:
a) the hydrogen atom, the mercapto radical, the formyl radical, the free, salified or esterified carboxy radical,
b) the R₄ₐ and -S(O)ₙR₄ₐ radicals such that n represents the value 0, 1 or 2,
in which R₄ₐ represents a linear or branched alkyl or alkenyl radical containing at most 4 carbon atoms, a cyclohexyl radical, a phenyl, pyridyl, pyrimidinyl or imidazolyl radical, all these radicals being optionally substituted by one or more identical or different radicals chosen from:
- halogen atoms,
- hydroxyl, mercapto, acylthio, trifluoromethyl, trifluoromethylthio, trifluoromethoxy, cyano, azido or nitro radicals,
- the phenyl radical,
- alkyl and alkoxy radicals containing at most 4 carbon atoms, free, salified or esterified carboxy radicals, tetrazolyl, acyl, acyloxy radicals, and
- the radicals 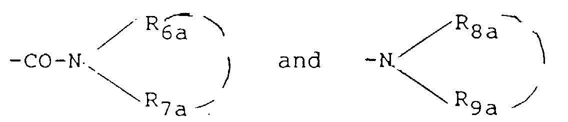 in which:
**either** R₆ₐ, R₇ₐ, R₈ₐ and R₉ₐ, identical or different, are chosen from the hydrogen atom, the hydroxyl radical, alkyl, alkoxy, acyloxy or acyl radicals, these radicals containing at most 6 carbon atoms, free, salified or esterified carboxy radicals, phenyl, benzyl, phenethyl, azepine, piperidyl, morpholine, pyrrolidinyl or piperazinyl radicals,
**or** on the one hand R₆ₐ and R₇ₐ and on the other hand R₈ₐ and R₉ₐ form respectively, with the nitrogen atom to which they are linked, a radical, these identical or different radicals being chosen from the following radicals: imidazolyl, pyrrolyl, pyrrolinyl, pyrrolidinyl, pyridyl, piperidinyl, pyrimidinyl, pyridazinyl, pyrazinyl, piperazinyl, phenylpiperazinyl, piperidyl, oxazolyl, morpholinyl and thiomorpholinyl, azepine, indolyl, these radicals being optionally substituted by one or more identical or different radicals chosen from halogen atoms, hydroxyl, trifluoromethyl, alkyl and alkoxy radicals, these radicals containing at most 6 carbon atoms, it being understood that at least one of R₂ₐ or R₃ₐ represents the -S(O)ₙR₄ₐ radical,
Yₐ represents the -Y₁ₐ-Bₐ-Y₂ₐ radical in which:
- Y₁ₐ represents a phenyl radical,
- Bₐ represents a single bond or the -CO-NH- radical,
- Y₂ₐ is such that:
**either,** if Bₐ represents a single bond or a -CO-NH- radical, Y₂ₐ represents a phenyl radical substituted in the ortho position by a free, salified or esterified carboxy radical or a tetrazolyl radical,
**or,** if Bₐ represents a single bond, Y₂ₐ represents a cyano radical or a free, salified or esterified carboxy radical or a tetrazolyl radical,
the said products of formula (Iₐ) being in all the possible racemic, enantiomeric and diastereoisomeric isomer forms, as well as the addition salts with mineral and organic acids or with mineral and organic bases of the said products of formula (Iₐ), characterized in that the starting product used is a product of formula (II) and (III) in which R'₁, R'_{2B}, R'_{3B} and Y'_{B} have the values indicated above respectively for R₁ₐ, R₂ₐ, R₃ₐ and Yₐ in which the reactive functions are optionally protected.

3. Process according to claim 2, characterized in that the starting product used is a product of formula (II) in which R'₁ represents an alkyl radical containing at most 4 carbon atoms, R'_{2B} and R'_{3B}, identical or different, are chosen from the following radicals:
- carboxy free, salified or esterified by a linear or branched alkyl radical containing at most 4 carbon atoms, formyl, acyloxy, chosen from the group consisting in formyloxy, acetyloxy, propionyloxy, butyryloxy and benzoyloxy,
- alkyl containing at most 4 carbon atoms optionally substituted by a hydroxyl radical,
- phenylthio, phenylsulphonyl, phenylsulphinyl,
- alkylthio, alkylsulphonyl and alkylsulphinyl,
in which the alkyl radical contains at most 4 carbon atoms, in which the reactive functions are optionally protected and a product of formula (III) in which Y'_{B} represents the -Y₁-B-Y₂ radical in which Y₁ represents a phenyl radical, B represents a single carbon-carbon bond and Y₂ represents a carboxy radical, free or esterified by a linear or branched alkyl radical containing at most 4 carbon atoms or a phenyl radical optionally substituted by a carboxy radical free or esterified by a linear or branched alkyl radical containing at most 4 carbon atoms, in which the reactive functions are optionally protected.

4. Process according to claim 1 or 2, characterized in that the starting product used is a product of formula (II) in which R'_{2B} or R'_{3B} represents an alkoxy, alkylthio or phenylthio radical, these two latter radicals being optionally oxidized in the form of the sulphoxide or sulphone and these three radicals being optionally substituted and the other represents a free, salified or esterified carboxy radical.

5. Preparation process for the starting products of formula (II) as defined in claim 1, characterized in that a compound of formula (IIₐ): 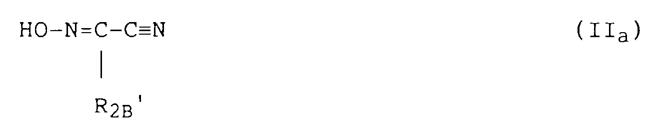 in which R_{2B} has the meaning indicated in claim 1 for R_{2B} in which the optional reactive functions are optionally protected by protective groups, is subjected to the action of a reducing agent, in order to obtain the corresponding amine of formula (II_{b}): 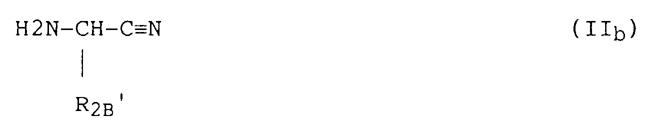 in which R_{2B}' has the meaning indicated previously, which is subjected to the action of a compound of formula (II_{c}): 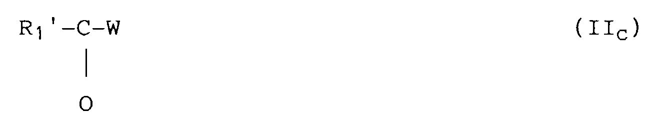 in which R₁' has the meaning indicated in claim 1 for R₁ in which the optional reactive functions are optionally protected by protective groups and W represents a hydroxyl radical or a halogen atom, in order to obtain a product of formula (II_{d}): 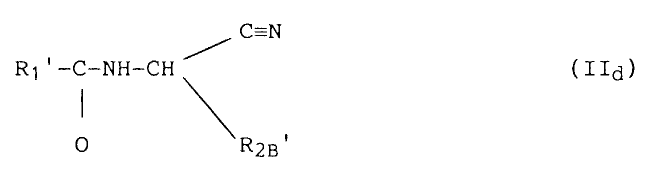 in which R₁' and R_{2B}' have the meanings indicated previously, which is reacted with a compound of formula (IIₑ):
R_{3B}'-SH (IIₑ)
in which R_{3B}' has the meaning indicated in claim 1 for R_{3B} in which the optional reactive functions are optionally protected by protective groups, in order to obtain a product of formula (II_{f}): 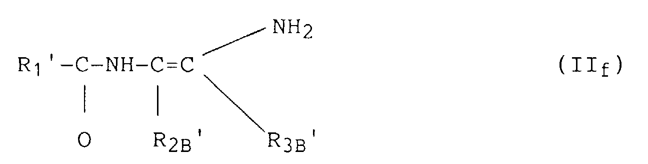 in which R₁', R_{2B}' and R_{3B}' have the meanings indicated previously, which is subjected to a cyclization reaction in order to obtain a product of formula (II), which product of formula (II) is subjected, if desired and if necessary, to one or more of the following reactions, in any order:
a) an elimination reaction of the protective groups that can be carried by the protected reactive functions,
b) a salification reaction by a mineral or organic acid or by a base in order to obtain the corresponding salt,
c) an esterification reaction of the acid function,
d) a saponification reaction of the ester function into an acid function,
e) a conversion reaction of the cyano function into an acid function,
f) a reduction reaction of the carboxy function to an alcohol function,
g) a conversion reaction of the alkoxy function into a hydroxyl function,
h) an oxidation reaction of the alkylthio or arylthio group into a corresponding sulphoxide or sulphone,
i) a conversion reaction of the sulphoxide or sulphone function into a corresponding sulphoximine function,
j) an oxidation reaction of the alcohol function into an aldehyde or acid function,
k) a conversion reaction of the nitrile function into tetrazole,
l) a resolution reaction of the racemic forms into resolved products,
m) a conversion reaction of the formyl radical into a carbamoyl radical,
n) a conversion reaction of the carbamoyl radical into a nitrile radical,
the said products of formula (II) thus obtained being in all the possible racemic, enantiomeric and diastereoisomeric isomer forms.

6. Process according to claim 1, characterized in that the compounds of formula (II) and (III) are used at the start and selected in such a way that products are prepared which correspond to the following formulae:
- 2-butyl-1-[[2'-carboxy-(1,1'-biphenyl)-4-yl]-methyl]-4-(phenylthio)-1H-imidazole-5-carboxylic acid
- 2-butyl-1-[[2'-carboxy-(1,1'-biphenyl)-4-yl]-methyl]-4-(methylthio)-1H-imidazole-5-carboxylic acid
- 4'-[[2-butyl-4-(ethylthio)-5-(hydroxymethyl)-1H-imidazol-1-yl]-methyl]-(1,1'-biphenyl)-2-carboxylic acid
- 2-butyl 1-[[2'-carboxy-(1,1'-biphenyl)-4-yl]-methyl]-4-(ethylsulphonyl)-1H-imidazole-5-carboxylic acid
- 2-butyl-1-[[2'-carboxy-(1,1'-biphenyl)-4-yl]-methyl]-4-(ethylsulphinyl)-1H-imidazole-5-carboxylic acid
- 2-butyl-1-[[2'-carboxy-(1,1'-biphenyl)-4-yl]-methyl]-4-(ethylthio)-1H-imidazole-5-carboxylic acid
- 2-butyl-1-[[2'-carboxy-(1,1'-biphenyl)-4-yl]-methyl]-4-(phenylsulphonyl)-1H-imidazole-5-carboxylic acid
- 2-butyl-1-[[2'-carboxy-(1,1'-biphenyl)-4-yl]-methyl]-4-(phenylsulphinyl)-1H-imidazole-5-carboxylic acid
- 2-butyl-1-[[2'-tetrazolyl-(1,1'-biphenyl)-4-yl]-methyl]-4-(methylthio)-1H-imidazole-5-carboxylic acid.

7. Process for the preparation of pharmaceutical compositions characterized in that, as active ingredient, at least one of the products of formula (I_{B}) is used as defined in claim 1 or at least one of their addition salts with pharmaceutically acceptable mineral or organic acids or with mineral or organic bases in a form intended for this use.

8. Process for the preparation of pharmaceutical compositions characterized in that, as active ingredient, at least one of the products of formula (I_{B}) is used as defined in any one of claims 2 to 4 or at least one of their addition salts with pharmaceutically acceptable mineral or organic acids or with mineral or organic bases in a form intended for this use.

9. Process for the preparation of pharmaceutical compositions characterized in that, as active ingredient, at least one of the products of formula (I_{B}) is used as defined in claim 6 or at least one of their addition salts with pharmaceutically acceptable mineral or organic acids or with mineral or organic bases in a form intended for this use.

10. As new industrial products, the compounds of formula (II) in which R₁' does not represent the methyl radical.
